(19) **Europäisches Patentamt / European Patent Office / Office européen des brevets**

(11) **EP 1 951 888 B1**

(12) # EUROPEAN PATENT SPECIFICATION

(45) Date of publication and mention of the grant of the patent:
**05.09.2012 Bulletin 2012/36**

(51) Int Cl.:
**C12Q 1/68** (2006.01)

(21) Application number: **06844292.0**

(22) Date of filing: **09.11.2006**

(86) International application number:
**PCT/US2006/043502**

(87) International publication number:
**WO 2007/056463 (18.05.2007 Gazette 2007/20)**

(54) **MULTIPLEXED QUANTITATIVE DETECTION OF PATHOGENS**

MULTIPLEXIERTE QUANTITATIVE ERKENNUNG VON KRANKHEITSERREGERN

DÉTECTION MULTIPLEX QUANTITATIVE D'AGENTS PATHOGÈNES

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.11.2005 US 735085 P**

(43) Date of publication of application:
**06.08.2008 Bulletin 2008/32**

(73) Proprietor: **PrimeraDx, Inc.**
**Mansfield, MA 02048 (US)**

(72) Inventors:
- **SLEPNEV, Vladimir, I.**
  **Newton, MA 02461 (US)**
- **SHIOSAKI, Kazumi**
  **Wellesley, MA 02481 (US)**
- **HART, Kyle**
  **Belmont, MA 02478 (US)**
- **GARCIA, Elizabeth**
  **Barrington, RI 02906 (US)**

(74) Representative: **Brown, David Leslie**
**Haseltine Lake LLP**
**Redcliff Quay**
**120 Redcliff Street**
**Bristol**
**BS1 6HU (GB)**

(56) References cited:
**WO-A-01/36442**

- **CANDOTTI DANIEL ET AL: "Multiplex real-time quantitative RT-PCR assay for hepatitis B virus, hepatitis C virus, and human immunodeficiency virus type 1" JOURNAL OF VIROLOGICAL METHODS, vol. 118, no. 1, 1 June 2004 (2004-06-01), pages 39-47, XP002548793 ISSN: 0166-0934**
- **CASAS I ET AL: "VIRAL DIAGNOSIS OF NEUROLOGICAL INFECTION BY RT MULTIPLEX PCR: A SEARCH FOR ENTERO- AND HERPESVIRUSES IN A PROSPECTIVE STUDY" JOURNAL OF MEDICAL VIROLOGY, ALAN R. LISS, NEW YORK, NY, vol. 57, no. 2, 1 February 1999 (1999-02-01), pages 145-151, XP009004672 ISSN: 0146-6615**
- **ROBERT PIERRE-YVES ET AL: "Multiplex detection of herpesviruses in tear fluid using the "stair primers" PCR method: Prospective study of 93 patients" JOURNAL OF MEDICAL VIROLOGY, vol. 66, no. 4, April 2002 (2002-04), pages 506-511, XP002548801 ISSN: 0146-6615**
- **BORSON N D ET AL: "Direct quantification of RNA transcripts by competitive single-tube RT-PCR and capillary electrophoresis" BIOTECHNIQUES, INFORMA LIFE SCIENCES PUBLISHING, WESTBOROUGH, MA, US, vol. 25, 1 January 1998 (1998-01-01), pages 130-134,136, XP002259185 ISSN: 0736-6205**
- **MERCIER BERNARD ET AL: "Simultaneous screening for HBV DNA and HCV RNA genomes in blood donations using a novel TaqMan PCR assay" JOURNAL OF VIROLOGICAL METHODS, ELSEVIER BV, NL, vol. 77, no. 1, 1 January 1999 (1999-01-01), pages 1-9, XP002193782 ISSN: 0166-0934**

- KIMURA H ET AL: "Quantitative analysis of Epstein-Barr virus load by using a real-time PCR assay." JOURNAL OF CLINICAL MICROBIOLOGY JAN 1999, vol. 37, no. 1, January 1999 (1999-01), pages 132-136, XP002548794 ISSN: 0095-1137
- MARTELL M ET AL: "High-throughput real-time reverse transcription-PCR quantitation of hepatitis C virus RNA." JOURNAL OF CLINICAL MICROBIOLOGY FEB 1999, vol. 37, no. 2, February 1999 (1999-02), pages 327-332, XP002548795 ISSN: 0095-1137
- ODIN ELISABETH ET AL: "Rapid method for relative gene expression determination in human tissues using automated capillary gel electrophoresis and multicolor detection" JOURNAL OF CHROMATOGRAPHY B, vol. 734, no. 1, 29 October 1999 (1999-10-29), pages 47-53, XP002548796 ISSN: 0378-4347
- SANCHEZ-VEGA B ET AL: "TAQMAN REAL-TIME PCR COMBINED WITH CAPILLARY ELECTROPHORESIS: A NOVEL AND RAPID APPROACH THAT ALLOWS QUANTIFICATION AND ACCURATE SIZE DETERMINATION OF BCL-2/JH FUSION SEQUENCES" LABORATORY INVESTIGATION, UNITED STATES AND CANADIAN ACADEMY OF PATHOLOGY, BALTIMORE, US, vol. 82, no. 1, SUPPL, 1 January 2002 (2002-01-01), page 345A, XP008062325 ISSN: 0023-6837
- ELNIFRO ET AL.: 'Multiplex PCR: Optimization and application in diagnostic virology' CLINICAL MICROBIOLOGY REVIEWS vol. 13, no. 4, October 2000, pages 559 - 570, XP002391196

**Description**

**FIELD OF THE INVENTION**

The invention relates to methods and compositions for quantitative testing in a sample for two or more viral, bacterial or protozoan pathogens contemporaneously. More specifically, the invention relates to methods and compositions for quantitative testing in a sample from an individual to detect and/or monitor pathogen infection quantitatively.

**BACKGROUND OF THE INVENTION**

Immune deficiency may result from many different etiologies including hereditary genetic abnormalities (e.g., Chediak-Higashi Syndrome, Severe Combined Immunodeficiency, Chronic Granulomatous Disease, DiGeorge Syndrome) exposure to radiation, chemotherapy, heavy metals or insecticides; or, acquired as a result of bacterial, viral (HIV), parasitic or fungal infection.

In organ transplant surgery, particularly kidney, liver, heart, lung and bone marrow transplant surgery, it is necessary to suppress the immune system of the graft recipient to minimize the likelihood of graft rejection after surgery. Various immunosuppressive therapies are used and have been proposed for this purpose. However, the immunosuppressive therapies need to be carefully monitored because they can cause the recipient to be particularly susceptible to infection by bacteria and viruses that otherwise would be controlled by a normal immune system. Immunosuppressive agents that have been used successfully in clinical practice include steroids, azathioprine and cyclosporin A. It is necessary in clinical practice to attempt to balance the degree of immunosuppression necessary to prevent or treat graft rejection episodes with the retention of a certain amount of the recipient's immune system to combat other infectious agents.

SUMMARY OF THE INVENTION

Disclosed herein are methods for identifying and determining the amount of two or more pathogens in an individual patient, including asymptomatic patients and patients who are immunocompromized and asymptomatic with respect to the pathogenic disease(s) of interest, in order to monitor disease emergence and/ or disease progression.

In one aspect, the methods disclosed herein permit identifying the presence and/or the amount of two or more target polynucleotides, e.g., DNAs or RNAs, specific for and prepared or isolated from two or more pathogens, particularly viral, bacterial, and protozoan pathogens, as well as fungal pathogens, which may be present in a given biological sample

The methods permit the detection and quantitation of pathogen specific target nucleic acids, e.g., DNAs or RNAs in a nucleic acid sample, both singly and in a multiplex format, that can further permit the determination of levels (e.g., expression levels or copy numbers) for two or more target nucleic acids in a single reaction. Identification and quantification of pathogen specific target in clinical samples have myriad clinical uses, including closely monitoring patients having a compromised immune system.

In one aspect, the methods described herein use internal standards generated through the use of various known concentrations of exogenously added competitor nucleic acids that generate amplification products of known sizes that differ from each other and from the size of the target nucleic acid(s). Size separation by, for example, capillary electrophoresis, coupled with detection by, for example, fluorescence detection, generates a standard curve from the abundance of the amplification products corresponding to the competitor nucleic acids. The standard curve permits the determination of the target nucleic acid concentration(s) in the original sample.

In one aspect, the methods described herein relate to methods of estimating or determining the level of a pathogen specific target nucleic acid, e.g., a DNA or RNA in a nucleic acid sample, the method comprising: for a given pathogen specific target nucleic acid, selecting a pair of amplification primers that will generate a target amplicon of known length upon amplification of the target, e.g., by PCR or RT-PCR. A set of at least two competitor nucleic acids (e.g., DNA or RNA molecules) is generated, where the competitors yield products of differing lengths but similar amplification efficiencies relative to the target nucleic acid when amplified using the same pair of amplification primers. An amplification reaction is performed in which a sample to be analyzed for target nucleic acid level is mixed with known and differing concentrations of the at least two competitor nucleic acids, followed by separation and detection of the amplified products. The set of competitor nucleic acids provides an internal reference for the determination of target nucleic acid amount in the original sample. This approach is readily adapted to measure multiple pathogen specific target nucleic acids in a single sample in a single run, which permits the generation of an amplification profile for the selected pathogen target gene sequences in a given sample. The profile permits accurate quantitation of the level of pathogen-specific nucleic acid in a given sample.

In one aspect, methods described herein relate to the detection of selected pathogens in pre-symptomatic immunocompromized patients. Since development of clinical symptoms is delayed in immunocompromized patients, particularly transplant recipients undergoing immunosuppressant therapy, quantitative detection of viral, bacterial and protozoan pathogens provides one way to guide anti-infective treatment at early stages of infection, by modulation of administration of immunosuppressive therapies (those designed for immunosupression and those having immunosuppressive side effects) and administration of antipathogenic agents (e.g., antiviral agents, antibiotics, antifungals) where treatment is likely to be the most effective.

In another aspect, the methods for analyzing a sample suspected of containing any of a plurality of predetermined pathogens by screening a sample for a plurality of pathogen specific targets to be used in a nucleic acid amplification reaction to produce an amplicon from each pathogen specific target. The methods include selecting a series of pathogen-specific primer pairs wherein each primer pair corresponds to and is targeted to nucleic acid sequences specific to a corresponding pathogen. The series of pathogen-specific primers when used together produce amplicons of distinct sizes such that the presence of a specific pathogen in the sample. Amplicons are detected by resolving a portion of the amplification mixture to determine if amplicons are present, and is so, their size. Portions of the sample may be collected throughout the amplification reaction to determine when amplicons are first present, or at the end of the amplification reaction.

In a further aspect, the methods for quantitating a plurality of predetermined pathogens in a sample suspected of containing at least one pathogen. The methods include obtaining a sample suspected of containing at least one of the predetermined pathogens. The sample may be obtained from the environment (e.g., soil, water, animal or human waste) or from a plant, animal, frozen tissue banks, or human source (e.g., a pathogen carrier or host). Nucleic acids are isolated from the sample for use as a template in an amplification reaction. Pathogen specific primers are selected to correspond to each of the plurality of pathogens suspected of being present in the sample. Control polynucleotides, preferably competitor polynucleotides, are also included in the amplification reaction. The competitor polynucleotides are templates for amplification by pathogen-specific primers, but produce amplicons of a distinct size from the products amplified from the sample nucleic acid using the same or any other pathogen-specific primers with sample or control templates. Competitor polynucleotides are added at specific concentrations (i.e., copy numbers) to allow for determination of the quantity (i.e., copy number) of a pathogen-specific nucleic acid. The quantity of a pathogen in a sample may be below the detection limit of the method or none.

In an aspect, the methods include monitoring of a series of samples from the same source for any of a predetermined plurality of pathogens. The methods include obtaining a sample from a source at regular intervals (e.g., about weekly, about monthly, about quarterly) and quantitating the amount of the plurality of pathogens in the sample using an amplification method with competitor polynucleotides. A source can be an immunocompromised individual who are frequently asymptomatic despite infection. By quantitating the amount of a plurality of pathogens at regular intervals, pathogens may be detected in the asymptomatic individual and appropriate measures can be taken, such as modification of administration of compositions that result in immunosupression of the individual or administration of a therapy to ameliorate and/or treat the pathogen infection.

DEFINITIONS

As used herein, the term "prepared or isolated from" when used in reference to a nucleic acid "prepared or isolated from" a pathogen refers to both nucleic acid isolated from a virus or other pathogen, and to nucleic acid that is copied from a virus, e.g., by a process of reverse-transcription or DNA polymerization using the viral nucleic acid as a template. The nucleic acid of the pathogen may be isolated from a sample in conjunction with host nucleic acid.

As used herein the term "pathogen" refers to an organism, including a microorganism, which causes disease in another organism (e.g., animals and plants) by directly infecting the other organism, or by producing agents that causes disease in another organism (e.g., bacteria that produce pathogenic toxins and the like). As used herein, pathogens include, but are not limited to bacteria, protozoa, fungi, nematodes, viroids and viruses, or any combination thereof, wherein each pathogen is capable, either by itself or in concert with another pathogen, of eliciting disease in vertebrates including but not limited to mammals, and including but not limited to humans. As used herein, the term "pathogen" also encompasses microorganisms which may not ordinarily be pathogenic in a non-immunocompromised host. Specific nonlimiting examples of viral pathogens include HSV1, HSV2, EBV, CMV, HHV 6, HHV7, HHV8, VZV, hepatitis C, hepatitis B, adenovirus, EEEV, WNE, JCV and BKV.

As used herein, the term "microorganism" includes prokaryotic and eukaryotic microbial species from the Domains of Archaea, Bacteria and Eucarya, the latter including yeast and filamentous fungi, protozoa, algae, or higher Protista. The terms "microbial cells" and "microbes" are used interchangeably with the term microorganism.

"Bacteria", or "Eubacteria", refers to a domain of prokaryotic organisms. Bacteria include at least 11 distinct groups as follows: (1) Gram-positive (gram+) bacteria, of which there are two major subdivisions: (i) high G+C group (Actinomycetes, Mycobacteria, Micrococcus, others) (ii) low G+C group (Bacillus, Clostridia, Lactobacillus, Staphylococci, Streptococci, Mycoplasmas); (2) Proteobacteria, e.g., Purple photosynthetic+non-photosynthetic Gram-negative bacteria (includes most "common" Gram-negative bacteria); (3) Cyanobacteria, e.g., oxygenic phototrophs; (4) Spirochetes and related species; (5) Planctomyces; (6) Bacteroides, Flavobacteria; (7) Chlamydia; (8) Green sulfur bacteria; (9) Green non-sulfur bacteria (also anaerobic phototrophs); (10) Radioresistant micrococci and relatives; (11) Thermotoga and Thermosipho thermophiles.

"Gram-negative bacteria" include cocci, nonenteric rods, and enteric rods. The genera of Gram-negative bacteria include, for example, Neisseria, Spirillum, Pasteurella, Brucella, Yersinia, Francisella, Haemophilus, Bordetella, Escherichia, Salmonella, Shigella, Klebsiella, Proteus, Vibrio, Pseudomonas, Bacteroides, Acetobacter, Aerobacter, Agrobacterium, Azotobacter, Spirilla, Serratia, Vibrio, Rhizobium, Chlamydia, Rickettsia, Treponema, and Fusobacterium.

"Gram-positive bacteria" include cocci, nonsporulating rods, and sporulating rods. The genera of Gram-positive bacteria include, for example, Actinomyces, Bacillus, Clostridium, Corynebacterium, Erysipelothrix, Lactobacillus, Listeria, Mycobacterium, Myxococcus, Nocardia, Staphylococcus, Streptococcus, and Streptomyces.

As used herein, the term "detection" refers to the qualitative determination of the presence or absence of a microorganism in a sample. The term "detection" also includes the "identification" of a microorganism, i.e., determining the genus, species, or strain of a microorganism according to recognized taxonomy in the art and as described in the present specification. The term "detection" further includes the quantitation of a microorganism in a sample, e.g., the copy number of the microorganism in a microliter (or a milliter or a liter) or a microgram (or a milligram or a gram or a kilogram) of a sample.

As used herein, the term "immunocompromised patient or individual" refers to an individual who is at risk for developing infectious diseases, because the immune system of the individual is not working at optimum capacity. In one aspect, the individual is immunocompromised due to a treatment regimen designed, for example, to prevent inflammation or to prevent rejection of a transplant.

As used herein, the term "sample" refers to a biological material which is isolated from its natural environment and contains a polynucleotide. A sample according to the methods described herein, may consist of purified or isolated polynucleotide, or it may comprise a biological sample such as a tissue sample, a biological fluid sample, or a cell sample comprising a polynucleotide. A biological fluid includes, but is not limited to, blood, plasma, sputum, urine, cerebrospinal fluid, lavages, and leukophoresis samples, for example. A sample may also be an environmental sample such as soil, water, or animal or human waste to detect the presence of a pathogen in an area where an outbreak of disease related to a specific pathogen has occurred. A sample may also be obtained from a tissue bank or other source for the analysis of archival samples or to test tissues prior to transplantation. A sample useful in the method described herein may be any plant, animal, bacterial or viral material containing a polynucleotide, or any material derived there from.

A sample is "suspected of containing at least one of a plurality of predefined pathogens" for any of a number of reasons. For example, a soil sample may be suspected of containing a pathogen if humans or animals living close to the location where the soil sample was collected show symptoms of a condition or diseases associated with a soil pathogen. Alternatively, an immunosuppressed individual or individual otherwise susceptible to infection may be suspected of being a host or carrier of a pathogen without showing overt signs of infection. Samples taken from such an individual may be suspected of containing at least one of a plurality of pathogens, even in the absence of infection.

As used herein, the term "amplicon" refers to an amplification product from a nucleic acid amplification reaction. The term generally refers to an anticipated, specific amplification product of known size, generated using a given set of amplification primers.

As used herein, the term "reverse transcript" refers to a DNA complement of an RNA strand generated by an RNA-dependent DNA polymerase activity.

As used herein, the term "competitor polynucleotide" or "nucleic acid competitor" refers to a nucleic acid template of known length and composition that can be amplified using a pair of oligonucleotide primers selected for the amplification of a target nucleic acid. In certain embodiments, the competitor nucleic acid can be an RNA molecule, in which case it can be referred to as a "competitor RNA" or an "RNA competitor." In other embodiments, the competitor nucleic acid can be a DNA molecule, in which case it can be referred to as a "competitor DNA" or a "DNA competitor." A "competitor nucleic acid" (whether DNA or RNA) will produce an amplicon that is longer or shorter than the amplicon produced from the target nucleic acid, e.g., by a known, distinguishable length, e.g., the length of an internal insertion or deletion in the target nucleic acid, respectively. The internal insertion or deletion should be from 1 to 20 nucleotides or bases, preferably 5 to 20 nucleotides or bases, or 5 to 10 nucleotides or bases. The difference in length of the target and competitor amplicons will be from 1 to 20 nucleotides in length, preferably 5 to 20 or 5 to 10 nucleotides in length. Inserted sequence will preferably not introduce the capacity for stable secondary structure not present in the target sequence. Software for predicting nucleic acid secondary structure is well known in the art. A "competitor polynucleotide" will have an amplification efficiency that is similar to that of the target nucleic acid when using a selected pair of amplification primers.

As used herein, the term "similar efficiency" when applied to nucleic acid amplification, means that the threshold cycle (Ct) for the detection of target and competitor nucleic acid amplification products generated using the same set of primers and equal amounts of target and competitor template is the same. It is possible to calculate Ct to a fraction of a cycle. However, the Ct for one amplicon is "the same" as the Ct for another amplicon when the whole cycle numbers are the same-i.e., Ct's of 2.0, 2.3 and 2.6 are "the same" as the term is used herein. As used herein, "Ct" is the PCR cycle at which at which signal intensity of PCR product reaches a threshold value of 10 standard deviations of background value of signal intensity for an amplified product. Signal intensity in this context refers to fluorescent signal from amplification product incorporating fluorescent label (either by labeled primer or labeled nucleotide incorporation), measured following capillary electrophoresis of amplified products present in samples withdrawn from a cycling reaction at a plurality of cycle points. Another measure of amplification efficiency is to measure the amount of amplification product (e.g., by fluorescence integrity or label incorporation) at successive cycles, calculating efficiency using the formula $E=(P_{n+1}-P_n) / (P_n-P_{n-1})$, where P=the amount of amplification product at cycle n. Amplification efficiency is "similar" if the difference in efficiency between target and competitor nucleic acid is less than 0.2 in absolute value.

In the methods described herein, efficiency is "similar" if the efficiency of amplification of target and competitor nucleic acid is "similar" by either of these criteria, and preferably, by both.

Primer pair "capable of mediating amplification" is understood as a primer pair that is specific to the target, has an appropriate melting temperature, and does not include excessive secondary structure. Guidelines for designing primer pairs capable of mediating amplification are provided herein.

"Conditions that promote amplification" as used herein are the conditions for amplification provided by the manufacturer for the enzyme used for amplification. It is understood that an enzyme may work under a range of conditions (e.g., ion concentrations, temperatures, enzyme concentrations). It is also understood that multiple temperatures may be required for amplification (e.g., in PCR). Conditions that promote amplification need not be identical for all primers and targets in a reaction, and reactions may be carried out under suboptimal conditions where amplification is still possible.

As used herein, the term "aliquot" refers to a sample volume taken from an amplification reaction mixture. The volume of an aliquot can vary, but will generally be constant within a given experimental run. An aliquot will be less than the volume of the entire reaction mixture. Where there are X aliquots to be withdrawn during an amplification regimen, the volume of an aliquot will be less than or equal to 1/X times the reaction volume.

As used herein, the term "dispense" means dispense, transfer, withdraw, extrude or remove.

As used herein, the phrase "dispensing an aliquot from the reaction mixture at plural stages" refers to the withdrawal of an aliquot at least twice, and preferably at least about 3, 4, 5, 10, 15, 20, 30 or more times during an amplification reaction. A "stage" will refer to a point at or after a given number of cycles, or, where the amplification regimen is non-cyclic, will refer to a selected time at or after the initiation of the reaction.

As used herein, "separating" or the "separation of" nucleic acids in a sample refers to a process whereby nucleic acid fragments are separated by size. The method of separating should be capable of resolving nucleic acid fragments that differ in size by 10 nucleotides or less (or, alternatively, by 10 base pairs or less, e.g., where non-denaturing conditions are employed). Preferred resolution for separation techniques employed in the methods described herein includes resolution of nucleic acids differing by 5 nucleotides or less (alternatively, 5 base pairs or less), up to and including resolution of nucleic acids differing by only one nucleotide (or one base pair).

As used herein, reference to a "size distinguishable by capillary electrophoresis" means a difference of at least one nucleotide (or base pair), but preferably at least 5 nucleotides (or base pairs) or more, up to and including 10 nucleotides (or base pairs) or more. As used herein, the term "distinct from" when used in reference to the length of a polynucleotide means that the length of the polynucleotide is distinguishable from the length of another by capillary electrophoresis.

As used herein, the term "amplified product" refers to polynucleotides that are copies of a particular polynucleotide, produced in an amplification reaction. An "amplified product," according to the invention, may be DNA or RNA, and it may be double-stranded or single-stranded. An amplified product is also referred to herein as an "amplicon".

As used herein, the term "amplification" or "amplification reaction" refers to a reaction for generating a copy of a particular polynucleotide sequence or increasing the copy number or amount of a particular polynucleotide sequence. For example, polynucleotide amplification may be a process using a polymerase and a pair of oligonucleotide primers for producing any particular polynucleotide sequence, i.e., the whole or a portion of a target polynucleotide sequence, in an amount that is greater than that initially present. Amplification may be accomplished by the in vitro methods of the polymerase chain reaction (PCR). See generally, PCR Technology: Principles and Applications for DNA Amplification (H. A. Erlich, Ed.) Freeman Press, NY, NY (1992); PCR Protocols : A Guide to Methods and Applications (Innis et al., Eds.) Academic Press, San Diego, CA (1990); Mattila et al., Nucleic Acids Res. 19 : 4967 (1991); Eckert et al., PCR Methods and Applications 1 : 17 (1991); PCR (McPherson et al. Ed.), IRL Press, Oxford; and U. S. Patent Nos. 4,683,202 and 4,683,195. Other amplification methods include, but are not limited to: (a) ligase chain reaction (LCR) (see Wu and Wallace, Genomics 4 : 560 (1989) and Landegren et al., Science 241 : 1077 (1988)); (b) transcription amplification (Kwoh et al., Proc. Natl. Acad. Sci. USA 86 : 1173 (1989)); (c) self-sustained sequence replication (Guatelli et al., Proc. Natl. Acad. Sci. USA, 87 : 1874 (1990)); and (d) nucleic acid based sequence amplification (NABSA) (see, Sooknanan, R. and Malek, L., Bio Technology 13 : 563-65 (1995)).

As used herein, a "target polynucleotide" (including, e.g., a target RNA or target DNA) is a polynucleotide to be analyzed. A target polynucleotide may be isolated or amplified before being analyzed using methods of the present invention. For example, the target polynucleotide may be a sequence that lies between the hybridization regions of two members of a pair of oligonucleotide primers that are used to amplify it. A target polynucleotide may be RNA or DNA (including, e.g., cDNA). A target polynucleotide sequence generally exists as part of a larger "template" sequence; however, in some cases, a target sequence and the template are the same.

As used herein, a "pathogen specific target polynucleotide" is a target polynucleotide as defined above, wherein the target polynucleotide is which is prepared or isolated from a pathogen of interest, and which is present in only one member of the group of different pathogens that are being analyzed.

As used herein, an "oligonucleotide primer" refers to a polynucleotide molecule (i.e., DNA or RNA) capable of annealing to a polynucleotide template and providing a 3' end to produce an extension product that is complementary to the polynucleotide template. The conditions for initiation and extension usually include the presence of four different deoxyribonucleoside triphosphates (dNTPs) and a polymerization-inducing agent such as a DNA polymerase or reverse transcriptase activity, in a suitable buffer ("buffer" includes substituents which are cofactors, or which affect pH, ionic strength, etc.) and at a suitable temperature. The primer as described herein may be single- or double-stranded. The primer is preferably single-stranded for maximum efficiency in amplification. "Primers" useful in the methods described herein are less than or equal to 100 nucleotides in length, e.g., less than or equal to 90, or 80, or 70, or 60, or 50, or 40, or 30, or 20, or 15, but preferably longer than 10 nucleotides in length.

As used herein, "label" or "detectable label" refers to any moiety or molecule that can be used to provide a detectable (preferably quantifiable) signal. A "labeled nucleotide" (e.g., a dNTP), or "labeled polynucleotide", is one linked to a detectable label. The term "linked" encompasses covalently and non-covalently bonded, e.g., by hydrogen, ionic, or Van der Waals bonds. Such bonds may be formed between at least two of the same or different atoms or ions as a result of redistribution of electron densities of those atoms or ions. Labels may provide signals detectable by fluorescence, radioactivity, colorimetry, gravimetry, X-ray diffraction or absorption, magnetism, enzymatic activity, mass spectrometry, binding affinity, hybridization radiofrequency, nanocrystals and the like. A nucleotide useful in the methods described herein can be labeled so that the amplified product may incorporate the labeled nucleotide and becomes detectable. A fluorescent dye is a preferred label according to the present invention. Suitable fluorescent dyes include fluorochromes such as Cy5, Cy3, rhodamine and derivatives (such as Texas Red), fluorescein and derivatives (such as 5-bromomethyl fluorescein), Lucifer Yellow, IAEDANS, 7-$Me_2$N-coumarin-4-acetate, 7-OH-4-$CH_3$-coumarin-3-acetate, 7-$NH_2$-4-$CH_3$-coumarin-3-acetate (AMCA), monobromobimane, pyrene trisulfonates, such as Cascade Blue, and monobromorimethyl-ammoniobimane (see for example, DeLuca, Immunofluorescence Analysis, in Antibody As a Tool, Marchalonis, et al., eds., John Wiley & Sons, Ltd., (1982).

It is intended that the term "labeled nucleotide", as used herein, also encompasses a synthetic or biochemically derived nucleotide analog that is intrinsically fluorescent, e.g., as described in U.S. Patent Nos. 6,268,132 and 5,763,167, Hawkins et al. (1995, Nucleic Acids Research, 23 : 2872-2880), Seela et al. (2000, Helvetica Chimica Acta, 83 : 910-927), Wierzchowski et al. (1996, Biochimica et Biophysica Acta, 1290 : 9-17), Virta et al. (2003, Nucleosides, Nucleotides & Nucleic Acids, 22 : 85-98). By "intrinsically fluorescent", it is meant that the nucleotide analog is spectrally unique and distinct from the commonly occurring conventional nucleosides in their capacities for selective excitation and emission under physiological conditions. For the intrinsically fluorescent nucleotides, the fluorescence typically occurs at wavelengths in the near ultraviolet through the visible wavelengths. Preferably, fluorescence will occur at wavelengths between 250 nm and 700 nm and most preferably in the visible wavelengths between 250 nm and 500 nm.

The term "detectable label" or "label" include a molecule or moiety capable of generating a detectable signal, either by itself or through the interaction with another label. The " label" may be a member of a signal generating system, and thus can generate a detectable signal in context with other members of the signal generating system, e.g., a biotin-avidin signal generation system, or a donor-acceptor pair for fluorescent resonance energy transfer (FRET) (Stryer et al., 1978, Ann. Rev. Biochem., 47:819; Selvin, 1995, Methods Enzymol., 246:300) or a nucleic acid - binding dye, producing detectable signal upon binding to nucleic acid (DNA or RNA molecule).

The term "nucleotide" or "nucleic acid" as used herein, refers to a phosphate ester of a nucleoside, e.g., mono, di, tri, and tetraphosphate esters, wherein the most common site of esterification is the hydroxyl group attached to the C-5 position of the pentose (or equivalent position of a non-pentose "sugar moiety"). The term "nucleotide" includes both a conventional nucleotide and a non-conventional nucleotide which includes, but is not limited to, phosphorothioate, phosphite, ring atom modified derivatives, and the like, e.g., an intrinsically fluorescent nucleotide.

As used herein, the term "conventional nucleotide" refers to one of the "naturally occurring" deoxynucleotides (dNTPs), including dATP, dTTP, dCTP, dGTP, dUTP, and dITP.

As used herein, the term "non-conventional nucleotide" refers to a nucleotide which is not a naturally occurring nucleotide. The term "naturally occurring" refers to a nucleotide that exists in nature without human intervention. In contradistinction, the term "non-conventional nucleotide" refers to a nucleotide that exists only with human intervention. A "non-conventional nucleotide" may include a nucleotide in which the pentose sugar and/or one or more of the phosphate esters is replaced with a respective analog. Exemplary pentose sugar analogs are those previously described in conjunction with nucleoside analogs. Exemplary phosphate ester analogs include, but are not limited to, alkylphosphonates, methylphosphonates, phosphoramidates, phosphotriesters, phosphorothioates, phosphorodithioates, phosphoroselenoates, phosphorodiselenoates, phosphoroanilothioates, phosphoroanilidates, phosphoroamidates, boronophosphates, etc., including any associated counterions, if present. A non-conventional nucleotide may show a preference of base pairing with another artificial nucleotide over a conventional nucleotide (e.g., as described in Ohtsuki et al. 2001, Proc. Natl. Acad. Sci., 98 : 4922-4925. The base pairing ability may be measured by the T7 transcription assay as described in Ohtsuki et al. (supra). Other non-limiting examples of "artificial nucleotides" may be found in Lutz et al. (1998) Bioorg. Med. Chem. Lett., 8 : 1149-1152); Voegel and Benner (1996) Helv. Chim. Acta 76, 1863-1880; Horlacher et al. (1995) Proc. Natl. Acad. Sci., 92: 6329-6333; Switzer et al. (1993), Biochemistry 32 : 10489-10496; Tor and Dervan (1993) J. Am. Chem. Soc. 115 : 4461-4467; Piccirilli et al. (1991) Biochemistry 30 : 10350-10356; Switzer et al. (1989) J. Am. Chem. Soc. 111 : 8322-8323.A "non-conventional nucleotide" may also be a degenerate nucleotide or an intrinsically fluorescent nucleotide.

As used herein, the term "degenerate nucleotide" denotes a nucleotide that may be any of dA, dG, dC, and dT; or may be able to base-pair with at least two bases of dA, dG, dC, and dT. An unlimiting list of degenerate nucleotide which base-pairs with at least two bases of dA, dG, dC, and dT include: Inosine, 5-nitropyrole, 5-nitroindole, hypoxanthine, 6H,8H,4-dihydropyrimido[4,5c][1,2]oxacin-7-one (P), 2-amino-6-methoxyaminopurine, dPTP and 8-oxo-dGTP.

As used herein, the term "opposite orientation", when referring to primers, means that one primer comprises a nucleotide sequence complementary to the sense strand of a target polynucleotide template, and another primer comprises a nucleotide sequence complementary to the antisense strand of the same target polynucleotide template. Primers with an opposite orientation may generate a PCR amplified product from matched polynucleotide template to which they complement. Two primers with opposite orientation may be referred to as a reverse primer and a forward primer.

As used herein, the term "same orientation", means that primers comprise nucleotide sequences complementary to the same strand of a target polynucleotide template. Primers with same orientation will not generate a PCR amplified product from matched polynucleotide template to which they complement.

As used herein, a "polynucleotide" or "nucleic acid" generally refers to any polyribonucleotide or poly-deoxyribonucleotide, which may be unmodified RNA or DNA or modified RNA or DNA. "Polynucleotides" include, without limitation, single- and double-stranded polynucleotides. The term "polynucleotides" as it is used herein embraces chemically, enzymatically or metabolically modified forms of polynucleotides, as well as the chemical forms of DNA and RNA characteristic of viruses and cells, including for example, simple and complex cells. A polynucleotide useful for the present invention may be an isolated or purified polynucleotide or it may be an amplified polynucleotide in an amplification reaction.

As used herein, the term "set" refers to a group of at least two. Thus, a "set" of oligonucleotide primers comprises at least two oligonucleotide primers. In one aspect, a "set" of oligonucleotide primers refers to a group of primers sufficient to specifically amplify a nucleic acid amplicon from each member of a plurality of target pathogens - generally, there will be a pair of oligonucleotide primers for each member of said plurality, (it is noted that these primer pairs will, in some aspects, also be used to amplify one or more competitor or internal standard templates).

As used herein, the term "pair" refers to two. Thus, a "pair" of oligonucleotide primers are two oligonucleotide primers. When a "pair" of oligonucleotide primers are used to produce an extended product from a double-stranded template (e.g., genomic DNA or cDNA), it is preferred that the pair of oligonucleotide primers hybridize to different stand of the double-stranded template, i.e., they have opposite orientations.

As used herein, "isolated" or "purified" when used in reference to a polynucleotide means that a naturally occurring sequence has been removed from its normal cellular environment or is synthesized in a non-natural environment (e.g., artificially synthesized). Thus, an "isolated" or "purified" sequence may be in a cell-free solution or placed in a different cellular environment. The term "purified" does not imply that the sequence is the only nucleotide present, but that it is essentially free (about 90-95%, up to 99-100% pure) of non-nucleotide or polynucleotide material naturally associated with it.

As used herein, the term "cDNA" refers to complementary or copy polynucleotide produced from an RNA template by the action of an RNA-dependent DNA polymerase activity (e.g., reverse transcriptase).

As used herein, "complementary" refers to the ability of a single strand of a polynucleotide (or portion thereof) to hybridize to an anti-parallel polynucleotide strand (or portion thereof) by contiguous base-pairing between the nucleotides (that is not interrupted by any unpaired nucleotides) of the anti-parallel polynucleotide single strands, thereby forming a double-stranded polynucleotide between the complementary strands. A first polynucleotide is said to be "completely complementary" to a second polynucleotide strand if each and every nucleotide of the first polynucleotide forms base-paring with nucleotides within the complementary region of the second polynucleotide. A first polynucleotide is not completely complementary (i.e., partially complementary) to the second polynucleotide if one nucleotide in the first polynucleotide does not base pair with the corresponding nucleotide in the second polynucleotide. The degree of complementarity between polynucleotide strands has significant effects on the efficiency and strength of annealing or hybridization between polynucleotide strands. This is of particular importance in amplification reactions, which depend upon binding between polynucleotide strands.

An oligonucleotide primer is "complementary" to a target polynucleotide if at least 50% (preferably, 60%, more preferably 70%, 80%, still more preferably 90% or more) nucleotides of the primer form base-pairs with nucleotides on the target polynucleotide.

As used herein, the term "analyzing," when used in the context of an amplification reaction, refers to a qualitative (i.e., presence or absence, size detection, or identity etc.) or quantitative (i.e., amount) determination of a target polynucleotide, which may be visual or automated assessments based upon the magnitude (strength) or number of signals generated by the label. The "amount" (e.g., measured in ug, umol or copy number) of a polynucleotide may be measured by methods well know in the art (e.g., by UV absorption or fluorescence intensity, by comparing band intensity on a gel with a reference of known length and amount), for example, as described in Basic Methods in Molecular Biology, (1986, Davis et al., Elsevier, NY); and Current Protocols in Molecular Biology (1997, Ausubel et al., John Weley & Sons, Inc. ). One way of measuring the amount of a polynucleotide in the present invention is to measure the fluorescence intensity emitted by such polynucleotide, and compare it with the fluorescence intensity emitted by a reference polynucleotide, i.e., a polynucleotide with a known amount.

As used herein, "cancer therapy" refers to any therapy that has as a goal to reduce the severity of a cancer or to at least partially eliminate a cancer. Alternatively, "cancer therapy" refers to any therapy that has as a goal to reduce or to at least partially eliminate metastasis of a cancer. As a further alternative, cancer therapy refers to any therapy which has as its goal to reduce or at least partially eliminate growth of metastatic nodules (e.g., after surgical removal of a primary tumor). Alternatively stated, cancer therapy refers to any therapy which has as its goal to slow, control, decrease the likelihood or probability, or delay the onset of cancer in the subject.

As used herein, the term "cancer" has its understood meaning in the art, for example, an uncontrolled growth of tissue and/or cells, which has the potential to spread to distant sites of the body (i.e., metastasize). Exemplary cancers include, but are not limited to, leukemias, lymphomas, colon cancer, renal cancer, liver cancer, breast cancer, lung cancer, prostate cancer, ovarian cancer, melanoma, and the like.

As used herein, the term "graft" refers to a body part, organ, tissue, cell, or portion thereof, that is transplanted from one individual to another individual. The graft can be for example, a xenogeneic, allogeneic, genetically engineered syngeneic, or genetically engineered autologous graft.

As used herein, the term "capillary electrophoresis" means the electrophoretic separation of nucleic acid molecules in an aliquot from an amplification reaction wherein the separation is performed in a capillary tube. Capillary tubes are available with inner diameters from about 10 to 300 um, and can range from about 0.2 cm to about 3 m in length, but are preferably in the range of 0.5 cm to 20 cm, more preferably in the range of 0.5 cm to 10 cm. In addition, the use of microfluidic microcapillaries (available, e.g., from Caliper or Agilent Technologies) is specifically encompassed within the meaning of "capillary electrophoresis".

As used herein, an "immunosuppressive drug" refers to an agent that reduces the ability of the immune system to mount an effective response against pathogens. For example, a drug, which, when administered at an appropriate dosage, results in the inactivation of thymic or lymph node T cells. Non-limiting examples of such agents are corticosteroids, cyclosporine, FK-506, and rapamycin.

As used herein, the term"aymptomatic" refers to an individual who does not exhibit physical symptoms characteristic of being infected with a given pathogen, or a given combinations of pathogens.

As used herein, "a plurality of' or "a set of' refers to more than two, for example, 3 or more, 4 or more, 5 or more, 6 or more, 7 or more, 8 or more, 9 or more 10 or more etc.

**BRIEF DESCRIPTION OF THE FIGURES**

Figure 1 shows a chart of Genbank accession numbers for representative viruses encompassed by the methods described herein.

Figure 2 is a representative example of an electrophoregram for an assay to simultaneously detect six viral pathogens. Amplified DNA fragments (i.e., amplicons) corresponding to the indicated viruses CMV (cytomegalovirus), BK (BK virus, a human polyoma virus), JC (JC virus, a human polyoma virus), HHV6 (human herpes virus 6), HHV7 (human herpes virus 7), and EBV (Epstein Barr virus) were separated on a 36cm capillary array using an ABI 3730 Genetic Analyzer System.

Figure 3 is a representative example of amplification plots for an assay to detect the same six viral pathogens as in Figure 2. Each of the viruses at the number of copies indicated was introduced into a reaction mixture containing fluorescently labeled primers to allow for real time analysis. Portions of the amplification mixture were removed at the end of the cycles indicated and resolved by capillary electrophoresis. The relative fluorescence units (log peak area) are plotted on a log scale versus cycle number.

Figure 4 is a representative example of a series of calibration plots that show the cycle threshold (Ct) for detection of a given copy number of each viral target.

Threshold cycle number was defined as the cycle number that corresponded to 35000 fluorescence units as calculated by Gene Mapper data analysis software (Applied Biosystems, Foster City, CA).

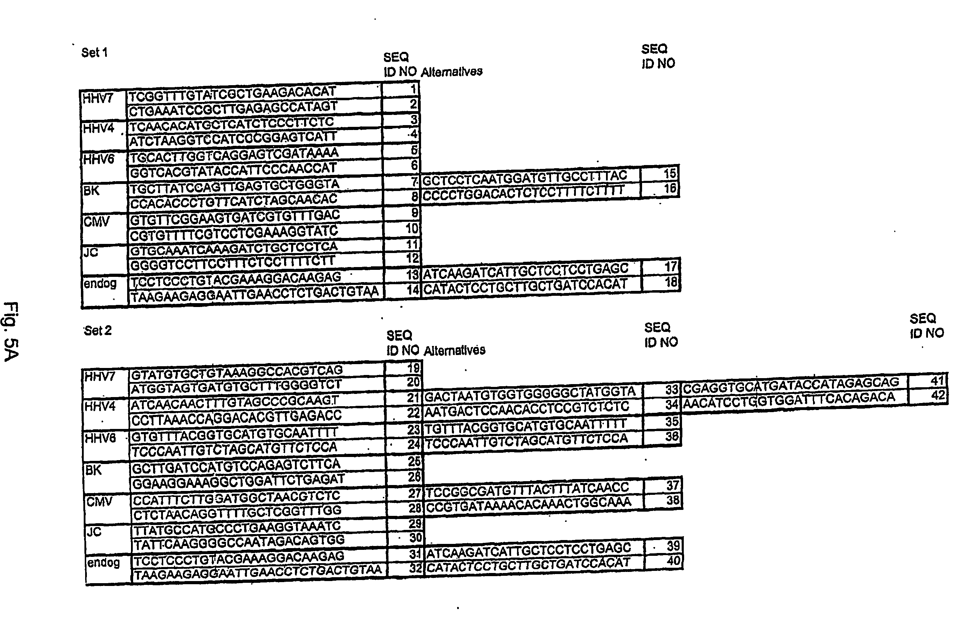

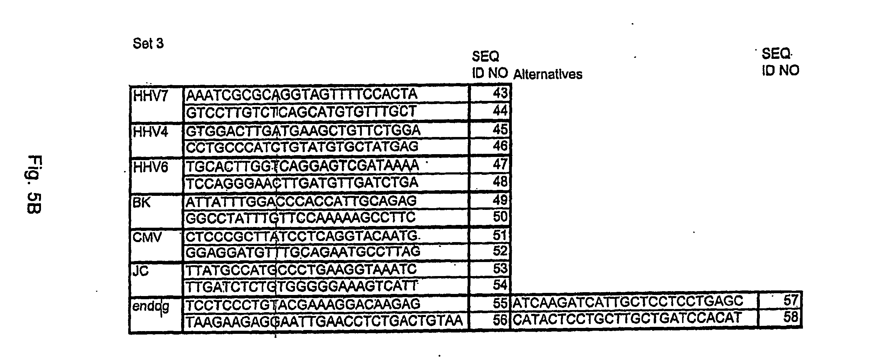

Figure 5 is a table of target specific oligonucleotides for the targetes listed.

## DETAILED DESCRIPTION

Due to the advent of genomics, microorganisms including pathogens, can now be identified based on the presence of microorganism-specific genes or transcripts. Expression patterns at both the transcriptional and protein levels have resulted in additional insights into pathogenicity and potential diagnostic tools.

The methods described herein are directed to an accurate, sensitive and contemporaneous method for the diagnosis and quantitation of multiple types of pathogen infection using a set of oligonucleotides specific for each of the pathogens to be detected, to act as primers to amplify either pathogen transcripts or particular regions of the genome of each specific pathogen sought to be detected in a clinical sample. The pathogen is selected from the group consisting of: virus, bacteria, protozoan, and fungi. Alternatively, the pathogen is selected from the group consisting of: virus, bacteria, and protozoan. Alternatively, the pathogen is selected from the group consisting of: virus and bacteria.

The methods described herein are directed to an accurate, sensitive and contemporaneous method for the diagnosis and quantitation of multiple types of virus infection using a set of oligonucleotides specific for each of the viruses to be detected, to act as primers to amplify either viral transcripts or particular regions of the genome of each specific virus sought to be detected in a clinical sample.

The methods described herein can be applied to the detection of pathogens in samples from any individual. However, because a decrease in immune function leads to an immunocompromised status that can predispose the host to serious and life threatening disease from pathogens, including viral pathogens, it is beneficial to monitor an individual having or suspected of having an immunocompromised status, for the presence of pathogens, including viral pathogens, which may be detrimental to the individual's health. Early detection of pathogens, including viral pathogens, in samples from a patient, particularly in an immunosuppressed patient, provide opportunities for preemptive therapy, including for example, modifying the dose of any immunosuppressive agents being administered to the patient.

Commonly, diagnostic testing for pathogens causing infectious diseases is conducted in patients who present symptoms characacteristic of infection by one or more pathogenic infections, or in persons who have been in contact with individuals having one or more pathogenic infections, or in people who are otherwise suspected to have developed an infectious disease resulting from one or more pathogens

Management of immunocompromised patients, and, in particular, patients undergoing immunosuppressive treatment after graft or tissue transplants, or patients undergoing treatment causing severe depression of the immune system (for example cancer chemotherapy treatment) represent a challenge to the traditional diagnostic paradigm. First, development of clinical symptoms characteristic for infectious disease is delayed in the immunodeficient patients, and typically coincides with later stages of infectious disease and higher pathogen titer when compared with immunocompetent indivuduals. This effect complicates anti-infective treatment, and can result in poorer outcome for a patient. Second, immunosuppressive treatment often results in re-activation of latent infection previously efficiently managed by a healthy immune system. In such situations, a simple detection of pathogen presence is not sufficient and instead, quantitative monitoring of pathogen titer and its changes will be more valuable for patient and medical professionals. In addition, detection of disease progression at the onset or early stages of infection can help to administer effective treatment early on, increasing chances of successful outcome. Also, in a specific example of immunosuppressive therapy of transplant patients, monitoring infectious disease progression could be used to adjust the regiment of immunosuppressive drugs in order to help the immune system combat pathogens while balancing the possibility of transplant rejection.

While quantitative monitoring of pathogens in aymptomatic individuals is not generally practical, it can be very beneficial for patients undergoing immunosuppressive treatment. In particular, monitoring of post-transplantation patients for pathogen infection can improve post-transplantation survival and minimizing transplant rejection. Quantitative pathogen monitoring in a patient is especially practical if applied not as a single test for each specific infection of interest, but if applied as a panel of parallel assays performed on a single sample from a patient or, preferably, as a multiplex assay for a panel of pathogens presenting the highest risk for immunocompromised patient. The pathogens monitored for can be selected based on a number of factors including, but not limited to, the cause of immunosupression in the patient, the environmental factors to which the individual is exposed, and symptoms preseted by the individual. Such considerations are well understood by those skilled in the art.

Such a multiplexed assay can be developed using molecular diagnostics methods, and, in particular, methods using PCR amplification of pathogen-specific nucleic acids.

Methods using PCR to detect and/or quantitate virus in a sample include, for example, Kimura H, et al. Quantitative analysis of Epstein-Barr virus load by using a real-time PCR assay. J. Clin Microbiol. 37:132, 1999; Martell M, et al. High-throughput real-time reverse transcription-PCR quantitation of hepatitis C virus RNA J Clin Microbiol. February 1999; 37(2):327-32; Mercier B, et al. Simultaneous screening for HBV DNA and HCV RNA genomes in blood donations using a novel TaqMan PCR assay. J Virol Methods. January 1999; 77(1):1-9.

PCR methods can comprise exogenous controls such as the use of an artificially introduced nucleic acid molecule of known concentration that is added, either to the extraction step, the reverse transcription strep, or to the PCR step. The concept of adding an exogenous nucleic acid at a known concentration in order to act as an internal standard for quantitation was introduced by Chelly et al. (1988) Nature 333: 858-860. The use of exogenous nucleic acids for internal standards in PCR is described for example, in WO 93/02215; WO 92/11273.; U.S. Patent Nos. 5,213,961 and 5,219,727. Similar strategies have proven effective for quantitative measurement of nucleic acids utilizing isothermal amplification reactions such as NASBA (Kievits et al., 1991, J. Virol. Methods 35: 273-86) or SDA (Walker, 1994, Nucleic Acids Res. 22: 2670-7).

Capillary electrophoresis has been used to quantitatively detect gene expression. Rajevic at el. (2001, Pflugers Arch. 442(6 Suppl 1):R190-2) discloses a method for detecting differential expression of oncogenes by using seven pairs of primers for detecting the differences in expression of a number of oncogenes simultaneously. Sense primers were 5' end-labeled with a fluorescent dye. Multiplex fluorescent RT-PCR results were analyzed by capillary electrophoresis on ABI-PRISM 310 Genetic Analyzer. Borson et al. (1998, Biotechniques 25:130-7) describes a strategy for quantitation of low-abundance mRNA transcripts based on quantitative competitive reverse transcription PCR (QC-RT-PCR) coupled to capillary electrophoresis (CE) for rapid separation and detection of products. George et al., (1997, J Chromatogr B Biomed Sci Appl 695:93-102) describes the application of a capillary electrophoresis system (ABI 310) to the identification of fluorescent differential display generated EST patterns. Odin et al. (1999, J Chromatogr B Biomed Sci Appl 734:47-53) describes an automated capillary gel electrophoresis with multicolor detection for separation and quantification of PCR-amplified cDNA.

In addition to nucleic acid based detection, multiplexed detection of virus, bacteria and/or protozoa can be achieved using virus, bacteria and/or protozoa specific markers. These markers include proteins, carbohydrates or lipids that are specific to each of the virus, bacteria and/or protozoa to be detected. Although the methods are useful for the detection of the presence of pathogens, they are less susceptible to a multiplex assay such as the methods taught herein, and frequently require more sample as they are frequently less sensitive than the method of the instant invention. A number of methods can be used to detect one or more biomarkers, including methods that use one or more antibodies that specifically bind the biomarkers. The phrase "specifically binds", when referring to an antibody or other binding moiety refers to a binding reaction that is determinative of the presence of the target marker even when the target marker is in the presence of a heterogeneous population of proteins and other biologics. Thus, under designated assay conditions, the specified binding moieties bind preferentially to a particular target marker and do not bind in a significant amount to other components present in a test sample.

A variety of immunoassay formats can be used to select antibodies specifically immunoreactive with a particular pathogen. For example, solid-phase ELISA immunoassays are routinely used to select monoclonal antibodies specifically immunoreactive with an analyte. See Harlow and Lane (1988) Antibodies, A Laboratory Manual, Cold Spring Harbor Publications, New York, which describes immunoassay formats and conditions that can be used to determine specific immunoreactivity. Typically an antibody that is specific for a specific target will bind the target in an amount at least twice as much as background, and more typically more than 10 to 100 times background.

Antibodies can be raised against any number of pathogen-specific biomolecules, including proteins, carbohydrates of lipids. Preferably, the marker molecules are produced during multiplication of the pathogen and reside on the surface of the pathogen particles, or on the surface of pathogen-infected cells. Further, markers can be secreted from pathogens or pathogen-infected cells, or can be liberated into solution during lysis of pathogen or pathogen-infected cells.

One viral marker specific for the CMV virus is pp65 matrix protein. Antibody that specifically binds pp65 matrix protein can be used for quantitative detection of actively replicating CMV in antibody based methods including, but not limited to, an immunofluorescence assay using peripheral blood leucocytes or enzyme-linked immunoassays (such as ELISA) (Clin Diagn Virol. 1996 May 5 (2-3):81-90 Grandien M.).

Human polyoma JC virus can be detected using an antibody that specifically binds the major capsid protein VP1 (J Virol Methods. 1996 May;59(1-2):177-87; Chang D, Liou ZM, Ou WC, Wang KZ, Wang M, Fung CY, Tsai RT.).

Human herpes simplex virus can be measured by immunoassays which use antibodies which, specifically bind matrix protein G. Moreover, two major types of HSV, HSV1 and HSV2, can be distinguished by antibodies which specifcally bind one of two variants of G protein, gG1 and gG2, (J Virol Methods. 1999 Dec;83(1-2):75-82. Coyle PV, Desai A, Wyatt D, McCaughey C, O'Neill HJ.)

Pathogenic Gram-negative bacteria can be detected using antibodies that specifically bind lipopolysaccharides (LPS), the major components of outer bacterial membrane (J Immunol Methods. 2005 Mar;298(1-2):73-81. Thirumalapura NR, Morton RJ, Ramachandran A, Malayer JR.).

Lysteria monocytogenes can be detected using antibodies that specifically bind a 60-kDa protein collectively termed p60, which is encoded by the iap (invasion-associated protein) gene and secreted in large quantities by Lysteria monocytogenes into the growth media (Clin Diagn Lab Immunol. 2004 May;11(3):446-51. Yu KY, Noh Y, Chung M, Park HJ, Lee N, Youn M, Jung BY, Youn BS.).

Mycobacterium tuberculosis can be measured with antibodies that specifically bind to lipoarabinomannan (LAM), major and specific glycolipid component of the outer mycobacterial cell wall (J Microbiol Methods. 2001 May; 45 (1):41-52. Hamasur B, Bruchfeld J, Haile M, Pawlowski A, Bjorvatn B, Kallenius G, Svenson SB.).

Multipltiplex detection using immunoassays can be performed by a number of different assay platforms that detect antibodies labeled with fluorescent dyes or chemically linked to enzymes capable to produce measurable signal (color dyes, fluorescent or luminescent dyes). Examples of such assay platforms include immunofluorescent or immunoenzymatic staining of pathogen-infected cells (Immunocytochemical Methods and Protocols (Methods in Molecular Biology), Lorette C. Javois (Editor), Humana Press, 1999; Enzyme-lynked immunoassay (ELISA) (The ELISA Guidebook (Methods in Molecular Biology), J. R. Crowther (Editor), Humana Press, 2000), color-encoded beads commercialized by Luminex Inc (as described in US Patent 6,524,793); multiplexed ELISA microarrays (such as Search Light platform commercialized by Endogen, a division of Fisher Scientific Co.).

The exact type of opportunistic infection (bacterial, viral, fungal, or protozoal/parasitic) that occurs depends upon the type and extent of immunologic alteration, whether it be cellular, humoral, phagocytic, or a combined defect; and upon organisms present in the internal and external environments. The administration of corticosteroids and other immunotoxic drugs to transplant recipients can result in massive depression of all phases of host defense, including a breakdown of cutaneous and mucosal barriers.

Aerobic enteric, primarily bacteria and *Candida,* are potential causes of infections in liver transplant recipients, occurring within the first and second month posttransplantation. The usual sites are the abdomen, bloodstream, lungs, and surgical wound.

Enteral nutrition is frequently necessary to provide adequate nutrients to debilitated patients in the posttransplant period and may be favored over parenteral nutrition in hopes of avoiding fungal sepsis. Enteral formulas, however, are also superb microbiologic culture media and are easily contaminated, and can lead to gastroenteritis and sepsis. Organisms that frequently contaminate enteral formulas include *Enterobacter cloacae, Klebsiella pneumoniae,* streptococci, *Pseudomonas* aeruginosa, *Serratia* spp, *Citrobacter* spp, and *Bacillus* spp.

There are several pathogens which could be dangerous for an individual having an immunocompromised status, including, but not limited to bacteria, including, but not limited to Group B *Streptococcus , Escherichia coli, Listeria monocytogenes, Neiseirria meningitidis, Streptococcus pneumoniae, Haemophilus influenzae, S. pneumoniae* or *N. meningitidis, , L. monocytogenes, Pseudomonas aeruginosa, meningococcal meningitis, pneumococcal pneumonia, Nocardia* spp., *Legionella* spp., gram-negative bacilli, *Bacillus anthracis, Yersinia pestis, clostridium botulinum, francisella tularensis, Escherichia coli, vibrio spp., Shigella spp. Liseria monocytogenes, Campylobacter jejuni, Yersinia enterocolitica, vibrio cholerae, Salmonella, L. monocytogenes, enteroinvasive E. coli,* and *mycobacterium tuberculosis,* and including, but not limited to, protozoa including *Cryptosporidium parvum, Cyclospora cayetanensis, Giardia lamblia, Enamoeba histolytica, toxoplasma gondii* and *Microsporidia.*

There are several viruses which could be dangerous for an individual having an immunocompromised status, including, but not limited to; HSV1, HSV2, EBV, CMV, HHV6, HHV7, HHV8, VZV, hepatitis C, hepatitis B, adenovirus, EEEV, WNE, JCV and BKV.

The threat of infection of harmful pathogens, including viral pathogens, in immunocompromised patients requires monitoring of the peripheral blood for viral levels, as well as the levels of other pathogens. The pathogens can be detected using individual serological techniques, specific for each virus being monitored. However, individual serological tests are costly and inefficient. Nucleic acid amplification methods, such as PCR, potentially allow the detection of the pathogens at an earlier stage of disease progression, as opposed to waiting for an immune response to be generated, if in fact any immune response is generated. Due to the sensitivity of PCR related methods and PCR's ability to detect the presence of a pathogenic genome in a sample, both the presence and the amount of pathogen in a sample can be more sensitively determined at an earlier stage using PCR techniques in comparison to serological techniques.

In one aspect the invention refers to a method for detecting in a single assay, the presence of any of a plurality of pathogens in a biological sample from an immunocompromised individual. The plurality of pathogens include virus, bacteria, protozoan, fungi, and any combination thereof. The method comprises the following four steps.

The first step comprises choosing for each pathogen of a plurality of pathogens, a pair of oligonucleotide primers which will, under a set of amplification conditions, mediate the amplification of a polynucleotide amplicon of a selected, known length from a nucleic acid prepared or isolated from the pathogen under consideration.

The length of the amplicon from the pathogen under consideration is designed to be different from the lengths of any of the other amplicons generated from each of the pathogen nucleic acid targets prepared or isolated from each of the remaining members of the plurality of pathogens being analyzed in the patient sample. The selection of a pair of primers for each member of the plurality of pathogens establishes a set of oligonucleotide primers for the simultaneous amplification of a set of amplicons, each corresponding to a pathogen in the plurality of pathogens.

The second step involves contacting nucleic acid from a biological sample, or nucleic acid prepared or isolated from a biological sample by a process such as reverse transcription, with the set of oligonucleotide primers, under conditions permitting the amplification of polynucleotides. When one or more members of the plurality of pathogens is present in the biological sample, an amplicon of known length indicative of the presence of each member present is generated by the amplification reaction.

The third step involves separating the amplified nucleic acid molecules by size. The fourth step involves detecting the separated nucleic acids. In practice the separation and detection steps can be combined, e.g., as when labeled nucleic acid is separated by, e.g. capillary electrophoresis and detected by e.g., fluorescence near or at the end of the capillary. The detection of the separated amplicons is based on the known length of each amplicon. Each amplicon was designed to be a length distinct from the lengths of the remaining amplicons generated from other target nucleic acids. Thus the size of each of the detected amplicons allows the determination of which if any of the plurality of pathogens under consideration are present in the biological sample.

Variations of this method include, but are not limited to, sampling the amplification reaction at one or more intervals during the amplification (e.g., removing an aliquot from the reaction mixture). This can permit the generation of an amplification profile that can provide for accurate determinations of original amounts of each pathogen template,

Additional variations of this method include, before the amplification step, reverse-transcribing the nucleic acid molecules purified from the biological sample. This can permit the detection, for example, of the viral genome of RNA viruses, or, alternatively, the presence of viral transcripts, as well as the transcripts from other types of pathogens.

Further, this method is capable of detecting the presence in a single assay of at least two pathogens in the biological sample, or at least three, four, five, six, seven, eight, nine, ten, eleven, twelve, thirteen, or fourteen, or fifteen or at least up to sixteen different pathogens in the biological sample. In one embodiment, the detection of the pathogens results from a single amplification reaction in which a multitude of pathogen derived target molecules are amplified.

In another embodiment, the viral pathogens to be detected are selected from the group consisting of; HSV1, HSV2, EBV, CMV, HHV 6, HHV7, HSV8, VZV, hepatitis C, hepatitis B, adenovirus, EEEV, WNE, JCV and BKV. Further, this method is capable of simultaneously detecting the presence of at least two virus specific target molecules in a nucleic acid sample prepared or isolated from a biological sample, or at least three, four, five, six, seven, eight, nine, ten, eleven, twelve, thirteen, or fourteen, or fifteen or at least up to sixteen virus specific target molecules in the test nucleic acid prepared or isolated from the biological sample, and can encompass at least two, or at least three, four, five, six, seven, eight, nine, ten, eleven, twelve, thirteen, or fourteen, or fifteen or at least up to 16 different specific virus targets selected from the group consisting of; HSV1, HSV2, EBV, CMV, HHV 6, HHV7, HHV8, VZV, hepatitis C, hepatitis B, adenovirus, EEEV, WNE, JCV and BKV.

Another aspect of the methods described herein is that the sample can be obtained from an individual to whom a course of therapy has been administered that causes the individual to become immunocompromised. Such therapies include, but are not limited to immunosuppressive therapies prescribed for transplant patients and for cancer patients. The methods described herein can be used in monitoring the course of immunosuppressive treatment or a treatment that causes immunosuppression.

The methods described herein can further comprise the step of quantitating each pathogen of the plurality of pathogens being assayed for in the sample. In one aspect, quantification is enhanced by adding to the test nucleic acid sample, at least two nucleic acid competitor molecules that will be amplified with the same primers and at a similar efficiency as a pathogen specific target nucleic acid prepared or isolated from a pathogen. The concentrations of each set of competitor targets added to the test nucleic acid sample are known and can differ from each other by at least one order of magnitude. The competitor nucleic acids can comprise RNA and/or DNA.

The methods described herein provide for an approach for the detection and quantification of a plurality of pathogens of interest in a sample from an immunocompromised patient, the method including for each given pathogen, selecting a pathogen specific target polynucleotide which is specific for the pathogen. In this approach, for each given pathogen specific target polynucleotide, a pair of oligonucleotide amplification primers is selected, such that the primer pair will generate an amplicon of a known length, which is specific for, and is generated from, at least a portion of the given pathogen specific target polynucleotide, and wherein the length of the amplicon is distinct from the length of an amplicon generated from any other of the selected pathogen specific target polynucleotides or from a competitor polynucleotide,

This approach further involves synthesizing one or more competitor polynucleotides, so that each competitor polynucleotide will generate an amplicon of known length when using the oligonucleotide amplification primer pair described in the preceding paragraph, and wherein the length of the amplicon is distinct from the length of an amplicon generated from any of the pathogen specific target polynucleotides or from any other of the competitor polynucleotides.

This approach further includes purifying polynucleotides from the patient sample, the polynucleotides being either RNA, DNA or both. In the case of RNA, a cDNA is formed using reverse transcriptase.

This approach further includes adding a predetermined amount of the one or more competitor polynucleotides to the polynucleotides purified and/or prepared from the individual's sample, thereby forming a polynucleotide test mixture. Each individual competitor polynucleotide will be added at known concentrations that differ from one another, e.g., on the order of one log. Each of the target polynucleotides present in the polynucleotide test mixture is then amplified in a single multiplexed assay using the pairs of first and second oligonucleotide amplification primers, each pair being specific for each pathogen being assayed, under conditions that allow the generation of amplicons from each of the pathogen specific target polynucleotides as well as the competitor nucleotides.

This approach further includes separating the amplicons generated in the PCR reaction described in the previous paragraph, and detecting each of these amplicons. The length of each of the generated amplicons can be used to identify from which target polynucleotide the amplicon was generated, and thus allows the identification of which pathogens were detected from the sample.

This approach can also include quantifying each of the pathogen specific target polynucleotides identified as described in the previous paragraph by comparing the amount of the amplicon generated from each of the pathogenic specific target polynucleotides with the amount of the amplicon(s) generated from one or more respective competitor polynucleotides, since each of the competitor polynucleotides was present in a predetermined quantity in the test polynucleotide test mixture immediately before amplification. The quantity of each pathogen specific target polynucleotide correlates with the quantity of the respective pathogen of interest present in the individual's sample.

The amplicons can be separated by capillary electrophoresis (CE), and the one or more of oligonucleotide amplification primers can be linked to a detectable label. The detectable label can include but is not limited to: fluorescent labels, radioactive labels, colorimetrical labels, magnetic labels, and enzymatic labels. The amount of each amplicon detected from an amplification assay can be determined by measurement of the label signal, e.g., by measurement of fluorescence.

In instances wherein each of the pathogen specific target polynucleotides comprises RNA, steps are provided for reverse-transcribing pathogen specific target polynucleotides and competitor RNA polynucleotides before amplification. Accordingly, in methods where both RNA and DNA are separately purified, the purified RNA and the purified DNA are analyzed in separate amplification reactions. Alternatively, a reverse transcription step can be employed whenever at least one target is an RNA virus or where viral transcripts or pathogen transcripts are sought to be detected. The amplicons can be generated through PCR or using transcription-mediated amplification such as TMA and NASBA.

Real time PCR can be used in the methods described herein. "Real-time" quantitative PCR analysis has been applied to the determination of viral DNA levels (Niesters H et al. Development of a real-time quantitative assay for detection of Epstein-Barr virus. J Clin Microbiol. February 2000; 38(2): 712-5). Kinetic PCR is a method for determining the initial template copy number. In that approach, the quantitative information in a PCR reaction comes from the few cycles where the amount of DNA grows logarithmically from barely above background to the plateau. Often, only 6 to 8 cycles out of 40 will fall in this log-linear portion of the curve.

In the methods described herein, the pathogens can be viruses including, but not limited to, HSV1, HSV2, EBV, CMV, HHV 6, HHV7, HHV8, VZV, hepatitis C, hepatitis B, adenovirus, EEEV, WNE, JCV and BKV.

In the methods described herein, the pathogens can be bacteria, including but not limited to, Group B *Streptococcus, Escherichia coli, Listeria monocytogenes, Neiserria meningitidis, Streptococcus pneumoniae, Haemophilus influenzae, S. pneumoniae* or *N. meningitidis, , L. monocytogenes, Pseudomonas aeruginosa, meningococcal meningitis, pneumococcal pneumonia Nocardia* spp., *Legionella* spp., *gram-negative bacilli , Bacillus anthracis, Yersinia pestis, Clostridium botulinum, Francisella tularensis, Escherichia coli, vibrio spp., Shigella spp., Listeria monocytogenes, Campylobacter jejuni, Yersinia enterocolitica, Vibrio cholerae, Salmonella, L. monocytogenes, enteroinvasive E. coli,* and *mycobacterium tuberculosis.*

In one aspect, primers specific for two or more, up to and including, for example, 15 or more, different viruses are included in a single assay permitting multiplex detection.

## VIRAL TARGETS

The methods described herein are effective to identify the presence and/or amount of any of a wide variety of viruses. Viruses of particular clinical relevance, particularly to immunocompromised patients, are described below.

HSV-1 and HSV-2

As described in U.S. Patent 5,558,863, more than 50 herpes viruses are known to infect over 30 different species. A. J. Nahmias and B. Roizman, New Engl. J. Med. 289, pp. 667-674 (1973). Herpes simplex virus 1 (HSV-1) and herpes simplex virus-2 (HSV-2) are among the most clinically significant, naturally occurring variants of herpes simplex virus (HSV). Man is the sole reservoir of this virus. HSV was first isolated in 1920. B. Lipschutz, Arch. Derm. Syph. (Berl) 136, pp. 428-482 (1921). In 1961, two serotypes were differentiated. Generally, HSV-1 infects non-genital sites while HSV-2 infects genital sites. It is possible, however, to isolate HSV-1 in a genital herpes case. Transmission is direct. Localized ulcers or lesions in the oral cavity, eye, skin or reproductive tract usually develop after infection. Dissemination can cause encephalitis in neonates and the immunosuppressed. The virus can remain latent, presumably for years, until a relapse is triggered by stress, environmental factors, other medications, food additives or food substances (see A. J. Nahmias and B. Roizman, New Engl. J. Med. 13, pp. 667-674 (1973); W. E. Rawls, E. H. Lennette (eds.), Laboratory Diagnosis of Viral Infections, Marcel Dekker, Inc., New York, pp. 313-328 (1985)).

EBV

Epstein-Barr virus (EBV) is another pathogen from the herpes virus group. Discovered in the 1960's, it is the principal etiologic agent of infectious mononucleosis and has been associated with Burkitt's lymphoma and nasopharyngeal carcinoma malignancies (see W. Henle and G. Henle, M. A. Epstein and B. G. Achong (eds.), The Epstein-Barr Virus, Springer-Verlag, Berlin, p. 297 (1979)). Infectious mononucleosis is characterized by lymphadenopathy, fever and pharyngitis. As with the HSV variants, the Epstein-Barr virus may establish a latent infection which may be reactivated when the host is immunosuppressed (see E. T. Lennette, E. H. Lennette (eds.), Laboratory Diagnosis of Viral Infection, Marcel Dekker, Inc., New York, pp. 257-271 (1985)). As such, EBV can also cause acute and rapidly progressive B lymphoproliferative disease in severely immune compromised patients.

Transplant patients are all at risk for developing EBV infection and therefore post transplant lymphoproliferative disorder (PTLD). However, the group at highest risk for this complication is the liver transplant population. This is because these patients are generally very young, frequently less than 5 years of age, and therefore they frequently have not yet been exposed to EBV and as a result do not have a natural immunity to the virus.

VZV

Varicella zoster virus (VZV) is also a herpes virus, and is the causative agent of both varicella (chicken pox) and zoster (shingles). Varicella occurs primarily in childhood, whereas the more localized zoster occurs in the elderly and immunocompromised. Zoster is, in fact, due to a reactivation of a latent VZ infection. Patients suffer painful, vesicular skin lesions (see A. Gershon, E. H. Lennette (eds.), Laboratory Diagnosis of Viral Infections, Marcel Dekker, Inc., New York, pp. 329-340 (1985)). Currently, analgesics provide the only treatment for shingles (see R. Boyd, et al., Basic Medical Microbiology, 2nd Edition, Little, Brown and Company, Boston, p. 527, (1981)).

CMV

Cytomegalovirus (CMV) is also a member of the human herpes virus family, infecting between 50-100% of all individuals worldwide, as described in U.S. Patent 6,936,251.

CMV is naturally transmitted via saliva, urine, or breast milk but can also be recovered from other body secretions. In addition, CMV can be transmitted transplacentally to the fetus, by geno-urinary contact during birth or intercourse, by blood transfusion (esp. white cells), and bone marrow or. organ transplant.

After primary infection CMV persists in the body for the lifetime of its host in a state of dynamic latency, well controlled by the host immune system, and may be recovered periodically from different sites and body secretions. Although generally benign, CMV infections can be devastating and fatal in individuals with immune defects, such as transplant recipients, AIDS patients, patients with genetically determined immunodeficiencies and newborns with an immature immune system.

HHV6

Human Herpes virus-6 (HHV6) viruses are also a member of the human herpes virus family, and contain double strand DNA. HHV6 strains have been isolated from lymphocytes of patients suffering from AIDS or having lymphoproliferative disorders. These viruses are also regarded as being the causal agent of exanthema subitum, as described in U.S. Patent 5,545,520.

HHV6 is a beta herpes virus first described by Salahuddin and colleagues in 1986, is present in a latent state in about ninety percent of the human population. During periods of active infection, however, the virus is associated with various clinical illnesses. As described in U.S. Patent 5,756,302, HHV-6 is the clinical etiological agent of roseola infantum and exanthem subitum in children and is commonly associated with clinically significant bone marrow suppression in infants with primary HHV-6 infections. In adults, HHV-6 is causally associated with a wide spectrum of clinical illness, which can be fatal in at-risk immunocompromised or immunosuppressed populations. Notably, HHV-6 is prominent in patients having pneumonitis and encephalitis and in patients immunosuppressed following allogeneic bone marrow transplant (AIBMT) or solid organ transplant. In AIBMT patients, HHV-6 associated bone marrow suppression (HBMS) correlates with direct viral infection of the bone marrow. Persistent infection by HHV-6 of bone marrow can cause chronic bone marrow suppression.

HHV-7

Human herpes virus 7 (HHV-7) is a β-herpes virus discovered in 1990 as described in U.S. Patent Publication 20040091852. HHV-7 is widespread in the general population and produces a primary phase infection early in life and, like other herpes viruses, persists indefinitely in the latent form in the infected organism. HHV-7 is genetically close to cytomegalovirus (CMV) and to human herpes virus 6 (HHV-6) which, especially in the case of CMV, are major pathogenic viruses. The responsibility of HHV-7 for human diseases is still being explored. It is thought that, during immunosuppression, its pathogenic power is exacerbated and gives rise to serious opportunistic infections, like other herpes viruses. In particular, this may be the case after organ transplant.

HHV-8

On the basis of sequence homologies HHV-8 belongs to the gamma herpes virus sub-family and is closely related to EBV and Herpes virus saimiri, as described in U.S. Patent Publication 20030013077. The HHV-8 genome is 140 kb in size and is flanked by several repetitive sequences having a length of approximately 800 bp (Russo et al., 1996). HHV-8 codes for about 80 proteins, 10 of which show homology to cellular gene products (Neipel et al., 1997). Similar to all other herpes viruses, HHV-8 is able to cause a lytic infection which then becomes a latent infection. In the latent phase, at least two viral transcripts are expressed: a differentially spliced mRNA encoding the v-cyclin, v-flip and LANA proteins, as well as T0.7, a short RNA 0.7 kb in length and of up to now unknown function (Zhong et al., 1996). The viral transcript T0.7 is the most abundant of the RNAs expressed in the latent phase and has three open reading frames corresponding to 60, 35, and 47 amino acids.
The human herpes virus 8 has been detected in all forms of Kaposi's sarcoma, in primary effusion lymphomas (PEL), in Castleman's disease, in angiosarcomas, in skin lesions of patients who underwent transplantations, in plasmacytomas, sarcoidosis as well as in healthy control individuals (Chang et al., 1994; Boshoff and Weiss, 1997).

HEPATITIS C VIRUS

Hepatitis C virus (HCV) is the major etiological agent of 90% of all cases of non-A, non-B hepatitis (Dymock, B. W. Emerging Drugs 6:13-42 (2001)). The incidence of HCV infection is becoming an increasingly severe public health concern with 2-15% individuals infected worldwide. While primary infection with HCV is often asymptomatic, most HCV infections progress to a chronic state that can persist for decades. Of those with chronic HCV infections, it is believed that about 20-50% will eventually develop chronic liver disease (e.g. cirrhosis) and 20-30% of these cases will lead to liver failure or liver cancer.
HCV is a plus (+) strand RNA virus which is well characterized, having a length of approximately 9.6 kb and a single, long open reading frame (ORF) encoding an approximately 3000-amino acid polyprotein (Lohman et al., Science 285:110-113 (1999).as described in U.S. Patent Publication 20040121975. The ORF is flanked at the 5' end by a nontranslated region that functions as an internal ribosome entry site (IRES) and at the 3' end by a highly conserved sequence essential for genome replication (Lohman, supra). The structural proteins are in the N-terminal region of the polyprotein and the nonstructural proteins (NS) 2 to 5B in the remainder.

HEPATITIS B VIRUS

Hepatitis B virus (HBV) is a compact, enveloped DNA virus belonging to the Hepadnavirus family. This virus is the major cause of chronic liver disease and hepatocellular carcinoma world-wide (Hoofnagle (1990) N. Eng. J. Med. 323:337-339). HBV is associated with acute and chronic hepatitis and hepatocellular carcinoma, and may also be a cofactor in the development of acquired immune deficiency syndrome (Dienstag et al. in Harrison's Principles of Internal Medicine, 13th Ed. (Isselbacher et al., eds.) McGraw-Hill, NY, N.Y. (1993) pp. 1458-1483).
HBV is a compact, enveloped DNA virus belonging to the Hepadnavirus family. It has a circular, partially single-stranded, partially double-stranded 3.2 kb genome which includes four overlapping genes: (1) the pre-S and S genes, which encode the various envelope or surface antigens (HBsAg); (2) the preC and C gene, which encodes the antigens HBcAg and HBeAg; (3) the P gene, which encodes the viral polymerase; and (4) the X gene, which encodes HBx, the transactivating protein. Full-length clones of many hepadnaviruses have been obtained and their nucleotide sequences obtained. (see, e.g., Raney et al. in Molecular Biology of the Hepatitis B Virus (McLachlan, ed.) CRC Press, Boston, Mass., (1991) pp. 1-38). Replication occurs in hepatocytes and involves converting the single stranded-region of the HBV genome to double-stranded circular DNA, generating the covalently closed circular (CCC) DNA. Transcription of this DNA by the host RNA polymerase generates an RNA template of plus stranded polarity, the pregenomic RNA, which serves as a template for the translation of viral proteins, and is also encapsulated into virus cores. In the virus cores, the RNA serves as a template for reverse transcription, generating a DNA minus strand. The viral polymerase then produces a DNA plus strand using an oligomer of viral RNA as a primer. The newly synthesized double-stranded DNA in the viral core is assembled with the viral envelope proteins, generating a newly infectious viral particle.

AAV

Adeno-associated virus (AAV), a parvovirus dependent upon adenovirus or herpes virus for full "lytic" infection (Buller et al., J. Virol. 40:241-47 (1981)). As described in U.S. Patent 6593123, AAV requires co-infection with an unrelated helper virus, e.g., adenovirus, herpes virus, or vaccinia, in order for a productive infection to occur. In the absence of a helper virus, AAV establishes a latent state by inserting its genome into a host cell chromosome. Subsequent infection by a helper virus rescues the integrated viral genome, which can then replicate to produce infectious viral progeny. For a review of AAV, see, e.g., Berns and Bohenzky (1987) Advances in Virus Research (Academic Press, Inc.) 32:243-307.

The AAV genome is composed of a linear, sing-stranded DNA molecule that contains 4681 bases (Berns and Bohenzky, supra). The genome includes inverted terminal repeats (ITRs) at each end that function in cis as origins of DNA replication and as packaging signals for the virus. The ITRs are approximately 145 bp in length. The internal nonrepeated portion of the genome includes two large open reading frames, known as the AAV rep and cap regions, respectively. These regions code for the viral proteins that provide AAV helper functions, i.e., the proteins involved in replication and packaging of the virion. Specifically, a family of at least four viral proteins is synthesized from the AAV rep region, Rep 78, Rep 68, Rep 52 and Rep 40, named according to their apparent molecular weight. The AAV cap region encodes at least three proteins, VP1, VP2 and VP3. For a detailed description of the AAV genome, see, e.g., Muzyczka, N. (1992) Current Topics in Microbiol. and Immunol. 158:97-129.

EEEV

Eastern Equine Encephalitis Virus (EEEV), is a member of the alphavirus genus of the family Togaviridae that is comprised of a large group of mosquito-borne RNA viruses found throughout much of the world. The viruses normally circulate among rodent or avian hosts through the feeding activities of a variety of mosquitoes. Epizootics occur largely as a result of increased-mosquito activity after periods of increased rainfall. EEE was first isolated in Virginia and New Jersey in 1933 (Ten Broeck, C. et al. [1935] J. Exp. Med. 62:677)

WNE

West Nile virus (WNE) is a member of the family Flaviviridae, genus Flavivirus belonging to the Japanese Encephalitis antigenic complexes of viruses. as described in U.S. Patent Publication 20040197769. This sero-complex includes JEV, SLEV, Alfuy, Koutango, Kunjin, Cacipacore, Yaounde, and Murray Valley Encephalitis viruses. WNE infections generally have mild symptoms, although infections can be fatal in elderly and immunocompromised patients. Typical symptoms of mild WNE infections include fever, headache, body aches, rash and swollen lymph glands. Severe disease with encephalitis is typically found in elderly patients (D. S. Asnis et al., supra). For the most part, treatment of a subject having a flavivirus infection is a symptomatic treatment, i.e. the general symptoms of a flavivirus infection are treated, such that for initial treatment, mere knowledge of the infection being a flavivirus infection may be sufficient. However, in certain other cases rapid and accurate diagnosis of the specific flavivirus, particularly WNE, is critical such that the most appropriate treatment can be initiated.

JCV

The JC virus (JCV) belongs to the group of human polyoma viruses. JCV can cause a sub-acute demyelinizing disease of the brain by a lytic infection of myelin-forming oligodendrocytes and an abortive infection of astrocytes, as described in U.S. Patent 6,238,859. This infection, which is referred to clinically as progressive multifocal leukoencephalopathy (PML), leads to the formation of demyelinizing foci in the cerebrum cerebellum and brain stem and usually ends lethally within a few months. Although JCV appears to be present in about 80% of the adult population, PML generally only develops in connection with a weakening of the immune system. The increasing use of immuno-suppressive drugs and the increasing number of HIV-infected patients has led to a considerable increase in PML diseases in recent years. According to current estimations a PML develops in about 2-5% of AIDS patients.

BKV

BK virus (BKV) is a human polyoma virus that was originally isolated from the urine of immunocompromised patients, as described in U.S. Patent. 6,605,602. Since then, a number of BKV variants (subtypes) have been isolated. BKV causes a subclinical (asymptomatic) infection in the majority of the general population within the first 10 years of life. Subsequent to infection, the virus normally remains latent in the kidney. However, the virus may become reactivated at a later point in time as a result of immunosuppression, for example, following renal transplantation.

BKV contains a double stranded DNA (dsDNA) genome. The complete DNA sequence of BKV is approximately 5,100 base pairs, however this varies with each variant of BKV. For example, the Dunlop strain of BKV contains 5,153 base pairs (see, for example, Self et al. (1979), Cell 18:963-77. The BKV genome contains a coding region and a non-coding control region, but is functionally divided into three regions. The coding region can be further divided into the early region and the late region. The early region contains the coding sequence for two non-structural proteins: the T-antigen protein and the t-antigen protein. The late region contains the coding sequence for four structural proteins: VP-1, VP-2, and VP-3. The non-coding control region contains the transcriptional control elements for both early and late gene expression, as well as containing the viral origin of replication.

SMALLPOX

Smallpox, which is caused by the virus *Variola major,* is considered one of the most dangerous potential biological weapons because it is easily transmitted from person to person, no effective therapy exists, and few people carry full immunity to the virus. Although a worldwide immunization program eradicated smallpox disease in 1977, small quantities of smallpox virus still exist in two secure facilities in the United States and Russia. However, it is likely that unrecognized stores of smallpox virus exist elsewhere in the world.

The symptoms of smallpox infection appear approximately 12 days (the range is from 7 to 17 days) after exposure. Initial symptoms include high fever, fatigue, headache, and backache. A characteristic rash, which is most prominent on the face, arms, and legs, follows in 2 to 3 days. The rash starts with flat red lesions (a maculopapular rash) that evolve into vesicles. Unlike chickenpox, the lesions associated with smallpox evolve at the same rate. Smallpox lesions become filled with pus and begin to crust early in the second week after exposure. Scabs develop, separate, and fall off after approximately 3 weeks. Individuals are generally infectious to others from the time immediately before the eruption of the maculopapular rash until the time scabs are shed. Smallpox spreads directly from person to person, primarily by aerosolized saliva droplets expelled from an infected person. Contaminated clothing or bed linens also can spread the virus. The mortality of smallpox infection is approximately 30 percent, and patients who recover frequently have disfiguring scars.

The variola virus has not been well studied because of the hazards associated with potential exposure. However, vaccinia virus, which is used as a smallpox vaccine and is closely related to variola, is well studied. The few comparative studies of the two viruses have shown that the major differences are in the host ranges: whereas vaccinia infects several hosts, variola infects only humans naturally and cynomolgus monkeys under artificial laboratory conditions. The two viruses can be distinguished by the appearance of lesions on chick embryo chorioallantoic membranes and by tissue culture growth characteristics. The viruses share antigens and generate cross-neutralizing antibodies, a characteristic that has been exploited in the use of the vaccinia vaccine to prevent smallpox. The two viruses can be distinguished by PCR, ELISA, radioimmunoassays, and monoclonal antibodies. Vaccinia is now being investigated extensively as a vector for the delivery of other vaccine genes.

Two forms of infectious orthopoxvirus are produced in infected cells: intracellular mature virus (IMV) that remain in the infected cell and extracellular enveloped virus (EEV) that are released from the cell late in infection. The EEV form of the virus contains an additional lipid envelope and cellular and viral proteins, thus making EEV immunologically different from IMV. In addition, the EEV and IMV forms enter cells by different mechanisms, use different cell receptors, and have different sensitivities to antibodies and complement. Immune evasion by poxviruses is accomplished through mechanisms related to the release of proteins that bind chemokines, EEV resistance to neutralizing antibodies, and EEV resistance to complement destruction through acquisition of host complement control proteins.

Variola and vaccinia belong to the *Orthopoxvirus* genus of poxviruses. These double-stranded DNA viruses replicate in the cytoplasm, unlike other DNA viruses that depend on host nuclear DNA replication enzymes. Several strains of variola and vaccinia have been genomically sequenced. The genes for structural, membrane, and core proteins appear to be highly conserved among orthopoxviruses. Genes responsible for growth in human cells also have been identified. NIAID will actively pursue further research in these areas.

ARTHROPOD-BORNE VIRUSES

Category B and C arthropod-borne viruses (arboviruses) that are important agents of viral encephalitides and hemorrhagic fevers and include a number of types. Alphaviruses are associated with Venezuelan equine encephalitis (VEE) virus, eastern equine encephalitis (EEE) virus, and western equine encephalitis (WEE) virus. Flaviviruses include West Nile virus (WNV), Japanese encephalitis (JE) virus, Kyasanur forest disease (KFD) virus, tick-borne encephalitis (TBE) virus complex, and yellow fever (YF) virus. Bunyaviruses are associated with California encephalitis (CE) virus, La Crosse (LAC) virus, Crimean-Congo hemorrhagic fever (CCHF) virus.

While arthropod vectors such as mosquitoes, ticks or sand flies are responsible for the natural transmission of most viral encephalitis and hemorrhagic fever viruses to humans, the threat of these viruses as potential bioterrorist weapons stems mainly from their extreme infectivity following aerosolized exposure. In addition, vaccines or effective specific therapeutics are available for only a very few of these viruses.

Many arboviruses are endemic in North America (EEE, WEE, WNV, CE, LAC), South America (VEE, WEE), Asia (JE, CCHF), and Africa (WNV, CCHF), including others which are not listed. The most prominent in the United States at the present time is WNV, which was first identified in North America in New York City in 1999. The virus has spread throughout the continental U.S., causing thousands of cases of disease and over a hundred deaths by the end of the summer of 2002.

Natural infection of humans and other animals by an arbovirus is acquired via the bite of an infected mosquito, tick or sand fly, depending on the virus. In general, the incubation period varies from 3 to 21 days, reflecting a period during which the virus replicates locally and spreads by means of the bloodstream to peripheral sites before invading the brain or other target organ. In the brain, certain of these viruses spread cell to cell, causing encephalitis. Other viruses, such as YF and CCHF, target the liver and other organs, causing hemorrhages and fevers. Relatively little is known about the pathogenesis of these encephalitis and hemorrhagic fever viruses. However, in studies of mice exposed to aerosolized VEE, virus was detected in the brain within 48 hours after infection.

In humans, arbovirus infection is usually asymptomatic or causes nonspecific flu-like symptoms such as fever, aches, and fatigue. A small proportion of infected people may develop encephalitis and, although most recover, some may be left with severe residual neurological symptoms such as blindness, paralysis, or seizures. Clinical disease and fatality vary by the specific infecting virus. For example, less than 1% of adults infected with VEE develop encephalitis; on the other hand, the fatality rate is higher among those infected with JE (25%) or EEE (50%) viruses. With LAC infection, disease is more severe and more common in children. However, with WNV, particularly in the U.S., older and immunosuppressed individuals are at greatest risk of developing serious or life-threatening disease. Several of these viruses, such as VEE, EEE, WNV, and JE, also represent important veterinary diseases, causing highly fatal (up to 90%) encephalitis or other symptoms in horses, birds, and other animals.

The transmission cycle of the alphaviruses, flaviviruses, and bunyaviruses generally involves cyclic passage of the virus from an infected vertebrate host (e.g., bird) to an arthropod/insect vector (e.g., mosquito) during feeding of the arthropod on the host. The viruses multiply to high numbers in the anthropod, and are then passed onto and infect a new host when the mosquito feeds/bites again. The transmission cycles of arboviruses are generally not well understood, including the species of vertebrate hosts and arthropod vectors involved in natural maintenance and spread of the virus to new geographic areas and hosts.

The Category B and C arboviruses are all enveloped RNA viruses that replicate in the cytoplasm of infected cells. Viral envelope glycoproteins have been identified that are involved in binding of the virus to host cells, that function in viral tropism, and that serve as targets of host-neutralizing antibodies. The viruses also code for nonstructural proteins, such as enzymes, that are needed in the viral replication process. The number and type of viral structural and nonstructural proteins is specific for each virus family; while some have been extensively studied, others have not. Genomic sequencing and other nucleic acid studies have established relationships among certain of these viruses and have led to identification of sites on genes and proteins that are important for virulence, attenuation of virulence, and associated pathogenesis. Crystallography studies of certain alphavirus and flavivirus structural proteins are providing insights into protein function and identification of potential targets for antiviral drug development.

The methods described herein can be used to deect various types of pathogens including, but not limited to pathogens from any of the following genera of viruses: Adenoviridae, Alfamovirus, Allexivirus, Allolevivirus, Alphacryptovirus, Alphaherpesvirinae, Alphanodavirus, Alpharetrovirus, Alphavirus, Aphthovirus, Apscaviroid, Aquabirnavirus, Aquareovirus, Arenaviridae, Arenavirus, Arteriviridae, Arterivirus, Ascoviridae, Ascovirus, Asfarviridae, Asfivirus, Astroviridae, Astrovirus, Aureusvirus, Avenavirus, Aviadenovirus, Avibirnavirus, Avihepadnavirus, Avipoxvirus, Avsunviroid, Avsunviroidae, Baculoviridae, Badnavirus, Barnaviridae, Barnavirus, Bdellomicrovirus, Begomovirus, Benyvirus, Betacryptovirus, Betaherpesvirinae, Betanodavirus, Betaretrovirus, Betatetravirus, Bimaviridae, Bornaviridae, Bornavirus, Bracovirus, Brevidensovirus, Bromoviridae, Bromovirus, Bunyaviridae, Bunyavirus, Bymovirus, "c2-like viruses," Caliciviridae, Capillovirus, Capripoxvirus, Cardiovirus, Carlavirus, Carmovirus, "Cassava vein mosaic-like viruses," Caulimoviridae, Caulimovirus, Chlamydiamicrovirus, Chloriridovirus, Chlorovirus, Chordopoxvirinae, Chrysovirus, Circoviridae, Circovirus, Closteroviridae, Closterovirus, Cocadviroid, Coleviroid, Coltivirus, Comoviridae, Comovirus, Coronaviridae, Coronavirus, Corticoviridae, Corticovirus, "Cricket paralysis-like viruses," Crinivirus, Cucumovirus, Curtovirus, Cypovirus, Cystoviridae, Cystovirus, Cytomegalovirus, Cytorhabdovirus, Deltarelrovirus, Deltavirus, Densovirinae, Densovirus, Dependovirus, Dianthovirus, "Ebola-like viruses," Enamovirus, Enterovirus, Entomobirnavirus, Entomopoxvirinae, Entomopoxvirus A, Entomopoxvirus B, Entomopoxvirus C, Ephemerovirus, Epsilonretrovirus, Errantivirus, Erythrovirus, Fabavirus, Fijivirus, Filoviridae, Flaviviridae, Flavivirus, Foveavirus, Furovirus, Fuselloviridae, Fusellovirus, Gammaherpesvirinae, Gammaretrovirus, Geminiviridae, Giardiavirus, Granulovirus, Hantavirus, Hemivirus, Hepacivirus, Hepadnaviridae, "Hepatitis E-like viruses," Hepatovirus, Herpesviridae, Hordeivirus, Hostuviroid, Hypoviridae, Hypovirus, Ichnovirus, "Ictalurid herpes-like viruses," Idaeovirus, Ilarvirus, "Infectious laryngotracheitis-like viruses," Influenzavirus A, Influenzavirus B, Influenzavirus C, Inoviridae, Inovirus, Ipomovirus, Iridoviridae, Iridovirus, Iteravirus, "L5-like viruses," Lagovirus, "-like viruses," Leishmaniavirus, Lentivirus, Leporipoxvirus, Leviviridae, Levivirus, Lipothrixviridae, Lipothrixvirus, Luteoviridae, Luteovirus, Lymphocryptovirus, Lymphocystivirus, Lyssavirus, Machlomovirus, Macluravirus, Marafivirus, "Marburg-like viruses," "Marek's disease-like viruses," Mastadenovirus, Mastrevirus, Metapneumovirus, Metaviridae, Metavirus, Microviridae, Microvirus, Mitovirus, Molluscipoxvirus, Morbillivirus, "Mu- like viruses," Muromegalovirus, Myoviridae, Nairovirus, Nanovirus, Narnaviridae, Narnavirus, Necrovirus, Nepovirus, Nodaviridae, "Norwalk-like viruses," Novirhabdovirus, Nucleopolyhedrovirus, Nucleorhabdovirus, Oleavirus, Omegatetravirus, Ophiovirus, Orbivirus, Orthohepadnavirus, Orthomyxoviridae, Orthopoxvirus, Orthoreovirus, Oryzavirus, Ourmiavirus, "P1-like viruses," "P2-like viruses," "P22-like viruses," Panicovirus, Papillomaviridae, Papillomavirus, Paramyxoviridae, Paramyxovirinae, Parapoxvirus, Parechovirus, Partitiviridae, Partitivirus, Parvoviridae, Parvovirinae, Parvovirus, Pecluvirus, Pelamoviroid, Pestivirus, "Petunia vein clearing-like viruses," Phaeovirus, "-29-like viruses," "-H-like viruses," Phlebovirus, Phycodnaviridae, Phytoreovirus, Picomaviridae, Plasmaviridae, Plasmavirus, Plectrovirus, Pneumovirinae, Pneumovirus, Podoviridae, Polerovirus, Polydnaviridae, Polyomaviridae, Polyomavirus, Pomovirus, Pospiviroid, Pospiviroidae, Potexvirus, Potyviridae, Potyvirus, Poxviridae, Prasinovirus, Prions, Prymnesiovirus, Pseudoviridae, Pseudovirus, "M1-like viruses", Ranavirus, Reoviridae, Respirovirus, Retroviridae, Rhabdoviridae, Rhadinovirus, Rhinovirus, Rhizidiovirus, "Rice tungro bacilliform-like viruses," Roseolovirus, Rotavirus, Rubivirus, Rubulavirus, Rudiviridae, Rudivirus, Rymovirus, "Sapporolike viruses," Satellites, Sequiviridae, Sequivirus, Simplexvirus, Siphoviridae, Sobermovirus, "Soybean chlorotic mottlelike viruses," Spiromicrovirus, "SP01-like viruses," Spumavirus, Suipoxvirus, "Sulfolobus SNDV-like viruses," "T1-like viruses," "T4-like viruses," "T5-like viruses," "T7-like viruses," Tectiviridae, Tectivirus, Tenuivirus, Tetraviridae, Thogotovirus, Tobamovirus, Tobravirus, Togaviridae, Tombusviridae, Tombusvirus, Torovirus, Tospovirus, Totiviridae, Totivirus, Trichovirus, Tritimovirus, Tymovirus, Umbravirus, Varicellovirus, Varicosavirus, Vesiculovirus, Vesivirus, Viroids, Vitivirus, Wäkavirus, and Yatapoxvirus.

BACTERIAL PATHOGENS

Bacterial microorganisms can also be detected using the methods described herein. Pathogenic bacteria of particular interest, including those of particular interest for immunocompromised individuals as well as those with potential for use in terrorist attacks, are described in the following.

ANTHRAX

*Bacillus antharacis,* the agent that causes anthrax, has several characteristics that make it a formidable bioterrorist threat. These characteristics include its stability in spore form, its ease of culture and production, its ability to be aerosolized, the seriousness of the disease it causes, and the lack of sufficient vaccine for widespread use.

Human anthrax has three major clinical forms: cutaneous, inhalational, and gastrointestinal. If left untreated, all three forms can result in septicemia and death. Early antibiotic treatment of cutaneous and gastrointestinal anthrax is usually curative; however, even with antibiotic therapy, inhalational anthrax is a potentially fatal disease. Although case-fatality estimates for inhalational anthrax are based on incomplete information, the historical rate is considered to be high (about 75 percent) for naturally occurring or accidental infections, even with appropriate antibiotics and all other available supportive care. However, the survival rate after the recent intentional exposure to anthrax in the United States was 60 percent for the first 10 cases.

Inhalational anthrax develops after spores are deposited in alveolar spaces and subsequently ingested by pulmonary alveolar macrophages. Surviving spores are then transported to the mediastinal lymph nodes, where they may germinate up to 60 days or longer. After germination, replicating bacteria release toxins that result in disease. Major virulence factors include an antiphagocytic outer capsule and at least two well-characterized toxins. The two toxins, called edema factor (EF) and lethal factor (LF), can destroy cells or inhibit their normal functioning. A third component, called protective antigen (PA), when associated with both EF and LF, enables EF and LF to bind to a specific receptor on mammalian cells. After this complex is internalized, the bacteria's toxic effects are activated. Researchers recently engineered mutant recombinant PAs (rPAs) that bind to the native receptor. These mutant rPAs also can displace wild-type PA by blocking and interrupting the delivery of LF and EF into cells. Recent studies also have identified the region of the mammalian cell receptor to which PA binds and have determined the structure of the LF binding site. Soluble fragments of the receptor containing the toxin-binding site can function as decoys to protect cells from damage by LF. Other recent studies have characterized the site where LF binds to MAPKK (mitogen-activated protein kinase kinase), a vital intracellular enzyme whose disruption by LF causes cell death.

Sequencing of the chromosomal genome of *B. anthracis* is nearly completed. The genes for LF, EF, and PA are contained on plasmids that already have been sequenced. NIAID is expanding sequencing efforts with a comprehensive genomic analysis of *B. anthracis* and related bacilli. Researchers will use sequence data derived from selected strains, isolates, and related species to assess the degree of genetic variation and diversity. This genetic information will provide a framework in which to evaluate the basis for differences in pathogenicity and virulence that have been noted between strains. Other uses for the genomic data include supporting basic research to identify specific molecular markers and targets for strain identification and molecular genotyping; developing sequence-based detection technologies; and designing effective vaccines, therapies, and diagnostic tools. In addition, the data will enhance the detection of genetic polymorphisms that correlate with phenotypes, such as drug resistance, morbidity, and infectivity, as well as key events or processes that influence the germination of spores *in vivo.* A comprehensive bioinformatics resource will support and maintain microbial genomic databases and the development of associated software and bioinformatics tools. These approaches will serve as a prototype for other microorganisms with potential to be used as agents of bioterrorism.

PLAGUE

Plague is caused by the bacterium *Yersinia pestis.* Its potential for use as a biological weapon is based on methods that were developed to produce and aerosolize large amounts of bacteria and on its transmissibility from person to person in certain of its forms. An additional factor is the wide distribution of samples of the bacteria to research laboratories throughout the world. Infection by inhalation of even small numbers of virulent aerosolized *Y. pestis* bacilli can lead to pneumonic plague, a highly lethal form of plague that can be spread from person to person. Natural epidemics of plague have been primarily bubonic plague, which is transmitted by fleas from infected rodents.

Symptoms of pneumonic plague, including fever and cough, resemble those of other respiratory illnesses such as pneumonia. Symptoms appear within 1 to 6 days after exposure and lead rapidly to death. If untreated, pneumonic plague has a mortality rate that approaches 100 percent. Antibiotics are effective against plague, but an effective vaccine is not widely available.

Although *Y. pestis* is very efficient at invading host epithelial cells, the molecular mechanisms that contribute to its invasiveness are not understood. Various iron transport mechanisms as well as the interaction of at least three quorum-sensing mechanisms appear to be involved.

Because the genome of *Y. pestis* has been completely sequenced, it should be possible to accelerate efforts to characterize key events in pathogenesis that will help identify suitable vaccine candidates, diagnostic reagents, and key targets for drug intervention. The *Y. pestis* outer surface membrane proteins (Yomps), of which there are several, appear to be important virulence factors and play a major role in pathogenesis. *Y. pestis* has a set of virulence-associated proteins that are plasmid encoded. Ambient temperature and Ca++ levels regulate the expression and secretion of these proteins through the so-called low-Ca++ response (LCR) mechanism. Further characterization of plasmid-encoded proteins and their role in pathogenesis could provide the basis for an effective subunit vaccine.

To cause infection, *Y. pestis* and other pathogenic bacteria need to remove iron—an essential trace nutrient—from host iron- and/or heme-chelating proteins. *Y. pestis* has three partially characterized iron transport systems that play an important role in iron transport and removal. One of these systems is siderophore-dependent and involves the synthesis of yersiniabactin (Ybt). Since the Ybt system is essential for iron acquisition during the early stages of plague, it may be an excellent target for early intervention and treatment.

BOTULISM

Botulinum toxin, which is produced by the spore-forming anaerobic bacterium *Clostridium botulinum,* is a highly toxic substance that presents a major threat from intentional exposure. The toxin is highly lethal and easily produced and released into the environment. Botulinum toxin is absorbed across mucosal surfaces and irreversibly binds to peripheral cholinergic nerve synapses. Seven antigenic types (A-G) of the toxin exist. All seven toxins cause similar clinical presentation and disease; botulinum toxins A, B, and E are responsible for the vast majority of food borne illnesses in the United States.

Exposure to the toxin induces symptoms that include muscle paralysis; difficulty in speaking, swallowing, or seeing; and, in severe cases, the need for mechanical respiration. People exposed to the toxin require immediate and intensive supportive care and treatment. The onset and severity of symptoms depend on the rate and amount of toxin absorbed into circulation. With food borne exposure, incubation varies from 2 hours to 8 days but is generally limited to 72 hours. Symptoms subside when new motor axon twigs reenervate paralyzed muscles, a process that can take weeks or months in adults.

*C. botulinum* does not normally infect humans. However, humans are exposed to the toxin after eating food contaminated with the organism. Botulinum toxin's mechanism of action is well understood. The toxin consists of a heavy chain and a light chain joined by a single disulfide bond that is essential for neurotoxicity. Both the sequence and three-dimensional structure of the toxin have been determined. The structure consists of three functional domains: a catalytic subunit, a translocation domain, and a binding domain. The toxin binds irreversibly to an unidentified receptor on presynaptic membranes of peripheral cholinergic synapses, mainly at neuromuscular junctions. After internalization of the toxin and translocation into the cytosol, a Zn++-containing endopeptidase on the light chain blocks acetylcholine release from motor neurons. The release is blocked by preventing fusion of acetylcholine-containing vesicles with the terminal membrane. The seven botulinum toxins exhibit somewhat different protease activities, cleaving three SNARE proteins (synaptobrevin/VAMP, SNAP-25, and syntaxin) at different sites. The molecular basis of this proteolytic specificity is not fully understood. The SNARE proteins are essential in the trafficking of synaptic vesicles to the presynaptic membrane.

TULAREMIA

Tularemia is a potential bioterrorist agent because of its high level of infectivity (a few as 10 organisms may cause disease) and its ability to be aerosolized. *Francisella tularensis,* which causes tularemia, is a non-spore-forming, facultative intracellular bacterium that can survive at low temperatures for weeks. Two strains of the organism have been characterized—type A, which is found in North America, is more virulent than type B, which is found in Europe and Asia. The disease is not transmitted from person to person; it spreads naturally from small mammals or contaminated food, soil, or water to humans. Natural infection occurs after inhalation of airborne particles.

Tularemia can take one of several forms, depending on the route of exposure. The disease resulting from the inhalation of airborne *F. tularensis* is the most likely intentional exposure. The inhalation form is an acute, nonspecific illness beginning 3 to 5 days after respiratory exposure; in some cases, pleuropneumonia develops after several days or weeks. If untreated, the disease could lead to respiratory failure. Treatment with antibiotics reduces mortality for naturally acquired cases by 2 to 60 percent. A live attenuated tularemia vaccine has been developed which has been administered under an IND (investigational new drug) application to thousands of volunteers. To date, use of this vaccine has been limited to laboratory and other high-risk personnel.

The fundamental mechanisms involved in virulence and pathogenesis are not known. The cell wall of *F. tularensis* is unusually high in fatty acids. Loss of the capsule may lead to loss of virulence but not viability; however, the capsule is neither toxic nor immunogenic. Infection with *F. tularensis* involves the reticuloendothelial system and results in bacterial replication in the lungs, liver, and spleen. After respiratory exposure, *F. tularensis* infects phagocytic cells, including pulmonary macrophages. In the liver, *F. tularensis* has been shown to invade and replicate in hepatocytes. Destruction of infected hepatocytes results in the release of bacteria and subsequent uptake by phagocytes. When lysis of hepatocytes was prevented by the administration of a monoclonal antibody, bacteria continued to replicate in the hepatocytes, leading to rapid lethality.

INHALATIONAL BACTERIA

The Category B and C bacteria with the potential to infect by the aerosol route include *Brucella* species (spp.), *Burkholderia pseudomallei, Burkholderia mallei, Coxiella burnetii,* and select *Rickettsia* spp. Most of these organisms cause zoonotic diseases or infections, i.e., infections or infectious diseases that may be transmitted from vertebrate animals (e.g., rodents, birds, livestock) to humans. The different bacteria infect humans through different routes, including ingestion, inhalation, or arthropod-mediated transmission. However, all of these agents are believed to be capable of causing infections following inhalation of small numbers of organisms. Consequently, these agents are of special concern for biodefense because they may be weaponized to be dispersed as an aerosol.

Brucellosis, caused by *Brucella* spp., is primarily a zoonotic infection of sheep, goats, and cattle, but occurs in certain species of wildlife, such as bison, elk, and deer. Human infections still occur in the Middle East, Mediterranean basin, India, and China, but are uncommon in the United States (U.S.). Natural human infection can occur following occupational exposure or ingestion of contaminated meat or unpasteurized dairy products. The incubation period is variable from 5 to 60 days. Symptoms are diverse, ranging from acute illness with fever to chronic infections of the brain, bone, genitourinary tract and endocardium. Less than 2% of infections result in death, primarily due to endocarditis caused by *B. melitensis.* Only four of the six *Brucella* spp. - *B. suis, B. melitensis, B. abortus* and - are known to cause brucellosis in humans; *B. melitensis* and *B. suis* are considered more virulent for humans *than B. abortus* or *B. canis.*

*Burkholderia pseudomallei,* which causes melioidosis in humans and other mammals and birds, is found in soil and surface water in countries near the equator, particularly in Asia. Human infection results from entry of organisms through broken skin, ingestion, or inhalation of contaminated water or dust. Several forms of the disease exist with incubation periods ranging from a few days to many years. Most human exposures result in seroconversion without disease. In acute septicemic melioidosis, disseminated *B. pseudomallei* may cause abscesses in the lungs, liver, spleen, and/or lymph nodes. In chronic or recurrent melioidosis, the lungs and lymph nodes are most commonly affected. Mortality is high, up to 50%, among those with severe or chronic disease, even with antibiotic treatment.

*Burkholderia mallei,* the organism that causes glanders, is primarily a disease of horses, mules, and donkeys.

Although eradicated from the U.S., it is still seen in Asian, African, and South American livestock. Natural transmission to humans is rare and usually follows contamination of open wounds resulting in skin lesions. Infection following aerosol exposure has been reported, leading to necrotizing pneumonia. Systemic spread can result in a pustular rash and rapidly fatal illness.

Livestock serve as the primary reservoir of *Coxiella burnetii,* the cause of Q fever. *C. burnetii* is highly infectious and has a worldwide distribution. Infected animals are often asymptomatic but pregnant animals may suffer abortion or stillbirth. Q fever is considered to be an occupational disease of workers in close contact with infected animals and carcasses, although infections have occurred through aerosolized bacteria in cases where close contact has not occurred. Inhalation of only a few organisms can cause infection. After an incubation period of 2 to 3 weeks, acute illness sets in consisting of fever, headache, and frequently, unilateral pneumonia. The organisms proliferate in the lungs and may then invade the bloodstream, resulting in endocarditis, hepatitis, osteomyelitis, or encephalitis in severe cases. Up to 65% of people with chronic infection may die from the disease. *C. burnetii* can remain viable in an inactive state in air and soil for weeks to months and is resistant to many chemical disinfectants and dehydration.

Typhus group rickettsiae such as *Rickettsia prowazekii* are transmitted in the feces of lice and fleas, where a form exists that remains stably infective for months. Spotted fever group rickettsiae, including *R. rickettsii* and *R. conorii,* are transmitted by tick bite. Limited studies have suggested that some rickettsial species have low-dose infectivity via the aerosol route. *R. prowazekii* and *R. rickettsii* cause the most severe infections, with case fatality rates averaging 20-25 percent due to disseminated vascular endothelial infection. The case fatality rate for *R. conorii* and *R. typhi* infections is 1-3 percent, and infected individuals present with similar clinical manifestations including fever, headache, myalgia, cough, nausea, vomiting. A rash often develops three to five days after symptoms begin. The case fatality rate is lower in children.

*Brucella* spp. are small, non-spore forming non-motile aerobic gram-negative coccobacilli. Once inside the body, the Brucella spp. are rapidly phagocytized by polymorphonuclear cells (PMNs) and *macrophages,* but may still survive intracellularly and remain viable. The mechanism(s) by which the organisms evade intracellular killing by PMNs is not completely understood; however, it may include suppression of the PMN myeloperoxide-$H_2O_2$-halide system, and a copper-zinc superoxide dismutase, which eliminates reactive oxygen intermediates. Intracellular survival within macrophages may be due to the inhibition of phagosome-lysosome fusion by soluble *Brucella* products. The smooth lipopolysaccharide (S-LPS) component of the outer cell wall is the major cell wall antigen and virulence factor. Non-smooth strains have reduced virulence and are more susceptible to lysis by normal serum. The genomic sequence of one strain of *B. suis* strain 1330 has just been completed, and published with the sequence of a second strain associated with sheep brucellosis nearing completion. The genomic sequence of B. melitensis strain 16M was completed and published earlier in 2002.

*Burkholderia mallei* and *B. pseudomallei* are both aerobic gram-negative bacilli: *B. mallei* is nonmotile while *B. pseudomallei* is motile. Very little is known about the molecular mechanisms underlying *Burkholderia* virulence. The polysaccharide capsule of *B. pseudomallei* is one important virulence factor, and toxins as well as type II lipopolysaccharides have also been proposed to play a role. The genomic sequencing *of B. mallei* is nearing completion, whereas that *of B. pseudomallei* is in progress.

*Coxiella burnetii* is a gram-negative, highly pleomorphic coccobacillus. It enters host phagocytes passively through existing cellular receptors, where it survives within the phagolysosome. A low pH is necessary for the metabolism of the organism. In nature, *C. burnetii* is resistant to complement and is a potent immunogen. The cell wall has an immunomodulatory activity that produces toxic reactions in mice. The genomic sequence of the Nine Mile strain of *C. burnetii* has been completed.

Rickettsiae are small, gram-negative, obligatory intracellular bacteria that reside mainly in the cytosol of endothelial cells or in cells of their arthropod host. The organism undergoes local proliferation at the site of the louse bite, disseminates through the blood, and then infects endothelial cells of capillaries, small arteries and veins. Spotted fever rickettsiae spread from cell to cell by acting-based mobility, and the infected cells are injured by the production of reactive oxygen species. Typhus group rickettsiae proliferate within the cytosol until the cell bursts. The genomic sequences of *R. prowazekii* (Madrid E strain) and *R. conorii* (Mulish 7 strain) have been completed, and those of *R. typhi* and *R. rickettsii* are nearing completion.

ARCHAEOBACTERIA

The methods described herein can be used to detect various types of pathogens including, but not limited to pathogens from any of the following genera of the domain of Archaea (or Archaeobacteria): Acidilobus, Aeropyrum, Archaeoglobus, Caldisphaera, Caldivirga, Desulfurococcus, Desulfurolobus, Ferroglobus, Ferroplasma, Geoglobus, Haloarcula, Halobacterium, Halobaculum, Halobiforma, Halococcus, Haloferax, Halogeometricum, Halomethanococcus, Halorhabdus, Halorubrobacterium, Halorubrum, Halosimplex, Haloterrigena, Hyperthermus, Ignicoccus, Metallosphaera, Methanimicrococcus, Methanobacterium, Methanobrevibacter, Methanocalculus, Methanocaldococcus, Methanococcoides, Methanococcus, Methanocorpusculum, Methanoculleus, Methanofollis, Methanogenium, Methanohalobium, Methanohalophilus, Methanolacinia, Methanolobus, Methanomicrobium, Methanomicrococcus, Methanoplanus, Methanopyrus, Methanosaeta, Methanosalsum, Methanosarcina, Methanosphaera, Methanospirillum, Methanothermobacter, Methanothermococcus, Methanothermus, Methanothrix, Methanotomis, Natrialba, Natrinema, Natronobacterium, Natronococcus, Natronomonas, Natronorubrum, Palaeococcus, Picrophilus, Pyrobaculum, Pyrococcus, Pyrodictium, Pyrolobus, Staphylothermus, Stetteria, Stygiolobus, Sulfolobus, Sulfophobococcus, Sulfurisphaera, Sulfurococcus, Thermocladium, Thermococcus, Thermodiscus, Thermofilum, Thermoplasma, Thermoproteus, Thermosphaera, and Vulcanisaeta.

EUBACTERIA

The methods described herein can be used to detect various types of pathogens including, but not limited to, pathogens from any of the following genera of the domain of Bacteria (or Eubacteria): Abiotrophia, Acetitomaculum, Acetivibrio, Acetoanaerobium, Acetobacter, Acetobacterium, Acetofilamentum, Acetogenium, Acetohalobium, Acetomicrobium, Acetonema, Acetothermus, Acholeplasma, Achromatium, Achromobacter, Acidaminobacter, Acidaminococcus, Acidimicrobium, Acidiphilium, Acidisphaera, Acidithiobacillus, Acidobacterium, Acidocella, Acidomonas, Acidothermus, Acidovorax, Acinetobacter, Acrocarpospora, Actinoalloteichus, Actinobacillus, Actinobaculum, Actinobispora, Actinocorallia, Actinokineospora, Actinomadura, Actinomyces, Actinoplanes, Actinopolymorpha, Actinopolyspora, Actinopycnidium, Actinosporangium, Actinosynnema, Aegyptianella, Aequorivita, Aerococcus, Aeromicrobium, Aeromonas, Afipia, Agitococcus, Agreia, Agrobacterium, Agrococcus, Agromonas, Agromyces, Ahrensia , Albibacter, Albidovulum, Alcaligenes, Alcalilimnicola, Alcanivorax, Algoriphagus, Alicycliphilus, Alicyclobacillus, Alishewanella, Alistipes, Alkalibacterium, Alkalilimnicola, Alkaliphilus, Alkalispirillum, Alkanindiges, Allisonella, Allochromatium, Allofustis, Alloiococcus, Allomonas, Allorhizobium, Alterococcus, Alteromonas, Alysiella, Amaricoccus, Aminobacter, Aminobacterium, Aminomonas, Ammonifex, Ammoniphilus, Amoebobacter, Amorphosporangium, Amphibacillus, Ampullariella, Amycolata, Amycolatopsis, Anaeroarcus, Anaerobacter, Anaerobaculum, Anaerobiospirillum, Anaerobranca, Anaerococcus, Anaerofilum, Anaeroglobus, Anaerolinea, Anaeromusa, Anaeromyxobacter, Anaerophaga, Anaeroplasma, Anaerorhabdus, Anaerosinus, Anaerostipes, Anaerovibrio, Anaerovorax, Anaplasma, Ancalochloris, Ancalomicrobium, Ancylobacter, Aneurinibacillus, Angiococcus, Angulomicrobium, Anoxybacillus, Anoxynatronum, Antarctobacter, Aquabacter, Aquabacterium, Aquamicrobium, Aquaspirillum, Aquifex, Arachnia, Arcanobacterium, Archangium, Arcobacter, Arenibacter, Arhodomonas, Arsenophonus, Arthrobacter, Asaia, Asanoa, Asteroleplasma, Asticcacaulis, Atopobacter, Atopobium, Aurantimonas, Aureobacterium, Azoarcus, Azomonas, Azomonotrichon, Azonexus, Azorhizobium, Azorhizophilus, Azospira, Azospirillum, Azotobacter, Azovibrio, Bacillus, Bacterionema, Bacteriovorax, Bacteroides, Bactoderma, Balnearium, Balneatrix, Bartonella, Bdellovibrio, Beggiatoa, Beijerinckia, Beneckea, Bergeyella, Beutenbergia, Bifidobacterium, Bilophila, Blastobacter, Blastochloris, Blastococcus, Blastomonas, Blattabacterium, Bogoriella, Bordetella, Borrelia, Bosea, Brachybacterium, Brachymonas, Brachyspira, Brackiella, Bradyrhizobium, Branhamella, Brenneria, Brevibacillus, Brevibacterium, Brevinema, Brevundimonas, Brochothrix, Brucella, Brumimicrobium, Buchnera, Budvicia, Bulleidia, Burkholderia, Buttiauxella, Butyrivibrio, Caedibacter, Caenibacterium, Calderobacterium, Caldicellulosiruptor, Caldilinea, Caldimonas, Caldithrix, Caloramator, Caloranaerobacter, Calymmatobacterium, Caminibacter, Caminicella, Campylobacter, Capnocytophaga, Capsularis, Carbophilus, Carboxydibrachium, Carboxydobrachium, Carboxydocella, Carboxydothermus, Cardiobacterium, Camimonas, Carnobacterium, Caryophanon, Caseobacter, Catellatospora, Catenibacterium, Catenococcus, Catenuloplanes, Catonella, Caulobacter, Cedecea, Cellulomonas, Cellulophaga, Cellulosimicrobium, Cellvibrio, Centipeda, Cetobacterium, Chainia, Chelatobacter, Chelatococcus, Chitinophaga, Chlamydia, Chlamydophila, Chlorobaculum, Chlorobium, Chloroflexus, Chloroherpeton, Chloronema, Chondromyces, Chromatium, Chromobacterium, Chromohalobacter, Chryseobacterium, Chryseomonas, Chrysiogenes, Citricoccus, Citrobacter, Clavibacter, Clevelandina, Clostridium, Cobetia, Coenonia, Collinsella, Colwellia, Comamonas, Conexibacter, Conglomeromonas, Coprobacillus, Coprococcus, Coprothermobacter, Coriobacterium, Corynebacterium, Couchioplanes, Cowdria, Coxiella, Craurococcus, Crenothrix, Crinalium (not validly published), Cristispira, Croceibacter, Crocinitomix, Crossiella, Cryobacterium, Cryomorpha, Cryptobacterium, Cryptosporangium, Cupriavidus, Curtobacterium, Cyclobacterium, Cycloclasticus, Cystobacter, Cytophaga, Dactylosporangium, Dechloromonas, Dechlorosoma, Deferribacter, Defluvibacter, Dehalobacter, Dehalospirillum, Deinobacter, Deinococcus, Deleya, Delftia, Demetria, Dendrosporobacter, Denitrobacterium, Denitrovibrio, Dermabacter, Dermacoccus, Dermatophilus, Derxia, Desemzia, Desulfacinum, Desulfitobacterium, Desulfobacca, Desulfobacter, Desulfobacterium, Desulfobacula, Desulfobulbus, Desulfocapsa, Desulfocella, Desulfococcus, Desulfofaba, Desulfofrigus, Desulfustis ,Desulfohalobium, Desulfomicrobium, Desulfomonas, Desulfomonile, Desulfomusa, Desulfonatronovibrio, Desulfonatronum, Desulfonauticus, Desulfonema, Desulfonispora, Desulforegula, Desulforhabdus, Desulforhopalus, Desulfosarcina, Desulfospira, Desulfosporosinus, Desulfotalea, Desulfotignum, Desulfotomaculum, Desulfovibrio, Desulfovirga, Desulfurella, Desulfurobacterium, Desulfuromonas, Desulfuromusa, Dethiosulfovibrio, Devosia, Dialister, Diaphorobacter, Dichelobacter, Dichotomicrobium, Dictyoglomus, Dietzia, Diplocalyx, Dolosicoccus, Dolosigranulum, Dorea, Duganella, Dyadobacter, Dysgonomonas, Ectothiorhodospira, Edwardsiella, Eggerthella, Ehrlichia, Eikenella, Elytrosporangium, Empedobacter, Enhydrobacter, Enhygromyxa, Ensifer, Enterobacter, Enterococcus, Enterovibrio, Entomoplasma, Eperythrozoon, Eremococcus, Erwinia, Erysipelothrix, Erythrobacter, Erythromicrobium, Erythromonas, Escherichia, Eubacterium, Ewingella, Excellospora, Exiguobacterium, Facklamia, Faecalibacterium, Faenia, Falcivibrio, Ferribacterium, Ferrimonas, Fervidobacterium, Fibrobacter, Filibacter, Filifactor, Filobacillus, Filomicrobium, Finegoldia, Flammeovirga, Flavimonas, Flavobacterium, Flectobacillus, Flexibacter, Flexistipes, Flexithrix, Fluoribacter, Formivibrio, Francisella, Frankia, Frateuria, Friedmanniella, Frigoribacterium, Fulvimarina, Fulvimonas, Fundibacter, Fusibacter, Fusobacterium, Gallibacterium, Gallicola, Gallionella, Garciella, Gardnerella, Gelidibacter, Gelria, Gemella, Gemmata, Gemmatimonas, Gemmiger, Gemmobacter, Geobacillus, Geobacter, Geodermatophilus, Georgenia, Geothrix , Geotoga, Geovibrio, Glaciecola, Globicatella, Gluconacetobacter, Gluconoacetobacter, Gluconobacter, Glycomyces, Gordonia, Gordonia, Gracilibacillus, Grahamella, Granulicatella, Grimontia, Haemobartonella, Haemophilus, Hafnia, Hahella, Halanaerobacter, Halanaerobium, Haliangium, Haliscomenobacter, Hallella, Haloanaerobacter, Haloanaerobium, Halobacillus, Halobacteroides, Halocella, Halochrornatium, Haloincola, Halomicrobium, Halomonas, Halonatronum, Halorhodospira, Halospirulina, Halothermothrix, Halothiobacillus, Halovibrio, Helcococcus, Heliobacillus, Helicobacter, Heliobacterium, Heliophilum, Heliorestis, Heliothrix, Herbaspirillum, Herbidospora, Herpetosiphon, Hippea, Hirschia, Histophilus, Holdemania, Hollandina, Holophaga, Holospora, Hongia, Hydrogenobacter, Hydrogenobaculum, Hydrogenophaga, Hydrogenophilus, Hydrogenothermus, Hydrogenovibrio, Hymenobacter, Hyphomicrobium, Hyphomonas, Ideonella, Idiomarina, Ignavigranum, Ilyobacter, Inquilinus, Intrasporangium, Iodobacter, Isobaculum, Isochromatium, Isosphaera, Janibacter, Jannaschia, Janthinobacterium, Jeotgalibacillus, Jeotgalicoccus, Johnsonella, Jonesia, Kersteria, Ketogulonicigenium, Ketogulonigeniurn, Kibdelosporangium, Kineococcus, Kineosphaera, Kineosporia, Kingella, Kitasatoa, Kitasatospora, Kitasatosporia, Klebsiella, Kluyvera, Knoellia, Kocuria, Koserella, Kozakia, Kribbella, Kurthia, Kutzneria, Kytococcus, Labrys, Lachnobacterium, Lachnospira, Lactobacillus, Lactococcus, Lactosphaera, Lamprobacter, Lamprocystis, Lampropedia, Laribacter, Lautropia, Lawsonia, Lechevalieria, Leclercia, Legionella, Leifsonia, Leisingera, Leminorella, Lentibacillus, Lentzea, Leptonema, Leptospira, Leptospirillum, Leptothrix, Leptotrichia, Leucobacter, Leuconostoc, Leucothrix, Levinea ,Lewinella, Limnobacter, Limnothrix, Listeria, Listonella, Lonepinella, Longispora, Lucibacterium, Luteimonas, Luteococcus, Lysobacter, Lyticum, Macrococcus, Macromonas, Magnetospirillum, Malonomonas, Mannheimia, Maricaulis, Marichromatium, Marinibacillus, Marinilabilia, Marinilactibacillus, Marinithermus, Marinitoga, Marinobacter, Marinobacterium, Marinococcus, Marinomonas, Marinospirillum, Marmoricola, Massilia, Megamonas, Megasphaera, Meiothermus, Melissococcus, Melittangium, Meniscus, Mesonia, Mesophilobacter, Mesoplasma, Mesorhizobium, Methylarcula, Methylobacillus, Methylobacter, Methylobacterium, Methylocaldum, Methylocapsa, Methylocella, Methylococcus, Methylocystis, Methylomicrobium, Methylomonas, Methylophaga, Methylophilus, Methylopila, Methylorhabdus, Methylosarcina, Methylosinus, Methylosphaera, Methylovorus, Micavibrio, Microbacterium, Microbispora, Microbulbifer, Micrococcus, Microcyclus, Microcystis, Microellobosporia, Microlunatus, Micromonas, Micromonospora, Micropolyspora, Micropruina, Microscilla, Microsphaera, Microtetraspora, Microvirga, Microvirgula, Mitsuokella, Mobiluncus, Modestobacter, Moellerella, Mogibacterium, Moorella, Moraxella, Morganella, Moritella, Morococcus, Muricauda, Muricoccus, Mycetocola, Mycobacterium, Mycoplana, Mycoplasma, Myroides, Myxococcus, Nannocystis, Natroniella, Natronincola, Natronoincola, Nautilia, Neisseria, Neochlamydia, Neorickettsia, Neptunomonas, Nesterenkonia, Nevskia, Nitrobacter, Nitrococcus, Nitrosococcus, Nitrosolobus, Nitrosomonas, Nitrosospira, Nitrospina, Nitrospira, Nocardia, Nocardioides, Nocardiopsis, Nonomuraea, Nonomuria, Novosphingobium, Obesumbacterium, Oceanicaulis, Oceanimonas, Oceanisphaera, Oceanithermus, Oceanobacillus, Oceanobacter, Oceanomonas, Oceanospirillum, Ochrobactrum, Octadecabacter, Oenococcus, Oerskovia, Okibacterium, Oleiphilus, Oleispira, Oligella, Oligotropha, Olsenella, Opitutus, Orenia, Oribaculum, Orientia, Ornithinicoccus, Ornithinimicrobium, Omithobacterium, Oscillochloris, Oscillospira, Oxalicibacterium, Oxalobacter, Oxalophagus, Oxobacter, Paenibacillus, Pandoraea, Pannonibacter, Pantoea, Papillibacter, Parachlamydia, Paracoccus, Paracraurococcus, Paralactobacillus, Paraliobacillus, Parascardovia, Parvularcula, Pasteurella, Pasteuria, Paucimonas, Pectinatus, Pectobacterium, Pediococcus, Pedobacter, Pedomicrobium, Pelczaria, Pelistega, Pelobacter, Pelodictyon, Pelospora, Pelotomaculum, Peptococcus, Peptoniphilus, Peptostreptococcus, Persephonella, Persicobacter, Petrotoga, Pfennigia, Phaeospirillum, Phascolarctobacterium, Phenylobacterium, Phocoenobacter, Photobacterium, Photorhabdus, Phyllobacterium, Pigmentiphaga, Pilimelia, Pillotina, Pimelobacter, Pirella, Pirellula, Piscirickettsia, Planctomyces, Planktothricoides, Planktothrix, Planobispora, Planococcus, Planomicrobium, Planomonospora, Planopolyspora, Planotetraspora, Plantibacter, Pleisomonas, Plesiocystis, Plesiomonas, Polaribacter, Polaromonas, Polyangium, Polynucleobacter, Porphyrobacter, Porphyromonas, Pragia, Prauserella, Prevotella, Prochlorococcus, Prochloron, Prochlorothrix, Prolinoborus, Promicromonospora, Propionibacter, Propionibacterium, Propionicimonas, Propioniferax, Propionigenium, Propionimicrobium, Propionispira, Propionispora, Propionivibrio, Prosthecobacter, Prosthecochloris, Prosthecomicrobium, Proteus, Protomonas, Providencia, Pseudaminobacter, Pseudoalteromonas, Pseudoamycolata, Pseudobutyrivibrio, Pseudocaedibacter, Pseudomonas, Pseudonocardia, Pseudoramibacter, Pseudorhodobacter, Pseudospirillum, Pseudoxanthomonas, Psychrobacter, Psychroflexus, Psychromonas, Psychroserpens, Quadricoccus, Quinella, Rahnella, Ralstonia, Ramlibacter, Raoultella, Rarobacter, Rathayibacter, Reichenbachia, Renibacterium, Rhabdochromatium, Rheinheimera, Rhizobacter, Rhizobium, Rhizomonas, Rhodanobacter, Rhodobaca, Rhodobacter, Rhodobium, Rhodoblastus, Rhodocista, Rhodococcus, Rhodocyclus, Rhodoferax, Rhodoglobus, Rhodomicrobium, Rhodopila, Rhodoplanes, Rhodopseudomonas, Rhodospira, Rhodospirillum, Rhodothalassium, Rhodothermus, Rhodovibrio, Rhodovulum, Rickettsia, Rickettsiella, Riemerella, Rikenella, Rochalimaea, Roseateles, Roseburia, Roseibium, Roseiflexus, Roseinatronobacter, Roseivivax, Roseobacter, Roseococcus, Roseomonas, Roseospira, Roseospirillum, Roseovarius, Rothia, Rubrimonas, Rubritepida, Rubrivivax, Rubrobacter, Ruegeria, Rugamonas, Ruminobacter, Runninococcus, Runella, Saccharobacter, Saccharococcus, Saccharomonospora, Saccharopolyspora, Saccharospirillum, Saccharothrix, Sagittula, Salana, Salegentibacter, Salibacillus, Salinibacter, Salinibacterium, Salinicoccus, Salinisphaera, Salinivibrio, Salmonella, Samsonia, Sandaracinobacter, Sanguibacter, Saprospira, Sarcina, Sarcobium, Scardovia, Schineria, Schlegelella, Schwartzia, Sebaldella, Sedimentibacter, Selenihalanaerobacter, Selenomonas, Seliberia, Serpens, Serpula, Serpulina, Serratia, Shewanella, Shigella, Shuttleworthia, Silicibacter, Simkania, Simonsiella, Sinorhizobium, Skermanella, Skermania, Slackia, Smithella, Sneathia, Sodalis, Soehngenia, Solirubrobacter, Solobacterium, Sphaerobacter, Sphaerotilus, Sphingobacterium, Sphingobium, Sphingomonas, Sphingopyxis, Spirilliplanes, Spirillospora, Spirillum, Spirochaeta, Spiroplasma, Spirosoma, Sporanaerobacter, Sporichthya, Sporobacter, Sporobacterium, Sporocytophaga, Sporohalobacter, Sporolactobacillus, Sporomusa, Sporosarcina, Sporotomaculum, Staleya, Staphylococcus, Stappia, Starkeya, Stella, Stenotrophomonas, Sterolibacterium, Stibiobacter, Stigmatella, Stomatococcus, Streptacidiphilus, Streptimonospora, Streptoalloteichus, Streptobacillus, Streptococcus, Streptomonospora, Streptomyces: S.abikoensis, S.erumpens, S.erythraeus, S.michiganensis, S. microflavus, S. zaomyceticus, Streptosporangium, Streptoverticillium, Subtercola, Succiniclasticum, Succinimonas, Succinispira, Succinivibrio, Sulfitobacter, Sulfobacillus, Sulfurihydrogenibium, Sulfurimonas, Sulfurospirillum, Sutterella, Suttonella, Symbiobacterium, Symbiotes, Synergistes, Syntrophobacter, Syntrophobotulus, Syntrophococcus, Syntrophomonas, Syntrophospora, Syntrophothermus, Syntrophus, Tannerella, Tatlockia, Tatumella, Taylorella, Tectibacter, Teichococcus, Telluria, Tenacibaculum, Tepidibacter, Tepidimonas, Tepidiphilus, Terasakiella, Teredinibacter, Terrabacter, Terracoccus, Tessaracoccus, Tetragenococcus, Tetrasphaera, Thalassomonas, Thalassospira, Thauera, Thermacetogenium, Thermaerobacter, Thermanaeromonas, Thermanaerovibrio, Thermicanus, Thermithiobacillus, Thermoactinomyces, Thermoanaerobacter, Thermoanaerobacterium, Thermoanaerobium, Thermobacillus, Thermobacteroides, Thermobifida, Thermobispora, Thermobrachium, Thermochromatium, Thermocrinis, Thermocrispum, Thermodesulfobacterium, Thermodesulforhabdus, Thermodesulfovibrio, Thermohalobacter, Thermohydrogenium, Thermoleophilum, Thermomicrobium, Thermomonas, Thermomonospora, Thermonema, Thermosipho, Thermosyntropha, Thermoterrabacterium, Thermothrix, Thermotoga, Thermovenabulum, Thermovibrio, Thermus, Thialkalicoccus, Thialkalimicrobium, Thialkalivibrio, Thioalkalicoccus, Thioalkalimicrobium, Thioalkalispira, Thioalkalivibrio, Thiobaca, Thiobacillus, Thiobacterium, Thiocapsa, Thiococcus, Thiocystis, Thiodictyon, Thioflavicoccus, Thiohalocapsa, Thiolamprovum, Thiomargarita, Thiomicrospira, Thiomonas, Thiopedia, Thioploca, Thiorhodococcus, Thiorhodospira, Thiorhodovibrio, Thiosphaera, Thiospira, Thiospirillum, Thiothrix, Thiovulum, Tindallia, Tissierella, Tistrella, Tolumonas, Toxotbrix, Trabulsiella, Treponema, Trichlorobacter, Trichococcus, Tropheryma, Tsukamurella, Turicella, Turicibacter, Tychonema, Ureaplasma, Ureibacillus, Vagococcus, Vampirovibrio, Varibaculum, Variovorax, Veillonella, Verrucomicrobium, Verrucosispora, Vibrio, Victivallis, Virgibacillus, Virgisporangium, Virgosporangium, Vitellibacter, Vitreoscilla, Vogesella, Volcaniella, Vulcanithermus, Waddlia, Weeksella, Weissella, Wigglesworthia, Williamsia, Wolbachia, Wolinella, Xanthobacter, Xanthomonas, Xenophilus, Xenorhabdus, Xylanimonas, Xylella, Xylophilus, Yersinia, Yokenella, Zavarzinia, Zobellia, Zoogloea, Zooshikella, Zymobacter, Zymomonas, and Zymophilus.

To date, the complete sequence for a number of bacterial genomes and viral genomes have been deposited in various databases and are publicly available, e.g., GenBank, The Institute for Genomic Research, www.tigr.org; GOLD genomes online database, integrated genomics; igweb.integratedgenomics.com/GOLD, www.ncbi.nlm.nih.gov/PMGifs/Genomes/10239.html. Fungal genomic information is also known in the art, e.g., see http://www.ncbi.nhn.nih.gov/genomes.

Surprisingly, up to 25% of a microorganism's open reading frames are unique (i.e., specific) to that genus or species, which indicates enormous diversity among microorganisms (Pucci MJ , B. T., Dougherty TJ. 2002. Bacterial "genes-to screens", p. 83-96. In K. Shaw (ed.), Pathogen Genomics. Humana Press Inc, Totowa, NJ.). With these data, diagnostics based on genetic sequence analysis becomes a powerful tool. Moreover, as antibiotic resistance genes are characterized, they also become a potential target for nucleic acid based detection and identification. WFCC-MIRCEN World Data Centre for Microorganisms (WDCM) provides a comprehensive directory of culture collections, databases on microbes and cell lines, and the gateway to biodiversity, molecular biology and genome projects (see http://wdcm.nig.ac.jp/). WDCM provides links to (1) microbial genome projects including: Bacillus subtilis Genome Database (BSORF) Bioinformatics Ceter, Kyoto University and Nara Institute of Science and Technology; Chlamydomonas Resource Center Duke University, USA; Database of Genomes Analysed in NITE (DOGAN); Dictyostelium cDNA Database Dictyostelium discoideum cDNA Project (Dicty_cDB); Dictyostelium Genome Sequencing Project Baylor College of Medicine; E-coli genome project (K-12 and -157) University of Wisconsin-Madison, US; Genome Analysis Project Japan on E. coli (GenoBase) Nara Institute of Science and Technology; Genome Database for Cyanobacteria (CyanoBase) Kazusa DNA Research Institute; Genome Information Broker (GIB) DNA Data Bank of Japan (84 microbes as of May 2002); Genome to Proteins and Functions; GOLD: Genomes OnLine Database HomePage by Integrated Genomics Inc., US; JGI Programs: Microbial Genomics DOE Joint Genome Institute; MagnaportheDB; Malaria Full-Length cDNA Database (Plasmodium falciparum) Institute of Medical Science, The University of Tokyo, Japan; Microbial Genome Database for Comparative Analysis (MBGD); PEDANT: Genome Analaysis and Annotation by MIPS, Germany; Profiling of E.coli Chromosome (PEC); Saccharomyces Genome Information Server; Synechocystis PCC6803 Gene Annotation Database (SYORF) Bioinformatics Ceter, Kyoto University and Cyanobacteria Research Community; The Institute for Genomic Research; (2)Microbial Genetic Stock Center including E. coli genetic resources National Institute of Genetics; E. coli Genetic Stock Center Collection (CGSC) Yale University, USA; Fungal Genetics Stock Center (FGSC), USA; Internet Directory of Biotechnology Resources; PGSC Pseudomonas Genetic Stock Center USA); The Microorganisms Section of the MAFF Gene Bank; Worldwide E.coli Stocks and Databases; (3)Other Genome Projects including: Aberrant Splicing Database HGC, University of Tokyo; Arabidopsis Information Resource TAIR; BODYMAP Anatomical Expression Database of Human Genes; BodyMap: Human and Mouse Gene Expression Database; Danish Centre For Human Genome Research Biobase, the Danish Biotechnological Database, at University of Aarhus, Denmark; DDBJ International Nucleotide Sequence Database; DNA Information and Stock Center (DISC); FlyBase: a genetic and molecular database for Drosophila NIG, Japan; Flybase: The Berkeley Drosophila Genome Project; GDB: The Genome Database; GenomeNet Bioinformatics Center, Institute for Chemical Research, Kyoto University; GENOTK: Human cDNA Database Otsuka GEN Research Institute and HGC, University of Tokyo; HOWDY (Human genome) Japan Science and Technology Corporation, Japan; Human Chromosome 21 Sequence Map RIKEN Genomic Sciences Center(GSC), Human Genome Research Group; Human Unidentified Gene-Encoded Large Proteins (HUGE) Kazusa DNA Research Institute; Human Genome Project Information; Human Genome Sequencing Center (former Biologist's Control Panel); INE (Rice Genome Research Program, Japan); John Wiley & Sons, Ltd.; JST Human Genome Sequencing Page Japan Science and Technology Corporation; MAGEST: Maboya (H. roretzi) Gene Expression Patterns and Sequence Tags Kyoto University; Medical Research Council; Metabolic Pathway; Moulon WWW server; Mouse Encyclopedia Index RIKEN Genomic Sciences Center; Mouse Genome Informatics (MGI) ; Munich Information Center for Protein Sequences Germany; NCBI Genbank; NEXTDB: Nematode Expression Pattern Database National Institute of Genetics; National Institutes of Health (NIH) ; Nucleic Acid Database Project (NDB) ; p53MDB: p53 Mutation Database HGC, University of Tokyo; RAT GENOME MAP Otsuka GEN Research Institute, Oxford University, Cambridge University, Research Genetics, Inc., and HGC, University of Tokyo; Rice Genome Research Program (RGP) ; SPAD: Signaling Pathway Database Kyushu University; The Integrated Mycobacterial Database (MycDB) ; The OGMP; UK MRC Human Genome Mapping Project Resource Centre.

Described herein are approaches to the detection of the presence and measurement of the levels of target nucleic acids specific to pathogens, including viral, bacterial, protaozoan and fungal pathogens, particularly viral, bacterial, and protozoan pathogens, for the purpose of detecting pathogens, in a biological sample, particularly in a sample obtained from an immunosuppressed patient. The methods permit the quantitation of pathogen specific target nucleic acids, e.g., pathogenic derived DNAs or RNAs present in a nucleic acid sample, both singly and in a multiplex format that permits the determination of levels (e.g., expression levels or copy numbers) for two or more target nucleic acids in a single reaction.

Additional pathogens encompassed by the methods and kits described herein include the following protozoa Cryptosporidium parvum, Cyclospora cayatenensis, Giardia lamblia, Entamoeba histolytica, Toxoplasma and Microsporidia. PROTOZOA

The methods described herein can be used to detect protazoan pathogens. Enteric protozoa and protists are included among the category B agents due to their potential for dissemination through compromised food and water supplies in the United States. Many of these organisms infect domestic and wild animals. These organisms include the protozoa *Cryptosporidium parvum, Cyclospora cayetanensis, Giardia lamblia, Entamoeba histolytica,* and *Toxoplasma gondii,* and the protists Microsporidia species such as *Encephalitozoon* and *Enterocytozoon.* Although infections by most of these organisms are usually asymptomatic or self-limiting in otherwise healthy persons, clinical symptoms occur in immunosuppressed persons.

The most important organisms in terms of bioterrorist potential include *C. parvum, E. histolytica* and *T gondii.* These organisms can infect large numbers of people through contaminated water and/or food. In addition, all these infections (with the exception of toxoplasmosis), can be easily transmitted person-to-person and are difficult to diagnosis. Also most can be genetically manipulated to increase virulence or resistance to anti-infectives.

The life cycles of most Category B food- and water-borne protozoa and protists are well understood. However, experimental studies of some of these organisms are limited by difficulties with *in vitro* cultivation and by the lack of animal models.

Ingestion of *C. parvum* oocsts leads to infection of intestinal epithelial cells, where the organism replicates within protective vacuoles. Because autoinfection can occur when released oocsts are released from the cells, ingestion of only a few oocsts can lead to severe and persistent infections in immunocompromised patients. The mechanism of pathogenesis is not well understood, but *C. parvum* may disrupt intestinal ion transport. Two distinct genotypes of *C.*

*parvum* infect humans, with the sequencing of genotype I almost complete and work on genotype II in progress.

*Cyclospora cayetanensis* was identified in association with diarrheal disease in 1979 although its taxonomical classification was not resolved until 1993. Oocysts are the infectious form and are resistant to both freezing and chlorination. The oocyst contains two sporocysts that each hold two sporozoites. Infection of the small intestine can result in atrophy of the villi and inflammatory infiltration of the lamina propnia. It is not known whether *C. cayetanensis* pathogenesis is due to a direct effect on enterocytes or involves a secreted toxin.

The trophozoite form of *G. lamblia* colonizes the small intestine after ingestion of as few as 10 to 25 cysts. The trophozoite consists of four flagellae and a sucking or adhesive disc, including microtubular structures that serve as important antigens for host recognition. The mechanism of adherence to epithelium is uncertain, but may involve specific receptors. Trophozoites undergo antigenic variation by changing a cystein-rich surface protein to variant specific surface protein (VSSP); these surface proteins also bind metals, such as zinc, that are important for brush border enzymes. Cell-mediated immune responses may play a role in histological damage of the intestine; no enterotoxin has been identified. There is a genome project for *G. lamblia* and gene expression data are also available.

Like *Giardia,* the life cycle of *E. histolytica* consists of trophozoites and cysts. Information about the pathogenesis of *E. histolytica* has been expanding rapidly due to development of new culture media. Adherence to intestinal epithelium is critical in pathogenesis as trophozoites kill target cells only on direct contact; adherence is mediated by the parasite's surface lectin. Other parasitic factors have been identified that degrade secretory IgA, mucins, and other host cell surface glycoproteins, and contribute to cell killing. Sequencing of the *E. histolytica* genome is in progress.

*Toxoplasma gondii* exists in three forms: oocysts, tissue cysts containing bradyzoites, and tachyzoites. Oocysts form only in the intestines of infected cats. Following ingestion, sporozoites, released from oocysts, penetrate and multiply in intestinal epithelial cells. Invasion of epithelial cells appears to be mediated via the conoid, a cone-shaped structure on the tachyzoite. Tachyzoites are contained within vacuoles within the epithelium, protected from lysosomal fusion, and destroy the host cell before spreading to lymph nodes and other tissues. Cyst formation occurs in infected tissues, including brain, retina, and muscles. Delayed-type hypersensitivity reactions result in rupture of the tissue cysts and necrosis of surrounding tissue, which can be clinically important in the retina. In immunocompromised hosts, reactivation can lead to significant tissue damage and result in death. Transplacental infection can also occur, and fetal infection occurs in 30% to 40% of women first infected with T. *gondii* during pregnancy. Genomic sequencing of *T. gondii* is in progress, with an extensive database of genomic and EST sequences now available.

Microsporidia are a unique group of intracellular, spore-forming protists. Microsporidia species that infect humans include *Encephalitozoon intestinalis, Enc. hellem, Enc. cuniculi,* and *Enterocytozoon bieneusi*, which is resistant to therapy. The spore consists of a resistant wall, one or two nuclei, sporoplasm, an anchoring disk, and a spiral coiled polar tube. During infection, the polar tube events, piercing the host cell and injecting the sporoplasm. Replication results in an increasing number of mature spores, which eventually rupture the cell. As with *C. parvum,* the potential for autoinfection increases production of the spores. Infection is usually limited to the intestine except in immunocompromised individuals where many tissues may be involved. The complete genomic sequence of *Enc. cuniculi* has been completed and sequencing of *Ent. bieneusi* is planned.

Quantitative Aspects of the Methods Described Herein:

In one aspect, the methods described herein use internal standards generated through the use of known differing concentrations of exogenously added competitor nucleic acids that generate amplification products of known sizes that differ from each other and from the size of the pathogen specific target nucleic acid(s). Size separation by, for example, capillary electrophoresis, coupled with detection by, for example, fluorescence detection, generates a standard curve from the abundance of the amplification products corresponding to the competitor nucleic acids. The standard curve permits the determination of the pathogen specific target nucleic acid concentration(s) in the original sample.

In one aspect, then, there is described a method of estimating and/or determining the level of a pathogen-specific target nucleic acid in a nucleic acid sample. That method comprises the following steps. First, for a given pathogen a target molecule is selected, and is specific to that pathogen in the sense that the target molecule will not react with other pathogen target molecules present in the assay. Then, for each given pathogen specific target nucleic acid, a pair of amplification primers is selected that will generate a target amplicon of a known length following reverse-transcription (for RNA target) and amplification (e.g., PCR amplification, for both RNA and DNA targets) using that pair of primers. Considerations for primer design are well known to those of skill in the art; however, among the more critical aspects are specificity, i.e., the primers should amplify only the desired target molecule under at least one set of amplification conditions, and compatibility with additional primers that may be employed in a reaction, e.g., where multiplex analyses are to be performed. The length and nucleotide content (e.g., the G+C content) of the oligonucleotide primer is instrumental in determining the specificity and hybridization characteristics (e.g., melting temperature) of the primer. Further considerations for oligonucleotide primer selection or design are known to those of skill in the art and/or described herein below.

Next, a set of at least two competitor nucleic acids is created. The competitor nucleic acids share the same primer binding sequences (or their complements) for the selected amplification primers as the pathogen specific target nucleic acid, but differ in the length of the amplicon that will be generated using the same set of amplification primers used to amplify the pathogen specific target sequence. It is important that the at least two competitor nucleic acids have similar amplification efficiencies (as the term is defined herein) relative to each other and to the pathogen specific target nucleic acids when the selected pair of amplification primers is used to generate an amplification product from each. In the set of at least two competitor nucleic acids, it is preferred that one competitor generates a longer amplicon using the same primers, and another generates a shorter amplicon. (As discussed herein below, additional longer or shorter competitors can also be included in differing amounts, e.g., to modify the resolution of the assay.) In other embodiments, each of the at least two competitor nucleic acids can generate a longer amplicon than that generated from the target nucleic acid. It should be understood that in this instance, each of the competitors should generate amplicons of differing known lengths relative to each other and to the target amplicon. In other embodiments, each of the at least two competitor nucleic acids can generate a shorter amplicon than that generated from the target nucleic acid - here again, the competitor amplicons must differ by known lengths from each other and from the target amplicon. Methods of generating nucleic acids for use in the methods described herein are well known in the art, e.g., PCR (for DNA competitors) or in vitro transcription from plasmid or other isolated template DNA (for RNA competitors), or chemical synthesis. Methods for PCR, in vitro transcription and for the generation of templates that differ in length from a given DNA template are well known to those of skill in the art and/or described herein below.

The difference in size of the competitor nucleic acid amplicons should be a difference that can be detected by a method capable of distinguishing nuclei acids differing in size by 10 nucleotides/base pairs or less, and preferably by 5 nucleotides/base pairs or less, or even by as little as 1 nucleotide or base pair. A well-suited method is, for example, capillary electrophoresis. Conditions under which capillary electrophoresis permits the detection of length differences of as little as one nucleotide are well known. While differences of as little as one nucleotide are intended to be encompassed within the methods described herein, it is preferable that the difference between competitors and target be at least 5 nucleotides, in order to better resolve the resulting amplicons from the target amplicon upon separation by, for example, capillary electrophoresis. Differences greater than 5 nucleotides are also contemplated, e.g., 10, 20, 30, 40 or 50 nucleotides. However, the difference should not be so great as to render the efficiency of amplification significantly different (i.e., resulting in a difference in amplification efficiency E of greater than 0.2 in absolute value, where $E = (P_{n+1} - P_n)/(P_n-P_{n-1})$ (where $P_n$ is the amount of PCR product at cycle n) with respect to the efficiency of the target amplicon or the at least one other competitor amplicon(s). Factors affecting the efficiency of amplification are well known to those of skill in the art and include, for example, $T_m$ of the primers, the length of the amplicon, nucleotide composition of the amplicon, potential for secondary structure in the target or in the primers, and the presence of, for example, modified nucleotides in the reaction. The measurement of amplification efficiency and factors affecting it are known to those of skill in the art and/or described herein below.

One straightforward approach to generating competitor nucleic acids involves the internal insertion or deletion of sequences from the sequence of the pathogen specific target amplicon. This approach maximizes the similarities between the competitor nucleic acids and the target nucleic acids, which in turn makes it more likely that amplification efficiencies will be similar. Thus, one would perform site-directed mutagenesis on a cloned or amplified copy of the sequence (e.g., a cloned cDNA) corresponding to the target nucleic acid, to either add or delete nucleotide sequence sufficient to change the size of the amplicon generated when the selected pair of primers is used for amplification. Of course, it should be clear that one would not mutate the sequences bound by the selected primer pair. Site-directed mutagenesis can be performed by any of a number of methods well known in the art.

It can be useful to generate sets of three, four or more competitor nucleic acids for each pathogen specific target nucleic acid. Having additional competitors can either expand or more narrowly define the range of quantitative determination within a given assay. That is, when first and second competitors are used at, for example, a range of concentrations between 10 and 10,000 molecules in a reaction, concentrations of target nucleic acid between 10 and 10,000 molecules in a given volume of the original sample can be determined from the standard curve generated by the competitors. While this determination can be quite accurate, a narrower range of competitor concentrations, e.g., 10 to 500 or 1,000 molecules can increase the accuracy. Similarly, where a first estimate is to be made, the range can be broader, e.g., 10 to 50,000 molecules, with later reactions run at narrower concentrations if desired to more accurately determine the target nucleic acid concentration. It can be advantageous to include three, four or more competitor nucleic acids for a given target nucleic acid at different concentrations in a given reaction. One of skill in the art will recognize that as the concentration of competitors goes up, there may need to be an adjustment in the amount of amplification primers or other parameters for the amplification reaction.

Once a pair of amplification primers is selected and a set of competitor nucleic acids is generated, target nucleic acids in a sample can be quantitated by combining a test nucleic acid sample with the set of at least two competitor nucleic acid molecules, reverse transcribing the target and competitor nucleic acids and amplifying the target and competitor sequences using the pair of amplification primers. In an alternative approach, competitor nucleic acids can be added to a sample prior to extraction of nucleic acid from the test sample. In this instance, target and competitor nucleic acids will be co-isolated.

In order to be most accurate, the competitors should be added to the sample such that at least one is added at a known concentration below that of the target nucleic acid and at least one is added at a known concentration above that of the target nucleic acid. The known concentrations of competitor nucleic acids should differ by at least an order of magnitude (i.e., 10-fold), but can advantageously differ by several orders of magnitude, e.g., at 100-fold, 1,000 fold or more. If the amount of target nucleic acid expected is completely unknown, it can be advantageous to perform one or more preliminary experiments using different ranges of competitors, in order to identify an anticipated range of concentrations for the given target. Alternatively, one or another of a number of less accurate quantitative amplification approaches can be employed to garner a rough estimate of the concentration to expect. Such methods are known in the art and use, for example, titration in a series of parallel reactions against a single reference template. '

Reverse transcription is used when the pathogen specific target nucleic acid is an RNA. Reverse transcription is well known in the art and can be performed by an enzyme separate from that used for amplification (e.g., where a reverse transcriptase such as MMLV reverse transcriptase is used) or by the same enzyme (e.g., Tth polymerase or another polymerase known in the art to possess both RNA template-dependent and DNA template-dependent primer extension abilities). Reverse transcription can either be performed in the same reaction mixture as the PCR step (one-step protocol) or reverse transcription can be performed first prior to amplification utilizing PCR (two-step protocol.

Similarly, DNA amplification is well known in the art. Both Taqman and QuantiTect SYBR systems can be used subsequent to reverse transcription of RNA.

Nucleic Acid Amplification Approaches:

The methods described herein lend themselves well to standard PCR in which a pair of selected primers flanking a target sequence directs the template-dependent synthesis of copied DNA. This does not, however, exclude other methods (e.g., ligase-mediated amplification or other, isothermal, amplification methods, e.g., Self-Sustained Sequence Replication (3SR), Gingeras et al., 1990, Annales de Biologie Clinique, 48(7): 498-501; Guatelli et al., 1990, Proc. Natl. Acad. Sci. U.S.A., 87: 1874; see below) that can be adapted to the approach described herein. A key element in any such alternative approach remains achieving similar efficiency of the amplification from a target RNA and a set of at least two competitor nucleic acids.

3SR is an outgrowth of the transcription-based amplification system (TAS), which capitalizes on the high promoter sequence specificity and reiterative properties of bacteriophage DNA-dependent RNA polymerases to decrease the number of amplification cycles necessary to achieve high amplification levels (Kwoh et al., 1989, Proc. Natl. Acad. Sci. U.S.A., 83: 1173-1177).

In 3SR, each priming oligonucleotide contains a bacteriophage RNA polymerase binding sequence and the preferred transcriptional initiation sequence, e.g., the T7 RNA polymerase binding sequence (TAATACGACTCACTATA) and the preferred T7 polymerase transcriptional initiation site. The remaining sequence of each primer is complementary to the target sequence on the molecule to be amplified.

Exemplary 3SR conditions are described herein as follows. The 3SR amplification reaction is carried out in 100 $\mu$l and contains the target RNA, 40 mM Tris-HCl, ph 8.1, 20 mM MgCl$_2$, 2 mM spermidine-HCl, 5mM dithiothreitol, 80 $\mu$g/ml BSA, 1 mM dATP, 1 mM dGTP, 1 mM dTTP, 4 mMATP, 4 mM CTP, 1 mM GTP, 4 mM dTTP, 4 mM ATP, 4 mM CTP, 4 mM GTP, 4 mMUTP, and a suitable amount of oligonucleotide primer (250 ng of a 57-mer; this amount is scaled up or down, proportionally, depending upon the length of the primer sequence). Three to six attomoles of the nucleic acid target for the 3SR reactions is used. As a control for background, a 3SR reaction without any target is run in parallel. The reaction mixture is heated to 100°C for 1 minute, and then rapidly chilled to 42°C. After 1 minute, 10 units (usually in a volume of approximately 2 $\mu$l) of reverse transcriptase, (e.g. avian myoblastosis virus reverse transcriptase, AMV-RT; Life Technologies/Gibco-BRL) is added. The reaction is incubated for 10 minutes, at 42°C and then heated to 100°C for 1 minute. (If a 3SR reaction is performed using a single-stranded template, the reaction mixture is heated instead to 65°C for 1 minute.) Reactions are then cooled to 37°C for 2 minutes prior to the addition of 4.6 $\mu$l of a 3SR enzyme mix, which contains 1.6 $\mu$l of AMV-RT at 18.5 units/$\mu$l, 1.0 $\mu$l T7 RNA polymerase (both e.g. from Stratagene; La Jolla, CA) at 100 units/$\mu$l, and 2.0 $\mu$l *E. Coli* RNase H at 4 units/$\mu$l (e.g. from Gibco/Life Technologies; Gaithersburg, MD). It is well within the knowledge of one of skill in the art to adjust enzyme volumes as needed to account for variations in the specific activities of enzymes drawn from different production lots or supplied by different manufacturers. Variations can also be made to the units of the enzymes as necessary. The reaction is incubated at 37°C for 1 hour and stopped by freezing.

Where the progress of the amplification is to be monitored by sampling, the sampling can be performed at any stage of the 3SR reaction. Because 3SR proceeds continuously at a single temperature, there are not individual cycles at which aliquots will be withdrawn. Thus, sampling can be performed at set times during the amplification incubation period, for example, every minute, every two minutes, every three minutes, etc. Nucleic acids in the aliquots withdrawn or extruded are then separated and nucleic acids detected, thereby permitting the generation of an amplification profile, from which the abundance of target in the initial sample can be determined.

3SR is also referred to by some as Nucleic Acid Sequence Based Amplification, or NASBA (see for example, Compton, 1991, Nature, 350: 91-92; Kievits et al., 1991, J. Virol Meth. 35: 273-286).

Another method of nucleic acid amplification that is of use according to the invention is the DNA ligase amplification reaction (LAR), which has been described as permitting the exponential increase of specific short sequences through the activities of any one of several bacterial DNA ligases (Wu and Wallace, 1989, Genomics, 4: 560; Barany, 1991, Proc. Natl. Acad. Sci. USA 88: 189). This technique is based upon the ligation of oligonucleotide probes. The probes are designed to exactly match two adjacent sequences of a specific target nucleic acid. The amplification reaction is repeated in three steps in the presence of excess probe: (1) heat denaturation of double-stranded nucleic acid, (2) annealing of probes to target nucleic acid, and (3) joining of the probes by thermostable DNA ligase. The reaction is generally repeated for 20-30 cycles. The sampling methods disclosed herein permit the generation of a detailed amplification profile. As with any cyclic amplification protocol, where desired, e.g., to establish an amplification profile, sampling can be performed after any cycle, but preferably after each cycle.

Rolling circle amplification (RCA) is an alternative amplification technology that may prove to have as large an impact as PCR. This technique draws on the DNA replication mechanism of some viruses. In RCA, similar to the replication technique used by many viruses, a polymerase enzyme reads off of a single promoter around a circle of DNA - continuously rolling out linear, concatenated copies of the circle. In such linear RCA, the reaction can run for three days, producing millions of copies of the small circle sequence. An exponential variant has been developed in which a second promoter displaces the double strands at each repeat and initiates hyperbranching in the DNA replication, creating as many as $10^{12}$ copies per hour.

Another amplification method that can benefit from the sampling methods disclosed herein is strand-displacement amplification (SDA; Walker et al., 1992, Nucleic Acids Res., 20: 1691-1696; Spargo et al., 1993, Mol. Cellular Probes 7: 395-404. SDA uses two types of primers and two enzymes (DNA polymerase and a restriction endonuclease) to exponentially produce single- stranded amplicons asynchronously. A variant of the basic method in which sets of the amplification primers were anchored to distinct zones on a chip reduces primer- primer interactions. This so-called "anchored SDA" approach permits multiplex DNA or RNA amplification without decreasing amplification efficiency (Westin et al., 2000, Nature Biotechnology 18: 199- 204) SDA can benefit from sampling and separation as described herein, as repeated sampling permits the generation of a detailed amplification profile.

Following reverse-transcription (where necessary or desired) and amplification, the methods described herein involve the separation of nucleic acid amplification products by size. Size separation of nucleic acids is well known, e.g., by agarose or polyacrylamide electrophoresis or by column chromatography, including HPLC separation. A preferred approach uses capillary electrophoresis, which is both rapid and accurate, readily achieving separation of molecules differing in size by as little as only one nucleotide. Capillary electrophoresis uses small amounts of sample and is well-adapted for detection by, for example, fluorescence detection. Capillary electrophoresis is well known in the art and is described in further detail herein below.

As discussed above, amplified nucleic acids corresponding to the pathogen specific target nucleic acid and competitor nucleic acids are detected after separation. The detection notes both the position of a given band of nucleic acid of a given size and the abundance of that nucleic acid by, for example, UV absorption or, preferably, fluorescent signal. Fluorescent nucleotides can be incorporated into the amplified nucleic acid by simply adding one or more such nucleotides to the amplification reaction mixture prior to or during amplification. An alternative approach is to fluorescently label one or more amplification primers such that every strand amplified from that primer has at least one fluorescent label associated with it. While the methods described here are fully intended to encompass the use of fluorescently labeled nucleotide analogs for labeling the amplified products, an advantage of labeling one or more amplification primers is that primers for different target nucleic acids can be differentially labeled with different fluorophores, to expand, for example, the scope of multiplexing possible with the methods described herein. With this approach, several sets of different pathogen specific target and competitor amplicons of even similar size can be distinguished in the same reaction.

Following detection of amplified, separated pathogen specific target and competitor molecules, the methods described herein use the amounts of the competitors detected as a standard. Because the original concentrations of the competitors is known, and the signal from the amplified sequences will be proportional to the starting amounts of each sequence, and the efficiency of amplification is similar for each of the target and the competitor molecules, the amount of the target nucleic acid in the original sample can be determined from the amount of the competitors. The accuracy of the method is further enhanced when, as is preferred, the competitors, as internal standards, were originally present at concentrations that flank the concentration of the target molecule.

It is noted that amplification approaches such as PCR generally exhibit kinetics such that there is a limited exponential phase of the amplification process in which the amount of amplified template is closely proportional to the amount of original template in the reaction. The exact location of this phase in a given cycling regimen will vary depending upon factors including the target sequence, primer sequences and the initial abundance of the target template. The methods described herein are well adapted to determining exactly when in the cycling regimen a given target sequence was (or is, when cycling and detection are performed simultaneously or at least contemporaneously) being amplified in the exponential phase. Thus, in one aspect, the methods described herein can benefit from repeated sampling during the amplification cycling regimen, coupled with separation and detection of the target and competitor nucleic acids in the withdrawn samples. The detection of, for example, fluorescently labeled target and competitor amplicons at multiple points or cycles during the amplification permits one to generate a plot (most often plotted automatically) of target, or of target and competitor amplicon abundance versus cycle number. This approach accurately identifies the phase for any given target or competitor at which the amplification is proceeding in exponential phase, which in turn permits the identification of the original quantity of the target template. The addition of internal standards represented, for example, by known concentrations of the longer and shorter competitors further enhances the accuracy of the data that can be obtained in this manner. That is, one not only has the internal standards that provide a curve from which to identify original concentration, but one also has the benefit of knowing at which point in the reaction the correspondence between initial template and amplified product is best. This point may differ for different amplicons in a single reaction. Again, the sampling approach and the profiles generated with it, permits the determination of such different points for each different amplicon in the reaction, permitting more accurate viral load determinations for each different virus targeted in a given assay.

Sample withdrawal during the amplification cycling regimen can be performed manually, or, preferably automatically, e.g., under robotic control. Automated sampling can enhance the uniformity of the timing of sample withdrawal, and can help to avoid cross-contamination that might occur under manual sampling conditions. Automated sampling and analysis apparatuses (including capillary electrophoresis apparatuses) are described in co-pending U.S. patent application No. 10/387,286, filed March 12, 2003).

The competitive quantitative approach described herein is well adapted for multiplexing - the determination of a plurality of different pathogen specific target nucleic acids in a given sample in a single reaction. This is preferably achieved by selecting target amplicon and competitor amplicon sizes such that different sets of target and competitor amplicons, distinguishable by amplicon size, are generated for each different target nucleic acid. Alternatively, or in addition, different target amplicons can be differentially detected in the same reaction by using differentially labeled amplification primers specific for different target/competitor amplicon sets. Basic multiplex PCR approaches and the considerations necessary to perform them successfully are known in the art and are readily applied to the methods described herein in which the ability to efficiently separate and detect amplicons of differing sizes from different known targets permits the detection ofmultiple (e.g., 2, 3, 5, 10, 20, 50 or more) target signals in a single reaction. Multiplex PCR generally requires that interactions between primers specific for different targets be minimized in order to reduce artifacts - that is, one seeks to avoid the ability of any two primers being used in a reaction to hybridize to each other, instead of to their respective target molecules. Commonly available software packages permit the analysis and prediction of primer-primer interactions for a given set of primers.

Primer design:

The methods described herein rely upon the use of DNA oligonucleotide primers for the amplification of pathogen specific target and competitor sequences. Oligonucleotide primers for use in these methods can be designed according to general guidance well known in the art as described herein, as well as with specific requirements as described herein for each step of the particular methods described.

1. *General Strategies for Primer Design*

Oligonucleotide primers are 5 to 100 nucleotides in length, preferably from 17 to 45 nucleotides, although primers of different length are of use. Primers for synthesizing cDNAs are preferably 10-45 nucleotides, while primers for amplification are preferably about 17-25 nucleotides. Primers useful in the methods described herein are also designed to have a particular melting temperature (Tm) by the method of melting temperature estimation. Commercial programs, including Oligo™, Primer Design, and programs available on the internet, including Primer3 and Oligo Calculator can be used to calculate a Tm of a polynucleotide sequence useful according to the invention. Preferably, the Tm of an amplification primer useful according to the invention, as calculated for example by Oligo Calculator, is preferably between about 45°C and 65°C and more preferably between about 50°C and 60°C.

Tm of a polynucleotide affects its hybridization to another polynucleotide (e.g., the annealing of an oligonucleotide primer to a template polynucleotide). In the subject methods, it is preferred that the oligonucleotide primer used in various steps selectively hybridizes to a target template or polynucleotides prepared or isolated from the target template (i.e., first and second strand cDNAs and amplified products). Typically, selective hybridization occurs when two polynucleotide sequences are substantially complementary (at least about 65% complementary over a stretch of at least 14 to 25 nucleotides, preferably at least about 75%, more preferably at least about 90% complementary). See Kanehisa, M., 1984, Polynucleotides Res. 12:203. As a result, it is expected that a certain degree of mismatch at the priming site is tolerated. Such mismatch may be small, such as a mono-, di- or tri-nucleotide. Alternatively, a region of mismatch may encompass loops, which are defined as regions in which there exists a mismatch in an uninterrupted series of four or more nucleotides. 100% complementarity is preferred for the methods described herein.

Numerous factors influence the efficiency and selectivity of hybridization of the primer to a second polynucleotide molecule. These factors, which include primer length, nucleotide sequence and/or composition, hybridization temperature, buffer composition and potential for steric hindrance in the region to which the primer is required to hybridize, are considered when designing oligonucleotide primers useful in the methods described herein.

A positive correlation exists between primer length and both the efficiency and accuracy with which a primer will anneal to a target sequence. In particular, longer sequences have a higher melting temperature ($T_M$) than do shorter ones, and are less likely to be repeated within a given target sequence, thereby minimizing promiscuous hybridization. Primer sequences with a high G-C content or that comprise palindromic sequences tend to self-hybridize, as do their intended target sites, since unimolecular, rather than bimolecular, hybridization kinetics are generally favored in solution. However, it is also important to design a primer that contains sufficient numbers of G-C nucleotide pairings since each G-C pair is bound by three hydrogen bonds, rather than the two that are found when A and T bases pair to bind the target sequence, and therefore forms a tighter, stronger bond. Hybridization temperature varies inversely with primer annealing efficiency, as does the concentration of organic solvents, e.g. formamide, that might be included in a priming reaction or hybridization mixture, while increases in salt concentration facilitate binding. Under stringent annealing conditions, longer hybridization probes, or synthesis primers, hybridize more efficiently than do shorter ones, which are sufficient under more permissive conditions. Preferably, stringent hybridization is performed in a suitable buffer (for example, 1X RT buffer, Stratagene Catalog # 600085, 1X Pfu buffer, Stratagene Catalog #200536; or 1X cloned Pfu buffer, Stratagene Catalog #200532, or other buffer suitable for other enzymes used for cDNA synthesis and amplification) under conditions that allow the polynucleotide sequence to hybridize to the oligonucleotide primers (e.g., 95°C for PCR amplification). Stringent hybridization conditions can vary (for example from salt concentrations of less than about 1M, more usually less than about 500 mM and preferably less than about 200 mM) and hybridization temperatures can range (for example, from as low as 0°C to greater than 22°C, greater than about 30°C, and (most often) in excess of about 37°C) depending upon the lengths and/or the polynucleotide composition or the oligonucleotide primers. Longer fragments may require higher hybridization temperatures for specific hybridization. As several factors affect the stringency of hybridization, the combination of parameters is more important than the absolute measure of a single factor.

The design of a primer set useful in the methods described herein can be facilitated by the use of readily available computer programs, developed to assist in the evaluation of the several parameters described above and the optimization of primer sequences. Examples of such programs are "PrimerSelect" of the DNAStar™ software package (DNAStar, Inc.; Madison, WI), OLIGO 4.0 (National Biosciences, Inc.), PRIMER, Oligonucleotide Selection Program, PGEN and Amplify (described in Ausubel et al., supra).

*2. Oligonucleotide Synthesis*

The oligonucleotide primers themselves are synthesized using techniques that are also well known in the art. Methods for preparing oligonucleotides of specific sequence include, for example, cloning and restriction digestion of appropriate sequences and direct chemical synthesis. Once designed, oligonucleotides can also be prepared by a suitable chemical synthesis method, including, for example, the phosphotriester method described by Narang et al., 1979, Methods in Enzymology, 68 : 90, the phosphodiester method disclosed by Brown et al., 1979, Methods in Enzymology, 68 : 109, the diethylphosphoramidate method disclosed in Beaucage et al., 1981, Tetrahedron Letters, 22 : 1859, and the solid support method disclosed in U.S. Patent No. 4,458,066, or by other chemical methods using either a commercial automated oligonucleotide synthesizer (which is commercially available) or VLSIPS™ technology.

Competitor RNA design and synthesis:

When employed in methods as described herein, competitor nucleic acids should be amplified by the same primer set selected for a given pathogen specific target nucleic acid and have similar amplification efficiency to the target nucleic acid with the same selected set of primers. The competitor nucleic acids should yield amplification products, with the selected set of primers, that are distinguishable in length from each other and from the amplification product from the target nucleic acid. The resolution of separation techniques will necessarily bear upon the differences in length that are distinguishable. As noted above, differences of as little as one nucleotide are routinely achievable, although even in these instances, it may be useful to have somewhat longer lengths, in order to provide better distinction in signal. A key consideration is having the length difference long enough to be detectable by the selected method, e.g., capillary electrophoresis, but short enough that it does not significantly modify the amplification efficiency relative to that of the target nucleic acid. That is, the amplification efficiency of the longer or shorter competitor nucleic acid must be similar to that of the target nucleic acid.

As discussed above, competitor nucleic acids are characterized by the presence of sequences which permit their amplification by the same pair of oligonucleotide primers selected to amplify a given pathogen specific target nucleic acid. Amplification of the competitor nucleic acid by the same pair of primers as used to amplify the pathogen specific target nucleic acid assures that the annealing efficiency of the primers to both the target and competitor sequences is the same, which is important for assuring similar amplification efficiency of the competitor and target nucleic acids.

To maintain similar amplification efficiency, it is important that competitor nucleic acids (or, more accurately, their amplification products) have similar $T_m$ to the target nucleic acid (or its amplification products). Methods for the estimation of $T_m$ for any given sequence are well known in the art. $T_m$ is similar if, for example, it is within 1-2°C, but preferably within 0.5 to 1°C or even less difference, relative to the target nucleic acid. It is preferred that competitor and target nucleic acids comprise at least 20 nucleotides or base pairs of identical sequence. This is preferably in addition to common primer binding sequences. The primer-binding sequences of the target and competitor nucleic acids do not need to be identical, but should operate to permit amplification by the same primers. Because differences in primer annealing efficiency affect amplification efficiency, it is most straight-forward to maintain identity in these sequences between the pathogen specific target and competitor sequences.

One of the most straightforward ways of generating competitor nucleic acids that will have the necessarily similar amplification efficiency to the pathogen specific target nucleic acid is to modify a cloned cDNA corresponding to the pathogen specific target nucleic acid, by inserting or deleting a short (e.g., a 1-20 nucleotide insertion or deletion e.g., a 5-20 nucleotide or 5-10 nucleotide insertion or deletion) stretch in the pathogen specific target sequence itself (i.e., an internal insertion or deletion). This assures similar characteristics for annealing and amplification efficiency, with the only differences being the internal insertion or deletion. While insertion or deletion of a short contiguous sequence, is more easily accomplished, the insertion or deletion encompassed by this embodiment can also include insertion or deletion on non-contiguous nucleotides or base pairs-that is, removal or insertion at more than one location within the pathogen specific target sequence. For shorter target amplicon sequences, e.g., 50 to 75 nucleotides, it is beneficial to keep the difference in length to the shorter end of this spectrum, e.g., 1 to 5 nucleotides, as this represents a smaller change in make-up of the sequence on a percentage basis. For longer target amplicon sequences, the length difference can be longer without having as dramatic an impact on the amplification characteristics of the molecule. Even in the context of longer target amplicon sequences, the insertion or deletion is still preferably 10 nucleotides (or base pairs) or fewer, particularly where the size separation will be performed with a method, e.g., CE, which is capable of resolution on the basis of as little as 1 nucleotide or base pair.

One of skill in the art will understand that one factor affecting amplification efficiency is the presence of repeat stretches of the same nucleotide, e.g., poly A, poly G, etc., which tend to reduce the efficiency of amplification relative to a similar sequence without the repeats. Thus, when considering the sequence to add, or, for that matter, to delete, it is best to add or delete sequence that is approximately balanced in nucleotide composition. The sequence added or deleted can be amino acid coding or non-coding sequence, and can optionally comprise conventional or non-conventional nucleotides, if so desired.

The insertion or deletion of sequence useful in generating a set of competitor nucleic acids is readily achieved using site-directed mutagenesis techniques well known in the art. A number of methods are known in the art that permit the targeted mutation of DNA sequences (see for example, Ausubel et. al. Short Protocols in Molecular Biology (1995) 3rd Ed. John Wiley & Sons, Inc.). In addition, there are a number of commercially available kits for site-directed mutagenesis, including both conventional and PCR-based methods. Examples include the GeneMorph Random mutagenesis kit (Stratagene Catalog No. 600550 or 200550), EXSITE™ PCR-Based Site-directed Mutagenesis Kit available from Stratagene (Catalog No. 200502) and the QUIKCHANGE™ Site-directed mutagenesis Kit from Stratagene (Catalog No. 200518), and the CHAMELEON® double-stranded Site-directed mutagenesis kit, also from Stratagene (Catalog No. 200509).

The measurement of amplification efficiency is described herein below.

Once competitor sequences are designed, the competitor nucleic acid for use in the methods described herein can be generated by, for example, chemical synthesis as known in the art, PCR, or, when the competitor nucleic acid is an RNA, by *in vitro* transcription. The technique of *in vitro* transcription is well known to those of skill in the art. Briefly, the sequence of interest is linked to a promoter sequence for a prokaryotic polymerase, such as the bacteriophage T7, T3 and Sp6 RNA polymerase promoter, followed by in vitro transcription of the DNA template using the appropriate polymerase. The template can itself be a linear PCR product into which the promoter has been incorporated, for example, by inclusion of the appropriate promoter sequence in one of the PCR amplification primers. Where desired, linkage to two different promoters, one on each end, creates the potential for also generating the complement of the competitor RNA.

Alternatively, a DNA sequence corresponding to a desired competitor RNA can be inserted into a vector containing an Sp6, T3 or T7 promoter. The vector is linearized with an appropriate restriction enzyme that digests the vector at a single site located downstream of the competitor sequence. Following a phenol/chloroform extraction, the DNA is ethanol precipitated, washed in 70% ethanol, dried and resuspended in sterile water. Regardless of the exact form of the promoter/template construct (i.e., linear PCR product or linearized vector construct), the *in vitro* transcription reaction is performed by incubating the linear DNA with transcription buffer (200 mM Tris-HCl, pH 8.0, 40 mM MgCl$_2$, 10 mM spermidine, 250 NaCl [T7 or T3] or 200 mM Tris-HCl, pH 7.5, 30 mM MgCl$_2$, 10 mM spermidine [Sp6]), dithiothreitol, RNase inhibitors, each of the four ribonucleoside triphosphates, and either Sp6, T7 or T3 RNA polymerase, e.g., for 30 min at 37°C. If it is desired to prepare a labeled polynucleotide comprising RNA, unlabeled UTP can be omitted and labeled UTP can be included in the reaction mixture. Labels can include, for example, fluorescent or radiolabels. The DNA template is then removed by incubation with DNaseI. Phenol extraction can be used to remove the DNAse and polymerase, followed by precipitation and quantitation of the RNA, e.g., by UV absorption and/or by electrophoresis and visualization relative to known standards.

Polymerase Chain Reaction:

PCR provides a well-established method for rapidly amplifying a particular DNA sequence by using multiple cycles of DNA replication catalyzed by a thermostable, DNA-dependent DNA polymerase to amplify the target sequence of interest. PCR requires the presence of a target nucleic acid sequence to be amplified, two single stranded oligonucleotide primers flanking the sequence to be amplified, a DNA polymerase, deoxyribonucleoside triphosphates, a buffer, and salts.

PCR is described in Mullis and Faloona, 1987, Methods Enzymol., 155: 335, as well as in U.S. Pat. Nos. 4,683,202, 4,683,195 and 4,800,159. Reaction conditions for the specific amplification of a target sequence can be readily selected or determined with a minimum of experimentation by one of ordinary skill in the art. Numerous variations on the basic theme are also known to those of skill in the art.

The length and temperature of each step of a PCR cycle (denaturation, primer annealing, and extension), as well as the number of cycles, are adjusted according to the stringency requirements in effect. Annealing temperature and timing are determined both by the efficiency with which a primer is expected to anneal to a template and the degree of mismatch that is to be tolerated. The ability to optimize the stringency of primer annealing conditions is well within the knowledge of one of ordinary skill in the art. An annealing temperature of between 30°C and 72°C is most often used. Initial denaturation of the template molecules normally occurs at between 92°C and 99°C, e.g., for 4 minutes, followed by 10-40 cycles consisting of denaturation (94°C -99°C for 15 seconds to 1 minute), annealing (temperature determined as discussed above; 30 seconds to 2 minutes), and extension (72°C for 30 seconds to 1 minute; this is optimal for Taq polymerase - one of skill in the art will know or can easily determine suitable extension conditions for different thermostable polymerases). Depending upon the intended use of the product, a final extension step is often carried out for a longer time, e.g., 4 minutes at 72°C, and may be followed by an indefinite (0-24 hour) storage at 4°C.

Polymerases:

A wide variety of DNA polymerases can be used in the methods described herein. Suitable DNA polymerases for use in the subject methods may or may not be thermostable, although thermostable polymerases are obviously preferred for the embodiments using thermocycling for amplification. Known conventional DNA polymerases include, for example, *Pyrococcus furiosus* (Pfu) DNA polymerase (Lundberg et al., 1991, Gene, 108:1, provided by Stratagene), *Pyrococcus woesei* (Pwo) DNA polymerase (Hinnisdaels et al., 1996, Biotechniques, 20 :186-8, provided by Boehringer Mannheim), *Thermus thermophilus* (Tth) DNA polymerase (Myers and Gelfand 1991, Biochemistry 30 :7661), *Bacillus stearothermophilus* DNA polymerase (Stenesh and McGowan, 1977, Biochim Biophys Acta 475 : 32), *Thermococcus litoralis* (Tli) DNA polymerase (also referred to as Vent DNA polymerase, Cariello et al., 1991, Polynucleotides Res, 19 : 4193, provided by New England Biolabs), Vent exó (New England Biolabs), 9°Nm DNA polymerase (discontinued product from New England Biolabs), *Thermotoga maritima* (Tma) DNA polymerase (Diaz and Sabino, 1998, Braz J. Med. Res, 31 : 1239), *Thermus aquaticus* (Taq) DNA polymerase (Chien et al., 1976, J. Bacteriol, 127 : 1550), *Pyrococcus kodakaraensis* KOD DNA polymerase (Takagi et al., 1997, Appl. Environ. Microbiol. 63 : 4504), JDF-3 DNA polymerase (from *thermococcus sp.* JDF-3, Patent application WO 0132887), *Pyrococcus GB-D* (PGB-D) DNA polymerase (also referred as Deep-Vent DNA polymerase, Juncosa-Ginesta et art, 1994, Biotechniques, 16 : 820, provided by New England Biolabs), UlTma DNA polymerase (from *thermophile Thermotoga maritima*; Diaz and Sabino, 1998, Braz J. Med. Res. 31:1239; provided by PE Applied Biosystems), Tgo DNA polymerase (from *thermococcus gorgonarius*, provided by Roche Molecular Biochemicals), *E. coli* DNA polymerase I (Lecomte and Doubleday, 1983, Polynucleotides Res. 11 : 7505), T7 DNA polymerase (Nordstrom et al., 1981, J. Biol. Chem. 256 : 3112), and archaeal DP1/DP2 DNA polymerase II (Cann et al., 1998, Proc. Natl. Acad. Sci. USA 95 : 14250-5).

For thermocyclic reactions, the polymerases are preferably thermostable polymerases such as Taq, Deep Vent, Tth, Pfu, Vent, and UlTma, each of which are readily available from commercial sources. Similarly, guidance for the use of each of these enzymes can be readily found in any of a number of protocols found in guides, product literature, the Internet (see, for example, www.alkami.com), and other sources.

For non-thermocyclic reactions, and in certain thermocyclic reactions, the polymerase will often be one of many polymerases commonly used in the field, and commercially available, such as DNA pol I, Klenow fragment, T7 DNA polymerase, and T4 DNA polymerase. In applications involving transcription, a number of RNA polymerases are also commercially available, such as T7 RNA polymerase and SP6 RNA polymerase. Guidance for the use of such polymerases can readily be found in product literature and in general molecular biology guides such as Sambrook or Ausubel, both supra.

Polymerases can incorporate labeled (e.g., fluorescent) nucleotides or their analogs during synthesis of polynucleotides. See, e.g., Hawkins et al., U.S. Patent No. 5,525,711, where the use of nucleotide analogs which are incorporated by Taq is described.

As described above, the amplification reactions required for the methods described herein can generally be carried out using standard reaction conditions and reagents unless otherwise specified. Such reagents and conditions are well known to those of skill in the art, and are described in numerous references and protocols. See, e.g. Innis supra; Sambrook, supra.; Ausubel, et al., eds. (1996) Current Protocols in Molecular Biology Current Protocols, a joint venture between Greene Publishing Associates, Inc. and John Wiley & Sons, Inc. Also, see, Mullis et al., (1987) U.S. Patent No. 4,683,202, and Arnheim & Levinson (1990) C&EN 6-47, The Journal Of NIH Research (1991) 3 : 81-94; Kwoh et al. (1989) Proc. Natl. Acad. Sci. USA 86 : 1173; Guatelli et al. (1990) Proc. Natl. Acad. Sci. USA 87 : 1874; Lomell et al. (1989) J. Clin. Chem 35 : 1826; Landegren et al., (1988) Science 241 : 1077-1080; Van Brunt (1990) Biotechnology 8 : 291-294; Wu and Wallace, (1989) Gene 4 : 560; Barringer et al. (1990) Gene 89 : 117, and Sooknanan and Malek (1995) Biotechnology 13 : 563-564.

Amplification efficiency:

As discussed above, the amplification efficiency of competitor nucleic acid when used, should be similar to that of the pathogen specific target nucleic acid. In one aspect, amplification efficiency is expressed as the fold amplification per PCR cycle, represented as a fraction or percentage relative to perfect doubling. A 100% or 1.0 amplification efficiency would refer to perfect doubling.

One way to monitor amplification efficiency is to measure the threshold cycle number (Ct) at which signal intensity of PCR product reaches a set threshold value (for example 10 standard deviations of background value of signal intensity) for an amplified product. Samples are withdrawn at, for example, each cycle during the amplification regimen and analyzed for the amount of target amplicon. Comparison of Ct for equal starting amounts of two different amplification templates, e.g., a target RNA and a competitor RNA will determine whether the amplification efficiency is similar. To enhance accuracy, the determination can be performed at several different equal starting concentrations of target and competitor RNAs. Amplification efficiency is considered "similar" if the threshold cycle, Ct, is the same for equal starting amounts of each competitor/target set.

Ct is linked to the initial copy number or concentration of starting DNA by a simple mathematical equation:

$$\mathrm{Log(copy\ number)} = aC_t + b \text{ , where a and b are constants.}$$

Therefore, by measuring $C_t$ for the fragments of the same gene originating from two different samples, the original concentration of this gene in these samples can be easily evaluated. Alternatively, amplification efficiency is monitored by measuring the amount of amplification product (e.g., by fluorescence intensity or label incorporation) at successive cycles, calculating efficiency using the formula $E=(P_{n+1}-P_n)/(P_n-P_{n-1})$, where P is the amount of amplification product at cycle n.

While the similarity in amplification efficiencies will ultimately be determined empirically, the maintenance of target sequence identity in the competitors, except for an insertion or deletion necessary to generate a detectable difference in length relative to the target, will assist in achieving similar efficiencies.

It is known that the presence of various contaminants in a nucleic acid sample preparation can have an effect on amplification efficiency. An advantage of the methods described herein is that any such contaminant will most likely affect the efficiency of amplification of both the competitor and target amplicons for any given pathogen-specific target to a similar degree, because each of these amplicons is generated in the same reaction. This will tend to reduce the impact of any such inhibition of efficient amplification.

Preparation of samples

A pathogen specific target polynucleotide of the present invention may be single- or double- stranded, and it may be DNA (e.g., gDNA or cDNA), RNA, a polynucleotide comprising both deoxyribo- and ribonucleotides, or a polynucleotide comprising deoxyribonucleotides, ribonucleotides, and/or analogs and derivatives thereof. Where one wishes to determine the level of expression of a viral gene, the target polynucleotide is an RNA molecule, e.g., an mRNA molecule.

Before the amplification reaction, the pathogen specific target polynucleotide may be obtained in suitable quantity and quality for the amplification method to be used. For example, in some instances, the samples contain such a low level of target polynucleotide that it is useful to conduct a pre-amplification reaction to increase the concentration of the target polynucleotide. If samples are to be amplified, amplification is typically conducted using the polymerase chain reaction (PCR) according to known procedures. In some embodiments, it may be preferred to add known quantities of competitor nucleic acids to a biological sample prior to coisolation of competitor and test nucleic acids in the sample.

Guidance for the preparation of a sample containing a target polynucleotide can be found in a multitude of sources, including PCR Protocols, A Guide to Methods and Applications (Innis et al., supra; Sambrook et al., supra; Ausubel et al., supra). Any such method can be used in methods described herein. Typically, these methods involve cell lysis, followed by purification of polynucleotides by methods such as phenol/chloroform extraction, electrophoresis, and/or chromatography. Often, such methods include a step wherein the polynucleotides are precipitated, e.g. with ethanol, and resuspended in an appropriate buffer for addition to a PCR or similar reaction.

In certain embodiments, two or more pathogen specifc target polynucleotides from one or more sample sources are analyzed in a single reaction. In these embodiments, a plurality of pathogen specifc target polynucleotides may be amplified from a single sample or individual, thereby allowing the assessment of a variety of pathogens potentially present in a sample from a single individual, e.g., to simultaneously screen for a multitude of pathogens in an individual who is immunosuppressed. Any of the above applications can be easily accomplished using the methods described herein.

A reaction mixture may comprise one pathogen specifc target polynucleotides, or it may comprise two or more pathogen specifc target polynucleotides, up to, for example, 15 or 16 pathogen specifc target polynucleotides. The present method thus allows for simultaneous analysis of two or more polynucleotides in a single sample, i.e., multiplex analysis.

Once the starting cells, tissues, organs or other samples are obtained, nucleic acids (including RNA and/or DNA) can be prepared from them by methods that are well-known in the art. Samples from immunocompromised individuals, e.g., transplant or graft recipients maintained on an immunosuppressant regimen, will most often be blood or serum samples. Methods of nucleic acid isolation from blood samples are well known to those of skill in the art.

RNA can be purified, for example, from tissues according to the following method. Following removal of the tissue of interest, pieces of tissue of ≤2g are cut and quick frozen in liquid nitrogen, to prevent degradation of RNA. Upon the addition of a suitable volume of guanidinium solution (for example 20 ml guanidinium solution per 2 g of tissue), tissue samples are ground in a tissuemizer with two or three 10-second bursts. To prepare tissue guanidinium solution (1 L) 590.8 g guanidinium isothiocyanate is dissolved in approximately 400 ml DEPC-treated $H_2O$. 25 ml of 2 M Tris-HCl, pH 7.5 (0.05 M final) and 20 ml $Na_2$EDTA (0.01 M final) is added, the solution is stirred overnight, the volume is adjusted to 950 ml, and 50 ml 2-ME is added.

Homogenized tissue samples are subjected to centrifugation for 10 min at 12,000 x g at 120C. The resulting supernatant is incubated for 2 min at 65°C in the presence of 0.1 volume of 20% Sarkosyl, layered over 9 ml of a 5.7M CsCl solution (0.1 g CsCl/ml), and separated by centrifugation overnight at 113,000 x g at 22°C. After careful removal of the supernatant, the tube is inverted and drained. The bottom of the tube (containing the RNA pellet) is placed in a 50 ml plastic tube and incubated overnight (or longer) at 4°C in the presence of 3 ml tissue resuspension buffer (5 mM EDTA, 0.5% (v/v) Sarkosyl, 5% (v/v) 2-ME) to allow complete resuspension of the RNA pellet. The resulting RNA solution is extracted sequentially with 25:24:1 phenol/chloroform/isoamyl alcohol, followed by 24:1 chloroform/isoamyl alcohol, precipitated by the addition of 3 M sodium acetate, pH 5.2, and 2.5 volumes of 100% ethanol, and resuspended in DEPC water (Chirgwin et al., 1979, Biochemistry, 18 : 5294).

Alternatively, RNA can be isolated from tissues according to the following single step protocol. The tissue of interest is prepared by homogenization in a glass teflon homogenizer in 1 ml denaturing solution (4M guanidinium thiosulfate, 25 mM sodium citrate, pH 7.0, 0.1M 2-ME, 0.5% (w/v) N-laurylsarkosine) per 100mg tissue. Following transfer of the homogenate to a 5-ml polypropylene tube, 0.1 ml of 2 M sodium acetate, pH 4, 1 ml water-saturated phenol, and 0.2 ml of 49:1 chloroform/isoamyl alcohol are added sequentially. The sample is mixed after the addition of each component, and incubated for 15 min at 0-4 °C after all components have been added. The sample is separated by centrifugation for 20 min at 10,000 x g, 4 °C, precipitated by the addition of 1 ml of 100% isopropanol, incubated for 30 minutes at -20 °C and pelleted by centrifugation for 10 minutes at 10,000 x g, 4 °C. The resulting RNA pellet is dissolved in 0.3 ml denaturing solution, transferred to a microfuge tube, precipitated by the addition of 0.3 ml of 100% isopropanol for 30 minutes at -20 °C, and centrifuged for 10 minutes at 10,000 x g at 40C. The RNA pellet is washed in 70% ethanol, dried, and resuspended in 100-200 μl DEPC-treated water or DEPC-treated 0.5% SDS (Chomczynski and Sacchi, 1987, Anal. Biochem., 162:156).

Kits and reagents for isolating total RNAs are commercially available from various companies, for example, RNA isolation kit (Stratagene, La Lola, CA, Cat # 200345); PicoPure™ RNA Isolation Kit (Arcturus, Mountain View, CA, Cat # KIT0202); RNeasy Protect Mini, Midi, and Maxi Kits (Qiagen, Cat # 74124).

In some embodiments, total RNAs are used in the subject method for subsequent analysis, e.g., for reverse transcription. In other embodiments, mRNAs can be isolated from the total RNAs or directly from the samples to use for reverse transcription. Kits and reagents for isolating mRNAs are commercially available from, e.g., Oligotex mRNA Kits (Qiagen, Cat # 70022).

Labeled Nucleotides

The methods described herein can benefit from the use of labels including, e.g., fluorescent labels. In one aspect, the fluorescent label can be a label or dye that intercalates into or otherwise associates with amplified (usually double-stranded) nucleic acid molecules to give a signal. One stain useful in such embodiments is SYBR Green (e.g., SYBR Green I or II, commercially available from Molecular Probes Inc., Eugene, OR). Others known to those of skill in the art can also be employed in the methods described herein. An advantage of this approach is reduced cost relative to the use of, for example, labeled nucleotides. Nonetheless, it may also be preferred that the label will be incorporated by attachment to a labeled nucleotide or nucleotide analog that is a substrate for the polymerizing enzyme. Label can alternatively be attached to an amplification primer. As taught above, a labeled nucleotide can be a fluorescent dye-linked nucleotide, or it can be an intrinsically fluorescent nucleotide. In one embodiment of the methods described herein, a conventional deoxynucleotide linked to a fluorescent dye is used. Non-limiting examples of some useful labeled nucleotide are listed in Table 1.

**Table 1.** Examples of labeled nucleotides

| Fluorescein Labeled | Fluorophore Labeled |
|---|---|
| Fluorescein - 12 - dCTP | Eosin - 6 - dCTP |
| Fluorescein - 12 - dUTP | Coumarin - 5 -ddUTP |
| Fluorescein - 12 - dATP | Tetramethylrhodamine - 6 - dUTP |
| Fluorescein - 12 - dGTP | Texas Red - 5 - dATP |
| Fluorescein - N6 - dATP | LISSAMINE™ - rhodamine - 5 - dGTP |
| **FAM Labeled** | **TAMRA Labeled** |
| FAM - dUTP | TAMRA - dUTP |
| FAM - dCTP | TAMRA - dCTP |
| FAM - dATP | TAMRA - dATP |
| FAM - dGTP | TAMRA - dGTP |
| **ROX Labeled** | **JOE Labeled** |
| ROX - dUTP | JOE - dUTP |
| ROX - dCTP | JOE - dCTP |
| ROX - dATP | JOE - dATP |
| ROX - dGTP | JOE - dGTP |
| **R6G Labeled** | **R110 Labeled** |
| R6G - dUTP | R110 - dUTP |
| R6G - dCTP | R110 - dCTP |
| R6G - dATP | R110 - dATP |
| R6G - dGTP | R110 - dGTP |

(continued)

| BIOTIN Labeled | DNP Labeled |
|---|---|
| Biotin - N6 - dATP | DNP - N6 - dATP |

Fluorescent dye-labeled nucleotide can be purchased from commercial sources. Labeled polynucleotides nucleotide can also be prepared by any of a number of approaches known in the art.

Fluorescent dyes useful as detectable labels are well known to those skilled in the art and numerous examples can be found in the Handbook of Fluoresdent Probes and Research Chemicals 6th Edition, Richard Haugland, Molecular Probes, Inc., 1996 (ISBN 0-9652240-0-7).

Preferably, fluorescent dyes are selected for compatibility with detection on an automated capillary electrophoresis apparatus and thus should be spectrally resolvable and not significantly interfere with electrophoretic analysis. Examples of suitable fluorescent dyes for use as detectable labels can be found in among other places, U.S. Patent Nos. 5,750,409; 5,366,860; 5,231,191; 5,840,999; 5,847,162; 4,439,356; 4,481,136; 5,188,934; 5,654,442; 5,840,999; 5,750,409; 5,066,580; 5,750,409; 5,366,860; 5,231,191; 5,840,999; 5,847,162; 5,486,616; 5,569,587; 5,569,766; 5,627; 027; 5,321,130; 5,410,030; 5,436,134; 5,534,416; 5,582,977; 5,658,751; 5,656,449; 5,863,753; PCT Publications WO 97/36960; 99/27020; 99/16832; European Patent EP 0 050 684; Sauer et al, 1995, J. Fluorescence 5 : 247-261; Lee et al., 1992, Nucl. Acids Res. 20 : 2471-2483; and Tu et al.., 1998, Nucl. Acids Res. 26 : 2797-2802.

Nucleotide can be modified to include functional groups, such as primary and secondary amines, hydroxyl, nitro and carbonyl groups, for fluorescent dye linkage (see Table 2).

**Table 2**

| Functional Group | Reaction | Product |
|---|---|---|
| Amine | dye - isothiocyanates | Thiourea |
| Amine | dye - succinimidyl ester | Carboxamide |
| Amine | dye - sulfonyl chloride | Sulphonamide |
| Amine | dye - aldehyde | Alkylamine |
| Ketone | dye - hydrazides | Hydrazones |
| Ketone | dye - semicarbazides | Hydrazones |
| Ketone | dye - carbohydrazides | Hydrazones |
| Ketone | dye - amines | Alkylamine |
| Aldehyde | dye - hydrazides | Hydrazones |
| Aldehyde | dye - semicarbazides | Hydrazones |
| Aldehyde | dye - carbohydrazides | Hydrazones |
| Aldehyde | dye - amines | Alkylamine |
| Dehydrobutyrine | dye - sulphydryl | Methyl lanthionine |
| Dehydroalanine | dye - sulphydryl | Lanthionine |

Useful fluorophores include, but are not limited to: Texas Red™ (TR), Lissamine™ rhodamine B, Oregon Green™ 488 (2',7' - difluorofluorescein), carboxyrhodol and carboxyrhodamine, Oregon Green™ 500, 6 - JOE (6 - carboxy - 4',5' - dichloro - 2',7' - dimethyoxyfluorescein, eosin F3S (6 - carobxymethylthio - 2',4',5',7' - tetrabromo - trifluorofluorescein), Cascade Blue™ (CB), aminomethylcoumarin (AMC), pyrenes, dansyl chloride (5 - dimethylaminonaphthalene - 1 - sulfonyl chloride) and other napththalenes, PyMPO, ITC (1 - (3 - isothiocyanatophenyl) - 4 - (5 - (4 - methoxyphenyl)oxazol - 2 - yl)pyridinium bromide), coumarin, fluorescein, tetrachlorofluorescein, hexachlorofluorescein, Lucifer yellow, rhodamine, BODIPY, tetramethylrhodamine, Cy3, Cy5, Cy7, eosine, and ROX. Combination fluorophores such as fluoresceinrhodamine dimers, described, for example, by Lee et al. (1997), Polynucleotides Research 25:2816, are also suitable. Suitable Fluorophores include those that absorb and emit in the visible spectrum or outside the visible spectrum, such as in the ultraviolet or infrared ranges. Suitable fluorescent dye labels are commercially available from Molecular Probes, Inc., Eugene, OR, US and Research Organics, Inc., Cleveland, OH, US, among other sources, and can be found in the Handbook of Fluorescent Probes and Research Chemicals 6th Edition, Richard Haugland, Molecular Probes, Inc., 1996 (ISBN 0-9652240-0-7).

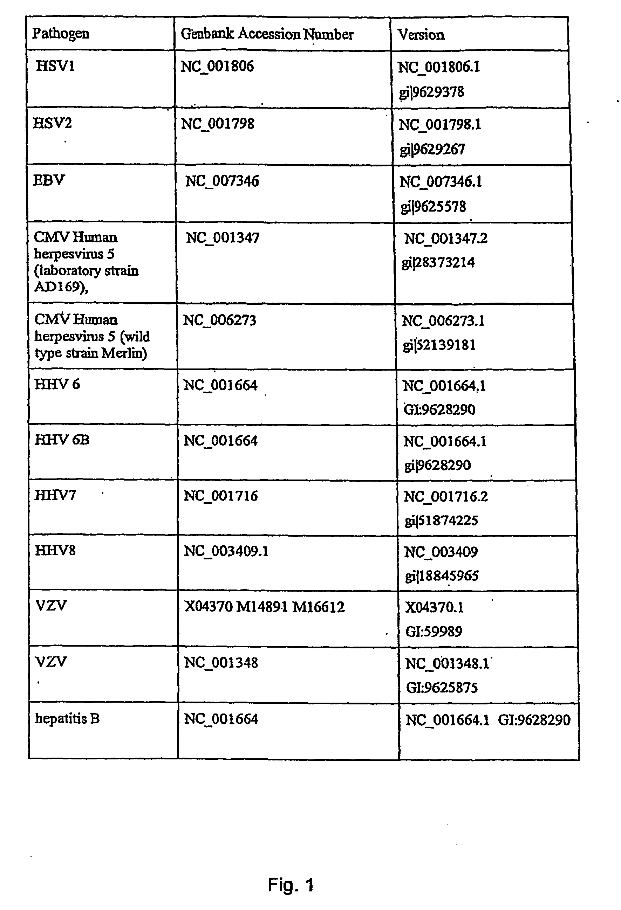

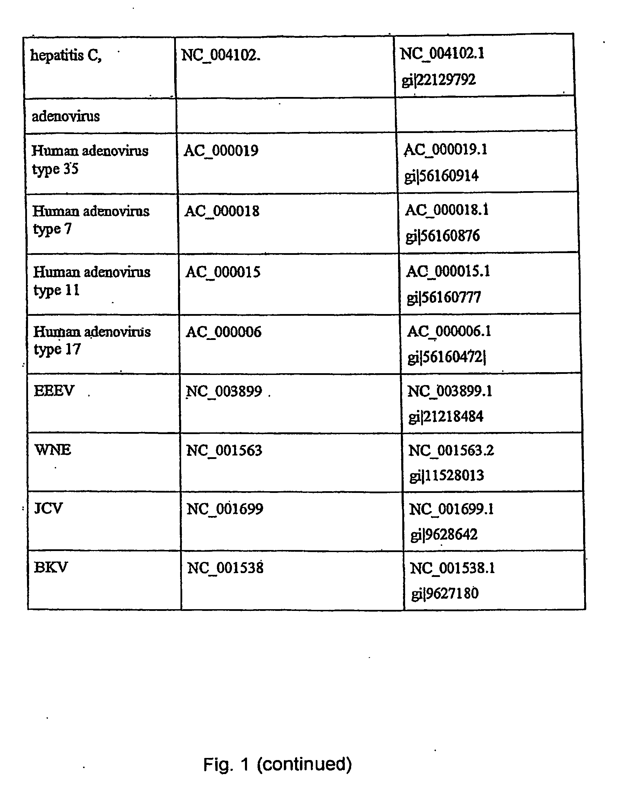

A labeled nucleotide useful in the methods described herein includes an intrinsically fluorescent nucleotide known in the art, e.g., the novel fluorescent nucleoside analogs as described in U.S. Patent No. 6,268,132. The fluorescent analogs of the U.S. Patent No. 6,268,132 are of three general types: (A) C-nucleoside analogs; (B) N-nucleoside analogs; and (C) N-azanucleotide and N-deazanucleotide analogs. All of these compounds have three features in common: 1) they are structural analogs of the common nucleosides capable of replacing naturally occurring nucleosides in enzymatic or chemical synthesis of oligonucleotides; 2) they are naturally fluorescent when excited by light of the appropriate wavelength(s) and do not require additional chemical or enzymatic processes for their detection; and 3) they are spectrally distinct from the nucleosides commonly encountered in naturally occurring DNA. At least 125 specific compounds have been identified in U.S. Patent No. 6,268,132. These compounds, which have been characterized according to their class, structure, chemical name, absorbance spectra, emission spectra, and method of synthesis, are tabulated as shown in FIGS. 21A-21F-1 of the U.S. Patent No. 6,268,132.

The labeled nucleotide as described herein also includes, but is not limited to, fluorescent N-nucleosides and fluorescent structural analogs. Formycin A (generally referred to as Formycin), the prototypical fluorescent nucleoside analog, was originally isolated as an antitumor antibiotic from the culture filtrates of Nocardia interforma (Hori et al. [1966] J. Antibiotics, Ser. A 17:96-99) and its structure identified as 7-amino-3-b-D-ribafuranosyl (1H-pyrazolo-[4,3d] pyrimidine)). This antibiotic, which has also been isolated from culture broths of Streptomyces lavendulae (Aizawa et al. [1965] Agr. Biol. Chem. 29:375-376), and Streptomyces gummaensis (Japanese Patent No. 10,928, issued in 1967 to Nippon Kayaku Co., Ltd.), is one of numerous microbial C-ribonucleoside analogs of the N-nucleosides commonly found in RNA from all sources. The other naturally-occurring C-ribonucleosides which have been isolated from microorganisms include formycin B (Koyama et al. [1966] Tetrahedron Lett. 597-602; Aizawa et al., supra; Umezawa et al. [1965] Antibiotics Ser. A 18:178-181), oxoformycin B (Ishizuka et al. [1968] J. Antibiotics 21:1-4; Sawa et al. [1968] Antibiotics 21:334-339), pseudouridine (Uematsu and Suahdolnik [1972] Biochemistry 11:4669-4674), showdomycin (Darnall et al. [1967] PNAS 57:548-553), pyrazomycin (Sweeny et al. [1973] Cancer Res. 33:2619-2623), and minimycin (Kusakabe et al. [1972] J. Antibiotics 25:44-47). Formycin, formycin B, and oxoformycin B are pyrazolopyrimidinenucleosides and are structural analogs of adenosine, inosine, and hypoxanthine, respectively; a pyrazopyrimidine structural analog of guanosine obtained from natural sources has not been reported in the literature. A thorough review of the biosynthesis of these compounds is available in Ochi et al. (1974) J. Antibiotics xxiv:909-916.

Separation and Detection of Amplified Products:

Methods for detecting the presence or amount of polynucleotides are well known in the art and any of them can be used in the methods described herein so long as they are capable of separating individual polynucleotides by at least the difference in length between competitor and target amplicons. The separation technique used should permit resolution of sequences from 25 to 1000 nucleotides or base pairs long and have a resolution of 10 nucleotides or base pairs or better. The separation can be performed under denaturing or under non-denaturing or native conditions-i.e., separation can be performed on single-or double-stranded nucleic acids. It is preferred that the separation and detection permits detection of length differences as small as one nucleotide. It is further preferred that the separation and detection can be done in a high-throughput format that permits real time or contemporaneous determination of amplicon abundance in a plurality of reaction aliquots taken during the cycling reaction. Useful methods for the separation and analysis of the amplified products include, but are not limited to, electrophoresis (e.g., capillary electrophoresis (CE), chromatography (dHPLC), and mass spectrometry).

In one embodiment, CE is a preferred separation means because it provides exceptional separation of the polynucleotides in the range of at least 10-1,000 base pairs with a resolution of a single nucleotide or base pair. CE can be performed by methods well known in the art, for example, as disclosed in U.S. Patent Nos. 6,217,731; 6,001,230; and 5,963,456. High-throughput CE apparatuses are available commercially, for example, the HTS9610 High throughput analysis system and SCE 9610 fully automated 96-capillary electrophoresis genetic analysis system from Spectrumedix Corporation (State College, PA); P/ACE 5000 series and CEQ series from Beckman Instruments Inc (Fullerton, CA); and ABI PRISM 3100 genetic analyzer (Applied Biosystems, Foster City, CA). Near the end of the CE column, in these devices the amplified DNA fragments pass a fluorescent detector that measures signals of fluorescent labels. These apparatuses provide automated high throughput for the detection of fluorescence-labeled PCR products.

The employment of CE in the methods described herein permits higher productivity compared to conventional slab gel electrophoresis. The separation speed is limited in slab gel electrophoresis because of the heat produced when the high electric field is applied to the gel. Since heat elimination is very rapid from the large surface area of a capillary, a higher electric field can be applied in capillary electrophoresis, thus accelerating the separation process. By using a capillary gel, the separation speed is increased about 10 fold over conventional slab-gel systems.

With CE, one can also analyze multiple samples at the same time, which is essential for high-throughput. This is achieved, for example, by employing multicapillary systems. In some instances, the detection of fluorescence from DNA bases may be complicated by the scattering of light from the porous matrix and capillary walls. However, a confocal fluorescence scanner can be used to avoid problems due to light scattering (Quesada et al., 1991, Biotechniques 10 : 616-25).

In one embodiment, the methods described herein measure the amount (i.e., copy number) of a particular pathogen specifc target polynucleotide (e.g., DNA or RNA) contained in the sample used as template for amplification.

In another embodiment, differences in pathogen levels may be monitored during the course of immunotherapy or the course of immunosuppression, rather than the exact copy numbers of the pathogen specifc target polynucleotides contained in the sample being measured. The detected signal strength following size separation can be recorded, for example, for each of at least two competitors and the pathogen specific target nucleic acid in two separate samples and used to determine the relative ratio of the target polynucleotide from two samples. A threshold cycle number (Ct) is calculated as a cycle number at which signal intensity of PCR product will reach a set threshold value (for example 10 standard deviations of background value of signal intensity) for an amplified product. Operational differential expression of a particular target is determined as a difference in threshold cycle number (Ct) for this target in two (or more) samples, of more than one cycle in value. In addition to the quantitation achieved by reference to the signals from at least two competitor nucleic acids in such an embodiment, the threshold cycle number for a given target in a given reaction can be further used to derive copy number for the target polynucleotide and to measure the difference in the expression by a ratio of copy numbers for the target in two or more samples.

The nucleic acid fragments that are products of the PCR or other amplification reaction may be separated (e.g., according to size) and detected, using standard methods known in the art, including, without limitation, gel electrophoresis (such as agarose gel electrophoresis, polyacrylamide gel electrophoresis, and capillary gel electrophoresis), chromatography (such as high-performance liquid chromatography (HPLC) and gas chromatography (GC)), spectrometry (such as mass spectrometry (MS) and GC-MS), infra-red spectrometry, and UV spectrometry), spectrophotometry (such as fluorescence spectrophotometry), atmospheric pressure chemical ionization mass spectroscopy, potentiostatic amperometry, immunoassays (such as ELISA), electrochemical detection, and melting-curve analysis.

Various mass spectrometry techniques have been used to analyze DNA of different sizes (Nelson et al., "Volatilization of High Molecular Weight DNA by Pulsed Laser Ablation of Frozen Aqueous Solutions, Science, 246, 1585-87 (1989); Huth-Fehre et al., Rapid Communications in Mass Spectrometry, 6, 209-13 (1992); K. Tang et al., Rapid Communications in Mass Spectrometry, 8, 727-730 (1994); Williams et al., "Time-of Flight Mass Spectrometry of Nucleic Acids by Laser Ablation and Ionization from a Frozen Aqueous Matrix," Rapid Communications in Mass Spectrometry, 4, 348-351 (1990)).

In recent years, the development of an ionization technique for mass spectrometers known as matrix-assisted laser desorption ionization (MALDI) has generated considerable interest in the use of time-of flight mass spectrometers and in improvement of their performance. MALDI is particularly effective in ionizing large molecules (e.g. peptides and proteins, carbohydrates, glycolipids, glycoproteins, and oligonucleotides (DNA)) as well as other polymers, (MALDI-TOF analysis: Ross, High level multiplex genotyping by MALDI-TOF mass spectrometry, Nature Biotechnology 16 (1998), 1347- 1351). Thus mass spectrophoretic methods may be used to detect and/or quantify amplified nucleic acid products of the methods described herein, as well as any of the pathogen specifc markers or host response gene products, be the products and markers nucleic acid, protein, lipid or other polymer.

Host Response

Host responses against pathogens are elicited upon infection by the parasites. The products of genes activated in a host response can be used in the methods described herein either as a marker of pathogen infection. Alternatively, host genes can be used as reference controls in the multiplex assay. In either case the products of host genes (transcripts or polypeptides) can be detected and/or quantified simultaneously with the identification and/or quantification of the pathogen specific sequences or other markers in a given biological sample. In one aspect, the products are encoded by early host response genes. Examples of host response gene products include but are not limited to cytokines, chemokines, ligands, and other molecules that might alter, increase or otherwise enhance the host response the pathogen. Depending on the type and course of immunosuppression, some of these host response genes may not be expressed in immunosuppressed patients to the same extent as in normal patients. However, optimally the host response gene is coexpressed with the pathogen specific marker of interest, allowing both to be detected simultaneously.

A host response against one or more pathogens typically elicits an inflammatory response, which includes activation of a cascade of factors that can be detected at the nucleic acid and/or protein level. Typically, a pathogen evades or destroy primary barriers of the host such as epithelial or endothelial cells, resulting in tissue damage. The tissue damage results in the production of proinflammatory mediators which include the plasma protease systems, lipid mediators and proinflammatory peptides and cytokines. Plasma proteases include those in the complement pathway, those in the kinin cascade, and those involved in homeostasis. Lipid mediators of inflammation include prostaglandins, leukotrienes and platelet activating factor. Proinflammatory peptides include histamine and serotonin, neuropeptides, and the acute phase plasma proteins including C-reactive protein, serum Amyloid A and fibrinogen. Proinflammatory cytokines include but are not limited to TNF alpha, IL-1-beta, and IL-6. Additional inflammation mediators include but are not limited to leptin and lipocalins.

The methods described herein also comprise monitoring the development of an infectious disease caused by infection by one or more pathogens of interest in an immunocompromised patient or from an individual who is at risk of developing infectious disease from said one or more pathogens of interest, wherein the pathogens of interest are selected from a group consisting of viruses, bacteria, or protozoans, and any combination there of, comprising a) obtaining a biological sample from the patient or individual, b) detecting and quantitating one or more pathogen-specific markers which are indicative of the one or more pathogens of interest, wherein the pathogen-specific markers can comprise nucleic acid, proteins, polysaccharides and/or or lipids, or any combination thereof, derived from said one or more of pathogens in said sample, and c) calculating the quantity of one or more of said pathogens of interest in a sample, wherein said quantity is expressed in terms of the copy number of the microorganism per volume and/or weight of said sample.

In the above mentioned methods of pathogen detection in a biological sample, the immunocompromized patient can and may likely be asymptomatic for an infectious disease. The calculated quantity of the one or more pathogens of interest in the sample tested allows for an assesment of the likelihood of development of a disease resulting from infection by the one or more pathogens, and can be one factor in determining what, if any, preventive therapeutic treatment will be administered to the tested immunocompromised patient, or can be one factor in determining what, if any, alteration there will be in the regimen of immunosuppressive treatment. The immunocomporomized patient can be a recipient of a transplant or a graft, and can be undergoing immunosuppressive therapy.

In the aforementioned methods of pathogen detection in a biological sample, the one or more pathogens of interest are assessed in a multiplexed assay, and can be assessed in a panel of pathogen-specific tests performed on a single patient sample. In one aspect, the patient sample can be selected from the group consisting of blood, saliva, and urine. The quantity of each of the pathogen-specific markers can be measured using antibodies specific to each of said pathogens, and can be performed on regular schedule to monitor emergence or progression of infectious disease. The monitoring can be for example, at least once a month, or more frequently.

**EXAMPLES**

*Example 1. Oligonucleotide Design and Synthesis.*

Primers are selected using PrimerSelect software (DNASTAR Inc, Madison, WI) based on the following criteria:

19-24 nucleotides in length; Melting temperature (Tm) 54.5-58.2°C; primer stability -45.9 to -39.9 kcal per mole; unique primer 3' sequence of 7 nucleotides; avoiding self-primer and primer pair formation longer than 2 contiguous bases (ignoring duplexing 8 bases from 3' end); avoiding internal primer hairpins longer than 2 bases; with minimal 3' pentamer stability of -8.5 kcal per mole or more.

In addition, selected primer pairs are assessed for dimer formation in multiplex across different pairs to eliminate any potential dimers with stability less than -6.0 kcal per mole. Furthermore, primers are screened against none-redundant DNA database (Gene Bank, NCBI) using BLAST search program to eliminate any primers with significant (greater than 14 contiguous nucleotides over or 10 contiguous nucleotides from 3'-end) homology to mammalian polynucleotides.

*Example 2. PCR Amplification and End Detection of Microorganisms.*

A. One-Step RT-PCR detection of microorganism RNA using microorganism-specific primers.

RNA template is added to the reaction mixture containing 0.25uM of each RT primer (optional), 0.25uM of gene-specific PCR primers (one primer of microorganism-specific pair labeled with FAM at 5' end), a modified 1X Stratagene RT-PCR buffer (Brilliant Single Q-RT-PCR kit cat.# 600532), 0.1% Triton X100, 0.2mM dNTP, 1.5M MgCl$_2$, and 1.25U of Stratascript RTase (Stratagene, La Jolla, CA) in a total volume of 50 or 100ul, and overlaid with a mineral oil. Reverse transcription is conducted at 45°C for 50min, followed by 2min incubation at 94C to inactivate the RTase. Samples are then PCR amplified using a protocol consisting of 44 cycles of 94°C for 30 seconds, 60°C for 30 seconds and 72°C for 1 minute. While ramping up to the first 72C extension, 1U of thermostable DNA polymerase (Vent exo(-) (New England Biolabs)) is added. After 44 cycles of amplification aliquots (3-5 ul) are immediately mixed with formamide to stop the reaction. Samples are analyzed by capillary electrophoresis as described below.

To make sure that absence of amplification product is not due to failure of reaction components a control RNA template at 10-1000 copies per reaction and a pair of primers (0.25 uM) for the control template are added to the reaction mixture prior to RT-PCR. Presence of the amplified control template in absence of microorganism -specific amplified products is considered as indication of the absence of the specific microorganism.

<u>Separation of samples by capillary electrophoresis.</u> Three ul of the sample is added to 7ul of formamide containing appropriate fluorescently labeled DNA size standards (Bio Ventures, Murfreesboro, TN). Samples are heat denatured, spun and loaded onto the 3100 Genetic Analyzer capillary electrophoresis instrument (ABI, Foster City, CA). Samples are injected at 3kV for 20 seconds then separated at 153kV on POP4 polymer (ABI, Foster City, CA). The data are analyzed for peaks and relative areas by Gene Scan v3.7.1 software provided with the instrument.

<u>B. Two-step RT-PCR protocol.</u>

For reverse transcription, RNA template and RT specific oligonucleotide primers are added to 10% glycerol, heated at 70°C for 10 minutes, then put on ice for 2 minutes. Buffer (final concentrations: 50mM Tris-HCl, pH 8.3, 75mM KCl, 3mM $MgCl_2$, 0.01M DTT, 0.8mM dNTP, 0.2mg/ml BSA, 20% trehalose), 160U of Superscript II RNase H$^-$ Reverse Transcriptase (SSRTII; Invitrogen, Carlsbad, CA) and 32U of RNAsin (Ambion, Austin, TX) are added for a total volume of 40ul. Reverse transcription proceeds at 45C for 20min, followed by a denaturation step at 75°C. A second round of reverse transcription at 48°C for 30min is initiated with the addition of 50U SSRTII. The sample undergoes another denaturation step at 80°C for 2 minutes followed by another round of reverse transcription at 52°C for 30 min with the addition of 50U SSRTII. Samples are alkaline treated with 0.04M NaOH (final concentration) and incubated for 15 min at 65°C, after which a final concentration of 0.07M Tris, pH 7.5 is added and the sample is then incubated for 5 min. at room temperature. Samples are then cleaned up using the QIAquick Gel Extraction Kit (Qiagen, cat. 28704, Valencia, CA) per manufacturers instructions except that 360ul of QG buffer is added to each RT sample to adjust for pH prior to extraction. Samples are eluted in 50ul 10mM Tris, pH 8.5. Second strand synthesis consists of adding first strand DNA to 40mM Tris-HCl (pH 7.5), 20mM $MgCl_2$, 50mM NaCl, 0.2 dNTP's and 1.6uM of upper second strand primer in a total volume of 60ul. The mixture without the primer is heated to 95°C and then the primer is added. The reaction is denatured at 95C for 4 minutes, ramped to 37°C and 6.5U of Sequenase DNA polymerase is added. The reaction is then incubated for 0.5-1 hour at 37°C. Samples are again purified using the QIAquick Gel Extraction Kit from Qiagen,(Cat. No. 28704) as above and subjected to PCR amplification. The reaction buffer consists of 10mM KCl, 10mM $(NH_4)_2SO_4$, 20mM Tris-HCl (pH 8.8), 2mM $MgSO_4$, 0.1% Triton X-100, 0.2mM dNTP's, 20% Q solution (Stratagene, La Jolla, CA), 2% DMSO, 2U Vent or Vent-exo(-) DNA polymerase (New England Biolabs, Beverly, Ma.) and 10uM of the appropriate primers in which one was labeled with a fluorescent probe. The sample is denatured at 95°C without primers and enzyme for 1 minute. PCR primers are then added, and denaturation continues for an additional 4 minutes. Amplification was performed at 95°C for 30 seconds, 62°C for 30 seconds and 72°C for 1 minute for 45 cycles. Vent polymerase is added while ramping up to the first 72°C extension cycle. After 44 cycles of amplification, or throughout the amplification cycle, aliquots (3-5 ul) were removed and immediately mixed with formamide to stop the reaction. Samples were analyzed by capillary electrophoresis as described above.

*Example 3. PCR Amplification and Real-time Detection of Microorganisms*

<u>A. One-Step RT-PCR detection of Microorganism RNA using gene-specific primers.</u>

Briefly, in a total volume of 50 or 100ul, RNA sample (1-5 ul) is added to the reaction mixture containing 0.25uM of each RT primer (optional), 0.25uM of microorganism-specific PCR primers (one primer of microorganism -specific pair labeled with FAM at 5' end), a modified 1X Stratagene RT-PCR buffer (Brilliant Single Q-RT-PCR kit cat.# 600532), 0.1% Triton X100, 0.2mM dNTP, 1.5mM $MgCl_2$, and 1.25U of StrataScript RTase (Stratagene, La Jolla, CA) and overlaid with a mineral oil. Reverse transcription is conducted at 45C for 50min, followed by 2 min incubation at 94°C to inactivate the RTase. Samples are then PCR amplified using a protocol consisting of 44 cycles of 94°C for 30 seconds, 60°C for 30 seconds and 72°C for 1 minute. While ramping up to the first 72°C extension, 1U of thermostable DNA polymerase (Vent exo(-) (New England Biolabs) is added. To add the DNA polymerase simultaneously to multiple tubes, polymerase is pre-dispensed to fresh tube caps and caps covering PCR tubes are replaced with caps containing DNA polymerase. After 20 cycles of PCR amplification, 3ul aliquots are successively collected at the end of the extension period for 24 cycles. Aliquots are immediately mixed with formamide to stop the reaction. Samples are analyzed by capillary electrophoresis as described below.

To make sure that absence of amplification product is not due to failure of reaction components a control RNA template at 10-1000 copies per reaction and a pair of primers (0.25 uM) for the control template are added to the reaction mixture prior to RT-PCR. Presence of the amplified control template in absence of microorganism -specific amplified products was considered as indication of the absence of the specific microorganism.

<u>Separation of samples by capillary electrophoresis.</u> Three ul of the sample is added to 7ul of formamide containing appropriate fluorescently labeled DNA size standards (Bio Ventures, Murfreesboro, TN). Samples are heat denatured, spun and loaded onto the 3100 Genetic Analyzer capillary electrophoresis instrument (ABI, Foster City, CA). Samples are injected at 3kV for 20 seconds then separated at 15kV on POP4 polymer (ABI, Foster City, CA). The data are analyzed for peaks and relative areas by Gene Scan v3.7.1 software provided with the instrument.

Data analysis: Relative peaks areas corresponding to target microorganism-specific amplicons are plotted as a logarithmic function of PCR cycle number in Microsoft Excel. The linear portion of the each curve is extrapolated to arbitrary threshold (e.g. 1000 relative fluorescent units) to calculate Threshold Cycle (Ct) number. Ct values for known copy numbers of microorganism in the reaction are used to generate a calibration curve.

B. Two-Step RT-PCR detection of Microorganism RNA using tagged gene-specific primers.

For reverse transcription, sample RNA and RT specific oligonucleotide primers are added to 10% glycerol, heated at 70C for 10 minutes, then put on ice for 2 minutes. Buffer (final concentrations: 50mM Tris-HCl, pH 8.3, 75mM KCl, 3mM $MgCl_2$, 0.01M DTT, 0.8mM dNTP, 0.2mg/ml BSA, 20% trehalose), 160U of Superscript II RNase H$^-$ Reverse Transcriptase (SSRTII; Invitrogen, Carlsbad, CA) and 32U of RNAsin (Ambion, Austin, TX) are added for a total volume of 40ul. Reverse transcription proceeds at 45°C for 20min, followed by a denaturation step at 75°C. A second round of reverse transcription at 48°C for 30min is initiated with the addition of 50U SSRTII. The sample undergoes another denaturation step at 80C for 2 minutes followed by another round of reverse transcription at 52°C for 30 min with the addition of 50U SSRTII. Samples are alkaline treated with 0.04M NaOH (final concentration) and incubated for 15 min at 65°C, after which a final concentration of 0.07M Tris, pH 7.5 is added and the sample is then incubated for 5 min. at room temperature. Samples are then cleaned up using the QIAquick Gel Extraction Kit (Qiagen, (cat. 28704, Valencia, CA) as per manufacturers instructions except that 360ul of QG buffer is added to each RT sample to adjust for pH prior to extraction. Samples are eluted in 50ul 10mM Tris, pH 8.5. Second strand synthesis consists of adding first strand DNA to 40mM Tris-HCl (pH 7.5), 20mM $MgCl_2$, 50mM NaCl, 0.2 dNTP's and 1.6uM of upper second strand primer in a total volume of 60ul. The mixture without the primer is heated to 95°C and then the primer is added. The reaction is denatured at 95°C for 4 minutes, ramped to 37°C and 6.5U of Sequenase DNA polymerase is added. The reaction is then incubated for 1 hour at 37°C. Samples are again purified using the QIAquick Gel Extraction Kit from Qiagen, (Cat. No. 28704) as above. PCR amplification was performed in a total volume of 100ul, with DNase free mineral oil overlaying the reaction to prevent evaporation during the experiment. The reaction buffer consists of 10mM KClk, 10mM $(NH_4)_2SO_4$, 20mM Tris-HCl (pH 8.8), 2mM $MgSO_4$, 0.1% Triton X-100, 0.2mM dNTP's, 20% Q solution (Stratagene, La Jolla, CA), 2% DMSO, 2U Vent DNA polymerase (New England Biolabs, Beverly, Ma.) and 10uM of the appropriate primers in which one is labeled with a fluorescent probe. The sample is denatured at 95°C without primers and enzyme for 1 minute. PCR primers are then added, and denaturation continues for an additional 4 minutes. Amplification consists of varying number of cycles (dependent on the experiment) of 95°C for 30 seconds, 62°C for 30 seconds and 72C for 1 minute. While ramping up to the first 72°C extension cycle, Vent polymerase is added. Aliquots of 3ul are taken for 24 successive cycles and immediately added to 7ul of formamide containing appropriate standards (see above).

*Example 4: End-Point Detection of microorganisms using PCR amplification with fluorescently* labeled dNTPs and separation of labeled DNA fragments by Capillary Electrophoresis.

Plasma RNA extract containing 5000 copies of microorganism RNA is mixed with unlabeled microorganism-specific primers (0.25uM) and dNTPs (100 uM of each, dATP, dCTP, dGTP and 65 uM dTTP), in 50 uL of Brilliant Single-Step Quantitative RT-PCR Core Reagent buffer (Stratagene Cat no. 600532) containing 0.1% Triton X-100, 1.5mM $MgCl_2$, and 1.25U of StrataScript RTase (Stratagene, La Jolla, CA) and reverse transcribed at 45°C for 50 min. Reaction is terminated by heating at 94°C for 2 min. Upon completion of RT, 1U of Vent(Exo-) DNA polymerase (NE Biolabs CAT no. M0257S) and 350uM fluorescein-12-2'-deoxy-uridine-5'-triphosphate (obtained from Roche CAT no. 1 373 242) are added to the mixture. PCR amplification is performed for 40 cycles as 30s at 94°C, 30 s at 60°C, 30 s at 72°C. 3 ul aliquot is taken at the end of PCR amplification and analyzed by capillary electrophoresis as described above.

*Examples 5: Real-time PCR amplification with fluorescently labeled dNTPs and separation of labeled DNA fragments by Capillary Electrophoresis.*

Serial dilutions microorganism RNA in plasma RNA extract ARE mixed with unlabeled microorganism-specific primers (0.25uM) and dNTPs (100 uM of each, dATP, dCTP, dGTP and 65 uM dTTP), in 50 uL of Brilliant Single-Step Quantitative RT-PCR Core Reagent buffer (Stratagene Cat no. 600532) containing 0.1% Triton X-100, 1.5M $MgCl_2$, and 1.25U of StrataScript RTase (Stratagene, La Jolla, CA) and reverse transcribed at 45 C for 50 min. Reaction Is terminated by heating at 94°C for 2 min. Upon completion of RT, 1U of Vent(Exo-) DNA polymerase (NE Biolabs Cat no. M0257S) and 350uM fluorescein-12-2'-deoxy-uridine-5'-triphosphate (obtained from Roche Cat no. 1 373 242) are added to the mixture. PCR amplification is performed for 40-45 cycles at 30s at 94°C, 30s at 60°C, 30 s at 72°C. 3 ul aliquot is taken at the end of each PCR cycle starting with cycle 24 and analyzed by capillary electrophoresis as described above. Relative peaks areas corresponding to microorganism-specific amplicons are plotted as a logarithmic function of PCR cycle number in Microsoft Excel. The linear portion of the each curve is extrapolated to arbitrary threshold (e.g. 1000 relative fluorescent units) to calculate Threshold Cycle (Ct) number. Ct values for known copy numbers of microorganism in the reaction are used to generate a calibration curve.

Alternatively, for detection experiments, each sample is serially diluted tenfold from the starting concentration in appropriate non-spiked control RNA and used in a OneStep RT-PCR protocol. For experiments using purified microorganism RNA, dilutions are performed in *E. coli* tRNA at 20ng/ul. Briefly, in a total volume of 50 or 100ul, RNA template and 0.25uM of each RT primer is added to a mixture containing a modified 1X Stratagene buffer (cat.# 600532), 0.1% Triton X100, 0.2mM dNTP, 1.5M $MgCl_2$, and 1.25U of StrataScript RTase (Stratagene, La Jolla, CA)and reverse transcribed at 45°C for 50min, followed by 2min at 94C to inactivate the RTase. Samples are then PCR amplified using a protocol consisting of 44 cycles of 94°C for 30 seconds, 60°C for 30 seconds and 72°C for 1 minute. While ramping up to the first 72°C extension, 1U of thermostable DNA polymerase is added. After 20 cycles, 3ul aliquots are successively collected at the end of the extension period for 24 cycles. Aliquots are immediately added to denaturant to stop the reaction. Samples are analyzed by capillary electrophoresis as described above.

*Example 6: Six-plex viral assay for the detection of pathogens in a sample*

Whole human blood was collected in an EDTA collection tube, and plasma was prepared using a standard method within 24 hours of collection. DNA was extracted using the Corbett Xtractor and eluted into 75uL of elution buffer. Once processed, samples were screened on the same day. Ten microliters of extracted DNA was tested in each reaction. Reactions were performed in duplicate.

Each reaction mixture contained the following: 1X Qiagen Multiplex buffer, 10% betaine, primers for each target at a concentration between 0.05 and 0.400uM, and a sensitivity control plasmid for each viral target at 100 copies per reaction. Each reaction mixture was overlaid with mineral oil to prevent evaporation. A no template control was included in each reaction run. A total of 16 reactions were run simultaneously.

Reactions were assembled in the PCR clean room and transferred to the templating area where DNA-extracted samples were added to each reaction. Reactions were then transferred to a dispensing thermocycler and PCR amplified using the following protocol:

| | | | | |
|---|---|---|---|---|
| a. | 1 cycle: | 95°C for 15min (enzyme activation) | | |
| b. | 3 cycles: | 95°C /30s | 62°C /90s | 72°C/1min |
| c. | 3 cycles: | 95°C /30s | 60°C /90s | 72°C/1min |
| d. | 3 cycles: | 95°C /30s | 58°C/90s | 72C°/1min |
| e. | 31 cycles: | 95°C /30s | 57°C /90s | 72°C/1min |

Two 96-well collection plates for each set of 8 samples were prepared to collect 2uL aliquots from each reaction during the last second of 72°C extension. Eight microliters of formamide containing 0.3uL of ROX-labeled MapMaker 1000 DNA standards (Bioventures) was dispensed into each well of a 96 well plate and placed in the collection area of the dispensing thermocycler.

Two microliter aliquots were removed from each reaction during the final second of 72°C extension phase beginning at cycle18 and continuing through cycle 40 and transferred to the collection plate.

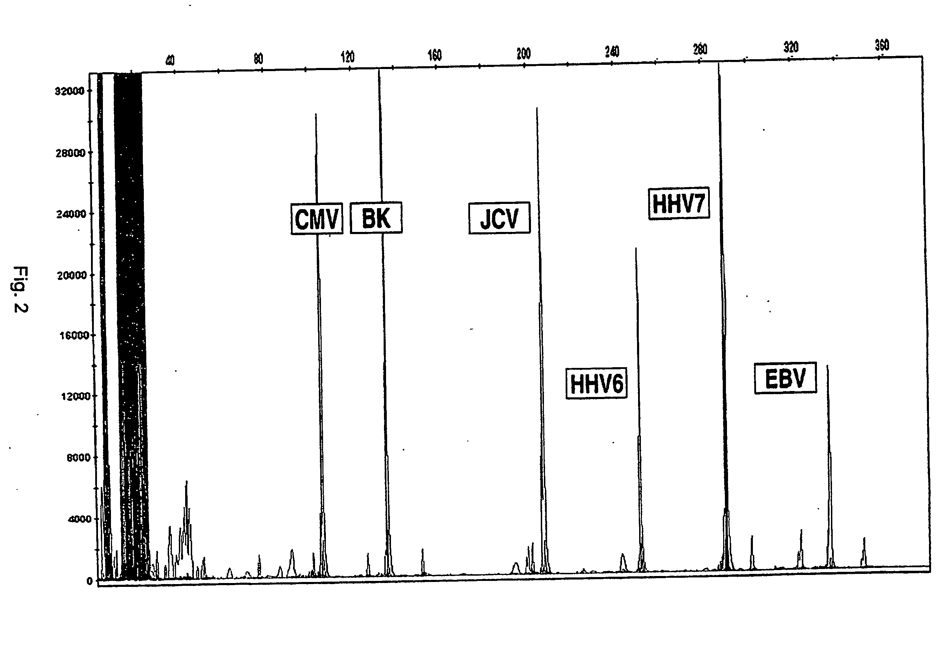

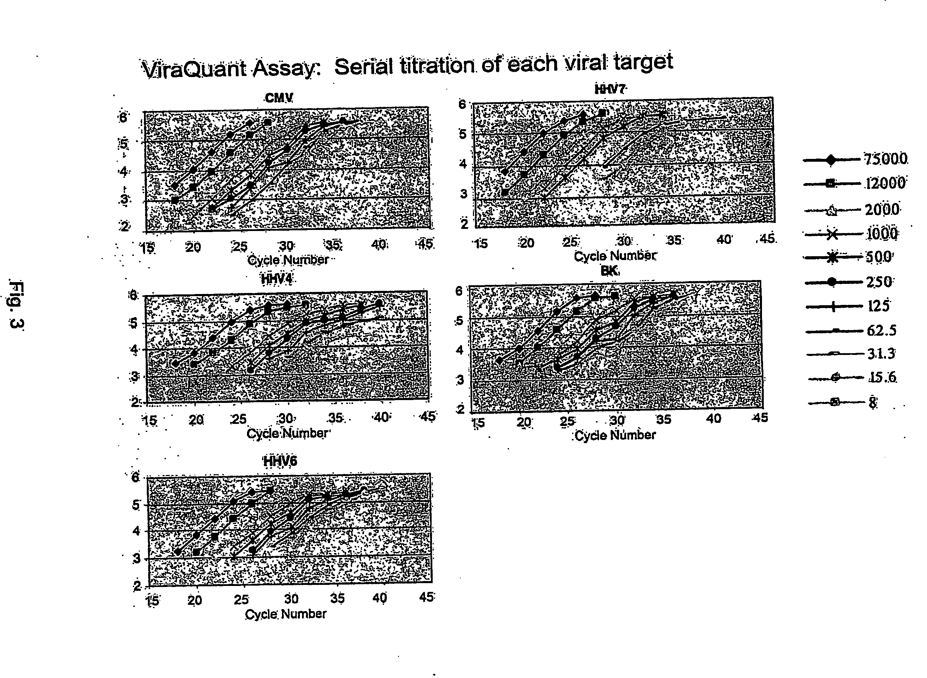

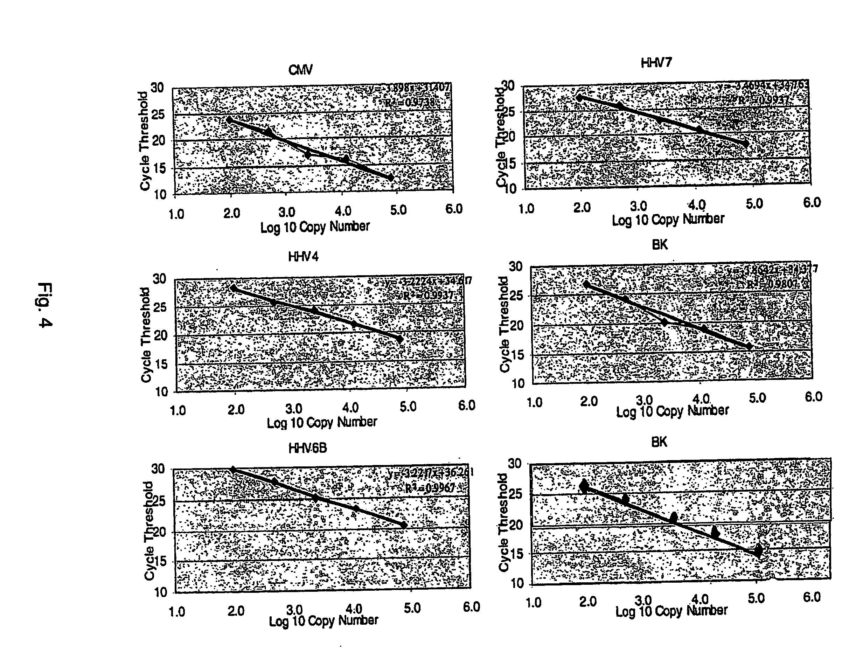

At the end of PCR amplification, collection plates were heat sealed, centrifuged and run on an ABI 3730XL (Applied Biosystems, Foster City, CA) genetic analyzer for fragment analysis (Figure 2). Data generated was processed to determine relative fluorescence units (log peak area) and plotted on a log scale versus cycle number (Figure 3). Threshold cycles for each viral target were calculated by plotting log of peak area for each specific amplicon versus cycle number and selecting cycle number value which corresponded to 35000 fluorescent units calculated by Gene Mapper data analysis software (Applied Biosystems, Foster City, CA) (Figure 4). Calibration plots to determine Threshold cycles (Ct) as a function of viral load for each specific target were created by measuring Ct of predetermined amount of viral DNA.

Viral load in clinical samples can be determined using specific calibration plot for each viral target by selecting viral load value corresponding to measured Threshold cycles for this specific viral target. Target specific oligonucleotides for detection of CMV, EBV, BK, HHV6, HHV7, JCV, and human mitochondrial DNA are provided in Table 5.

The assay quantitatively detected the presence of CMV, EBV, BK, HHV6, HHV7, and JCV. Human mitochondrial sequence was also detected in the assay as a quality measure to confirm successful DNA extraction from clinical samples (sample preparation was considered to be successful as the measured Threshold Cycle for mitochondrial amplicon was in the range of 23-27 cycles).

OTHER EMBODIMENTS

The foregoing embodiments demonstrate experiments performed and techniques contemplated by the present inventors in making and carrying out the invention. It is believed that these embodiments include a disclosure of techniques which serve to both apprise the art of the practice of the invention and to demonstrate its usefulness. It will be appreciated by those of skill in the art that the techniques and embodiments disclosed herein are preferred embodiments only that in general numerous equivalent methods and techniques may be employed to achieve the same result.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

The present invention may be as defined in any one of the following numbered paragraphs.

1. A method of analyzing a sample suspected of containing any of a plurality of predetermined pathogens to detect a pathogen specific target, the method comprising:

a) selecting a pathogen-specific primer pair corresponding to each pathogen of the plurality of predetermined pathogens, wherein the pathogen-specific primer pairs are capable of mediating amplification of a polynucleotide amplicon of a selected, known length from a nucleic acid pathogen specific target of the corresponding pathogen;

b) contacting a nucleic acid from the sample suspected of containing any of the plurality of predetermined pathogens with a plurality of pathogen-specific primer pairs in a reaction mixture in an amplification step under conditions that promote amplification of a polynucleotide amplicon;

c) removing an aliquot of the reaction mixture at one or more intervals during the amplification step;

d) separating the amplicons in each aliquot; and

e) detecting the amplicons in each aliquot;
wherein the detection of one or more amplicons of a selected, known length is indicative of the sample containing the pathogen specific target corresponding to the amplicon.

2. The method of para. 1 further comprising quantifying the detected amplicon and correlating the amount of detected amplicon with the amount of pathogen present in the sample.

3. The method of para. 1 or 2, wherein the amplification step comprises amplification by the polymerase chain reaction (PCR).

4. The method of any of paras. 1 to 3, wherein the nucleic acid from the sample is subjected to reverse transcription (RT) prior to the amplification step.

5. The method of any of paras. 1 to 4, wherein the amplicons of step (c) are separated by capillary electrophoresis.

6. The method of any of paras. 1 to 5, wherein the one or more of the pathogen specific primers comprise a detectable label.

7. The method of any of paras. 1 to 6, wherein the amplicon is detected by a nucleic acid binding dye that includes a detectable label.

8. The method of para. 6 or 7, wherein the detectable label is selected from the group consisting of fluorescent label, radioactive label, colormetric label, magnetic label, and enzymatic label.

9. The method of any of paras. 6 to 8, wherein the detectable label is a fluorescent label.

10. The method of any of Paras. 6 to 9, further comprising quantifying the amount of pathogen in the sample by quantifying the amount of detectable label in the aliquot of the reaction mixture.

11. The method of any of paras. 1 to 10, wherein the pathogens are selected from the group consisting of virus, bacteria, protozoa, and a combination thereof.

12. The method of any of paras. 1 to 11, wherein the plurality of specific pathogen targets comprises specific viral targets selected from the group consisting of: HSV1, HSV2, EBV, CMV, HHV 6, HHV7, HHV8, VZV, hepatitis C, hepatitis B, adenovirus, EEEV, WNE, JCV and BKV.

13. The method of any of paras. 1 to 12, wherein the method is capable of detecting at least two pathogens in the sample.

14. The method of any of paras. 1 to 12, wherein the method is capable of detecting at least three pathogens in the sample.

15. The method of any of paras. 1 to 12, wherein the method is capable of detecting at least four pathogens in the sample.

16. The method of any of paras. 1 to 12, wherein the method is capable of detecting at least five pathogens m the sample.

17. The method of any of paras. 1 to 12, wherein the method is capable of detecting at least six pathogens in the sample.

18. The method of any of paras. 1 to 12, wherein the method is capable of detecting at least eight pathogens in the sample.

19. The method of any of paras. 1 to 18 wherein all of the pathogen-specific primer pairs are able to promote amplification of a polynucleotide under at least one common set of reaction conditions.

20. The method of any of paras. 1 to 19, wherein the sample is a pathogen host sample.

21. The method of para. 20, wherein the host is a mammal.

22. The method of para. 20 or 21, wherein the host is a human.

23. The method of any of paras. 20 to 22, wherein the host is an immunocompromised human.

24. The method of para. 23, wherein said immunocompromised individual has received a transplant.

25. The method of para. 23, wherein said immunocompromized individual has undergone chemotherapy for the treatment of cancer.

26. The method of any of paras. 23 to 25, wherein said method is used to monitor a course of immunosuppressive treatment.

27. The method of any of paras. 22 to 26, wherein the host is a human asymptomatic for pathogen infection.

28. The method of any of paras.: 20 to 27, wherein the method includes amplification of nucleic acid of the host.

29. The method of any of paras. 1 to 28, wherein the method also detects the presence of a control nucleic acid molecule not derived from the sample by amplification of an amplicon from the control nucleic acid molecule.

30. The method of para. 29, wherein the control nucleic acid molecule is a competitor nucleic acid molecule.

31. The method of para. 29 or 30, wherein a plurality of distinct control nucleic acid molecules are detected.

32. The method of any of paras. 29 to 31, wherein the control oligonucleotide is present in the reaction at a known concentration.

33. The method of para. 31, wherein the distinct control oligonucleotides are present in the reaction at different concentrations.

34. The method of any of paras. 29 to 33, wherein the amplicon amplified by the control oligonucleotide is amplified using a pathogen specific primer pair.

35. The method of any of paras. 29 to 33, where the control nucleic acid is amplified at a similar efficiency as the pathogen specific target nucleic acid.

36. The method of any of paras. 29 to 35, wherein the control nucleic acid molecule is DNA and/or RNA.

37. The method of any of Paras. 29 to 36, wherein the pathogen specific primers amplify the nucleic acid of the pathogen and the control nucleic acid molecule with similar efficiency.

38. A method for detection and quantitation of a plurality of predetermined pathogens in a sample, the method comprising:

a) obtaining a sample suspected of containing at least one of a plurality of pathogens wherein each pathogen comprises a pathogen-specific polynucleotide;

b) isolating nucleic acid from the sample;

c) selecting a pathogen-specific primer pair targeted to each of the plurality pathogen-specific polynucleotides wherein the primer pair capable of mediating amplification of an amplicon of a length that is distinct from amplicons produced by each of the other pathogen-specific primer pairs targeted to each other member of the plurality of pathogen-specific nucleic acids;

d) selecting at least one competitor polynucleotide wherein the competitor polynucleotide is capable of acting as a template to mediate amplification of an amplicon by one pathogen-specific primer pair wherein the amplicon produced is of a length that is distinct from amplicons produced by each of the other pathogen-specific primer pairs targeted to each other member of the plurality of pathogen-specific nucleic acids and other competitor polynucleotides;

e) adding a predetermined amount of at least one competitor polynucleotide to nucleic acid isolated from the sample to create a test mixture;

f) contacting the test mixture with a plurality of pathogen-specific primer pairs in the reaction mixture under conditions that promote amplification of an amplicon;

g) separating the amplicons generated in step (f);

h) detecting the length of each amplicon generated in step (f);

i) correlating the length of each amplicon detected with a pathogen or competitor polynucleotide present in the reaction mixture;

j) quantitating the amount of each amplicon generated in step (f); and

k) determining an amount of pathogen present in the sample based on the predetermined amount of competitor polynucleotide added to the test mixture.

39. The method of para. 38, wherein an aliquot of the reaction mixture of step (f) is removed at one or more intervals during the amplification step, and wherein steps (g) through (k) are performed on the aliquot.

40. The method of paras. 38 and 39, wherein the amount of pathogen present in the sample is not detectable.

41. The method of any of Paras. 38 to 40, wherein the amplification of step (f) is performed in a single reaction.

42. The method of any of paras. 38 to 41, wherein the resolving of step (g) is performed by capillary electrophoresis

43. The method of any of paras. 38 to 42, wherein the one or more of the pathogen specific primers comprise a detectable label.

44. The method of any of paras. 38 to 43, wherein the amplicon is detected by a nucleic acid binding dye that includes a detectable label.

45. The method of para. 43 or 44, wherein the detectable label is selected from the group consisting of fluorescent label, radioactive label, colormetric label, magnetic label, and enzymatic label.

46. The method of any of paras. 43 to 45, wherein the detectable label is a fluorescent label.

47. The method of any of paras. 43 to 47, further comprising quantifying the amount of pathogen in the sample by quantifying the amount of detectable label in an aliquot of the reaction mixture.

48. The method of any of paras. 38 to 47, wherein the pathogens are selected from the group consisting of virus, bacteria, protozoa, and a combination thereof.

49. The method of any of paras. 38 to 48, wherein the plurality of specific pathogen targets comprises specmc viral targets selected from the group consisting of HSV1, HSV2, EBV, CMV, HHV 6, HHV7, HHV8, VZV, hepatitis C, hepatitis B, adenovirus, EEEV, WNE, JCV and BKV.

50. The method of any of paras. 38 to 49, wherein the method is capable of detecting at least two pathogens in the sample.

51. The method of any of paras. 38 to 50, wherein the method is capable of detecting at least three pathogens in the sample.

52. The method of any of paras. 38 to 50, wherein the method is capable of detecting at least four pathogens m the sample.

53. The method of any of paras. 38 to 50, wherein the method is capable of detecting at least five pathogens in the sample.

54. The method of any of paras. 38 to 50, wherein the method is capable of detecting at least six pathogens in the sample.

55. The method of any of paras. 38 to 50, wherein the method is capable of detecting at least eight pathogens in the sample.

56. The method of any of paras. 38 to 55, wherein all of the pathogen-specific primer pairs are able to promote amplification of a polynucleotide under at least one common set of reaction conditions.

57. The method of any of paras. 38 to 56, wherein the sample is a pathogen host sample.

58. The method of para. 57, wherein the host is a mammal.

59. The method of para. 57 or 58, wherein the host is a human.

60. The method of any of paras. 57 to 59, wherein the host is an immunocompromised human.

61. The method of para. 60, wherein said immunocompromised individual has received a transplant.

62. The method of para. 60, wherein said immunocompromized individual has undergone chemotherapy for the treatment of cancer.

63. The method of any of paras. 60 to 62, wherein said method is used to monitor course of immunosuppressive treatment.

64. The method of any of paras. 59 to 63, wherein the host is a human asymptomatic for pathogen infection.

65. The method of any of paras. 57 to 64, wherein the method includes amplification of nucleic acid of the host.

66. The method of any of paras. 38 to 65, where the competitor polynucleotide is amplified at a similar efficiency as the pathogen specific target nucleic acid.

67. The method of any of paras. 38 to 66, wherein the competitor polynucleotide is DNA and/or RNA.

68. The method of any of paras. 38 to 67, wherein the nucleic acid from the sample is DNA and/or RNA.

69. The method of para. 68, wherein DNA and RNA are isolated separately from the sample.

70. The method of any of paras. 38 to 69, wherein the quantitation of at least two of the plurality of predetermined pathogens from said sample are combined in a single reaction.

71. The method of any of paras. 38 to 70, wherein the quantitation of at least three of the plurality of predetermined pathogens from said sample are combined in a single reaction.

72. The method of paras. 38 to 71, wherein said sample is blood.

73. The method of any of paras. 60 to 72, wherein said method is used to monitor a course of immunosuppressive treatment.

74. A method of monitoring a subject for development of a disease caused by an infection by a predetermined pathogen, the method comprising:

obtaining a sample from a subject;

quantitating five or more pathogen specific markers indicative of at least one predetermined pathogen;

calculating the amount of at least one predetermined pathogen in the sample wherein the amount is expressed in terms of copy number of the pathogen per volume or weight of the sample.

75. The method of para. 74, wherein the amount of pathogen present is not detectable.

76. The method of paras. 74 or 75, wherein the amount of pathogen present in the sample is none.

77. The method of any of paras. 74 to 76, wherein the subject is an immunocompromised patient

78. The method of any of paras. 74 to 77, wherein the subject is an asymptomatic patient.

79. The method of any of paras. 74 to 78, wherein the calculated quantity of at least one predetermined pathogen.

80. The method of any of paras. 74 to 79, wherein the quantity of at least one pathogens in the sample is used to determine a treatment regimen for the immunocompromised patient.

81. The method of any of paras. 74 to 81, wherein said patient is a recipient of a transplant or a graft, and is undergoing immunosuppressive therapy.

82. The method of any of paras. 74 to 80, wherein immunosuppressive drugs have been administered to the immunocompromised patient, or the immunocompromised patient is currently receiving immunosuppressive drugs.

83. The method of para. 81 or 82, wherein the quantity of one or more of said pathogens of interest in a sample is a factor in determining an alteration an immunosuppressive treatment regimen.

84. The method of any of paras. 74 to 83, wherein the quantity of more than one pathogen in the sample is determined in a single reaction.

85. The method of any of paras. 74 to 83, wherein the quantity of more than one pathogen in the sample is determined in a multiplex assay.

86. The method of any of paras. 74 to 85, wherein the sample is blood, saliva, or urine.

87. The method of any of paras. 74 to 86, wherein the quantity of a plurality of pathogens is determined through amplification of target pathogen-specific nucleic acid.

88. The method of any of paras. 74 to 87, wherein the monitoring method is performed on a regular schedule to monitor the emergence or progression of infectious disease.

89. The method of 88, wherein a regular schedule is about once per month.

**Claims**

1. A method of analyzing a sample to detect the presence of any one of a set of predetermined viral pathogens, the method comprising:

   a) providing a set of pathogen-specific primer pairs corresponding to each of four or more of a set of viral pathogens selected from the group consisting of EBV (HHV4), CMV, HHV6, HHV7, JCV and BKV, wherein the set of primer pairs is selected from the group consisting of

      1) four or more amplification primer pairs selected from the group consisting of

         a) CMV-specific primer pair SEQ ID NOS: 9 and 10;
         b) BKV-specific primer pair SEQ ID NOS: 7 and 8;
         c) BKV-specific primer pair SEQ ID NOS: 15 and 16;
         d) HHV4-specific primer pair SEQ ID NOS: 3 and 4;
         e) HHV6-specific primer pair SEQ ID NOS: 5 and 6;
         f) HHV7-specific primer pair SEQ ID NOS: 1 and 2; and
         g) JCV-specific primer pair SEQ ID NOS: 11 and 12; or

      2) four or more amplification primer pairs selected from the group consisting of:

         h) CMV-specific primer pair SEQ ID NOS: 27 and 28;
         i) CMV-specific primer pair SEQ ID NOS: 37 and 38;
         j) BKV-specific primer pair SEQ ID NOS: 25 and 26;
         k) HHV4-specific primer pair SEQ ID NOS: 21 and 22;
         l) HHV4-specific primer pair SEQ ID NOS: 33 and 34;
         m) HHV4-specific primer pair SEQ ID NOS: 41 and 42;
         n) HHV6-specific primer pair SEQ ID NOS: 23 and 24;
         o) HHV6-specific primer pair SEQ ID NOS: 35 and 36;
         p) HHV7-specific primer pair SEQ ID NOS: 19 and 20; and
         q) JCV-specific primer pair SEQ ID NOS: 29 and 30; or

      3) four or more amplification primer pairs selected from the group consisting of:

r) CMV-specific primer pair SEQ ID NOS: 51 and 52;
s) BKV-specific primer pair SEQ ID NOS: 49 and 50;
t) HHV4-specific primer pair SEQ ID NOS: 45 and 46;
u) HHV6-specific primer pair SEQ ID NOS: 47 and 48;
v) HHV7-specific primer pair SEQ ID NOS: 43 and 44; and
w) JCV-specific primer pair SEQ ID NOS: 53 and 54;

wherein the pathogen-specific primer pairs are capable of mediating amplification of a polynucleotide amplicon of a different selected, known length from a nucleic acid of each of said at least four viral pathogens;

b) contacting a nucleic acid from the sample suspected of containing any of said four or more viral pathogens with said pathogen-specific primer pairs of step (a) in a reaction mixture in an amplification step under conditions that promote amplification of a polynucleotide amplicon;
c) removing an aliquot of the reaction mixture at a plurality of intervals during the amplification step;
d) separating the amplicons in each aliquot; and
e) detecting the amplicons in each aliquot, wherein the detection of an amplicon of a selected, known length is indicative of the sample containing the pathogen specific target corresponding to the amplicon.

2. The method of claim 1 further comprising quantifying the detected amplicon and correlating the amount of detected amplicon with the amount of pathogen present in the sample.

3. The method of claim 1 or 2, wherein the amplification step comprises amplification by the polymerase chain reaction (PCR).

4. The method of any of claims 1 to 3, wherein the nucleic acid from the sample is subjected to reverse transcription (RT) prior to the amplification step.

5. The method of any of claims 1 to 4, wherein the amplicons of step (c) are separated by capillary electrophoresis.

6. The method of any of claims 1 to 5, wherein the one or more of the pathogen specific primers comprise a detectable label.

7. The method of claim 6, wherein the detectable label is a fluorescent label.

8. The method of claim 6 or 7, further comprising quantifying the amount of pathogen in the sample by quantifying the amount of detectable label in the aliquot of the reaction mixture.

9. The method of any of claims 1 to 8, wherein the method is capable of detecting at least four pathogens in the sample.

10. The method of any of claims 1 to 8, wherein the method is capable of detecting at least five pathogens in the sample.

11. The method of any of claims 1 to 10 wherein all of the pathogen-specific primer pairs are able to promote amplification of a polynucleotide under at least one common set of reaction conditions.

12. The method of any one of claims 1 to 11, wherein the sample is obtained from an immunocompromised human.

13. The method of claim 12, wherein said immunocompromised individual has received a transplant.

14. A method for detection and quantitation of a plurality of predetermined pathogens in a sample, the method comprising:

a) obtaining a sample suspected of containing at least one of a plurality of pathogens selected from the group consisting of EBV (HHV4), CMV, HHV6, HHV7, JCV and BKV, wherein each pathogen comprises a pathogen-specific polynucleotide;
b) isolating nucleic acid from the sample;
c) selecting a set of pathogen-specific primer pairs targeted to each of the plurality pathogen-specific polynucleotides, wherein the set of primer pairs is selected from the group consisting of

1) four or more amplification primer pairs selected from the group consisting of

a) CMV-specific primer pair SEQ ID NOS: 9 and 10;
b) BKV-specific primer pair SEQ ID NOS: 7 and 8;
c) BKV-specific primer pair SEQ ID NOS: 15 and 16;
d) HHV4-specific primer pair SEQ ID NOS: 3 and 4;
e) HHV6-specific primer pair SEQ ID NOS: 5 and 6;
f) HHV7-specific primer pair SEQ ID NOS: 1 and 2; and
g) JCV-specific primer pair SEQ ID NOS: 11 and 12; or

2) four or more amplification primer pairs selected from the group consisting of:

h) CMV-specific primer pair SEQ ID NOS: 27 and 28;
i) CMV-specific primer pair SEQ ID NOS: 37 and 38;
j) BKV-specific primer pair SEQ ID NOS: 25 and 26;
k) HHV4-specific primer pair SEQ ID NOS: 21 and 22;
l) HHV4-specific primer pair SEQ ID NOS: 33 and 34;
m) HHV4-specific primer pair SEQ ID NOS: 41 and 42;
n) HHV6-specific primer pair SEQ ID NOS: 23 and 24;
o) HHV6-specific primer pair SEQ ID NOS: 35 and 36;
p) HHV7-specific primer pair SEQ ID NOS: 19 and 20; and
q) JCV-specific primer pair SEQ ID NOS: 29 and 30; or

3) four or more amplification primer pairs selected from the group consisting of:

r) CMV-specific primer pair SEQ ID NOS: 51 and 52;
s) BKV-specific primer pair SEQ ID NOS: 49 and 50;
t) HHV4-specific primer pair SEQ ID NOS: 45 and 46;
u) HHV6-specific primer pair SEQ ID NOS: 47 and 48;
v) HHV7-specific primer pair SEQ ID NOS: 43 and 44; and
w) JCV-specific primer pair SEQ ID NOS: 53 and 54;

and wherein each primer pair is capable of mediating amplification of an amplicon of a length that is distinct from amplicons produced by each of the other pathogen-specific primer pairs targeted to each other member of the plurality of pathogen-specific nucleic acids;

d) selecting at least one competitor polynucleotide wherein the competitor polynucleotide is capable of acting as a template to mediate amplification of an amplicon by one pathogen-specific primer pair wherein the amplicon produced is of a length that is distinct from amplicons produced by each of the other pathogen- specific primer pairs targeted to each other member of the plurality of pathogen- specific nucleic acids and other competitor polynucleotides;
e) adding a predetermined amount of at least one competitor polynucleotide to nucleic acid isolated from the sample to create a test mixture;
f) contacting the test mixture with a plurality of pathogen-specific primer pairs in the reaction mixture under conditions that promote amplification of an amplicon;
g) separating the amplicons generated in step (f);
h) detecting the length of each amplicon generated in step (f);
i) correlating the length of each amplicon detected with a pathogen or competitor polynucleotide present in the reaction mixture;
j) quantitating the amount of each amplicon generated in step (f); and
k) determining an amount of pathogen present in the sample based on the predetermined amount of competitor polynucleotide added to the test mixture.

15. The method of any one of the preceding claims, in which the set of primer pairs comprises five or more of primer pairs (a)-(g), five or more of primer pairs (h)-(q) or five or more of primer pairs (r)-(w).

16. A composition for multiplex detection of viral pathogens according to the method of any one of the preceding claims, wherein the composition comprises a set of amplification primer pairs which, when used in a PCR reaction, permit multiplex amplification of a plurality of viral nucleic acid template sequences, the set comprising:

1) four or more amplification primer pairs selected from the group consisting of

    a) CMV-specific primer pair SEQ ID NOS: 9 and 10;
    b) BKV-specific primer pair SEQ ID NOS: 7 and 8;
    c) BKV-specific primer pair SEQ ID NOS: 15 and 16;
    d) HHV4-specific primer pair SEQ ID NOS: 3 and 4;
    e) HHV6-specific primer pair SEQ ID NOS: 5 and 6;
    f) HHV7-specific primer pair SEQ ID NOS: 1 and 2; and
    g) JCV-specific primer pair SEQ ID NOS: 11 and 12; or

2) four or more amplification primer pairs selected from the group consisting of:

    h) CMV-specific primer pair SEQ ID NOS: 27 and 28;
    i) CMV-specific primer pair SEQ ID NOS: 37 and 38;
    j) BKV-specific primer pair SEQ ID NOS: 25 and 26;
    k) HHV4-specific primer pair SEQ ID NOS: 21 and 22;
    l) HHV4-specific primer pair SEQ ID NOS: 33 and 34;
    m) HHV4-specific primer pair SEQ ID NOS: 41 and 42;
    n) HHV6-specific primer pair SEQ ID NOS: 23 and 24;
    o) HHV6-specific primer pair SEQ ID NOS: 35 and 36;
    p) HHV7-specific primer pair SEQ ID NOS: 19 and 20; and
    q) JCV-specific primer pair SEQ ID NOS: 29 and 30; or

3) four or more amplification primer pairs selected from the group consisting of:

    r) CMV-specific primer pair SEQ ID NOS: 51 and 52;
    s) BKV-specific primer pair SEQ ID NOS: 49 and 50;
    t) HHV4-specific primer pair SEQ ID NOS: 45 and 46;
    u) HHV6-specific primer pair SEQ ID NOS: 47 and 48;
    v) HHV7-specific primer pair SEQ ID NOS: 43 and 44; and
    w) JCV-specific primer pair SEQ ID NOS: 53 and 54.

**17.** The composition of claim 16, in which the set of amplification primer pairs comprises five or more of primer pairs (a)-(g), five or more of primer pairs (h)-(q) or five or more of primer pairs (r)-(w).

**Patentansprüche**

**1.** Analyseverfahren für eine Probe zum Erfassen der Anwesenheit irgendeines aus einem Satz vorbestimmter viraler Krankheitserreger, das Verfahren umfassend

a) Bereitstellen eines Satzes Krankheitserreger-spezifischer Primerpaare, die jedes vier oder mehr aus einem Satz viraler Krankheitserreger entsprechen, ausgewählt aus der Gruppe EBV (HHV4), CMV, HHV6, HHV7, JCV und BKV, wobei der Satz Primerpaare ausgewählt ist aus der Gruppe

1) vier oder mehr Vervielfältigungs-Primerpaare, ausgewählt aus der Gruppe

    a) CMV-spezifisches Primerpaar SEQ ID NOS: 9 und 10;
    b) BKV-spezifisches Primerpaar SEQ ID NOS: 7 und 8;
    c) BKV-spezifisches Primerpaar SEQ ID NOS: 15 und 16;
    d) HHV4-spezifisches Primerpaar SEQ ID NOS: 3 und 4;
    e) HHV6-spezifisches Primerpaar SEQ ID NOS: 5 und 6;
    f) HHV7-spezifisches Primerpaar SEQ ID NOS: 1 und 2; und
    g) JCV-spezifisches Primerpaar SEQ ID NOS: 11 und 12; oder

2) vier oder mehr Vervielfältigungs-Primerpaare, ausgewählt aus der Gruppe

    h) CMV-spezifisches Primerpaar SEQ ID NOS: 27 und 28;

i) CMV-spezifisches Primerpaar SEQ ID NOS: 37 und 38;
j) BKV-spezifisches Primerpaar SEQ ID NOS: 25 und 26;
k) HHV4-spezifisches Primerpaar SEQ ID NOS: 21 und 22;
l) HHV4-spezifisches Primerpaar SEQ ID NOS: 33 und 34;
m) HHV4-spezifisches Primerpaar SEQ ID NOS: 41 und 42;
n) HHV6-spezifisches Primerpaar SEQ ID NOS: 23 und 24;
o) HHV6-spezifisches Primerpaar SEQ ID NOS: 35 und 36;
p) HHV7-spezifisches Primerpaar SEQ ID NOS: 19 und 20; und
q) JCV-spezifisches Primerpaar SEQ ID NOS: 29 und 30; oder

3) vier oder mehr Vervielfältigungs-Primerpaare, ausgewählt aus der Gruppe

r) CMV-spezifisches Primerpaar SEQ ID NOS: 51 und 52;
s) BKV-spezifisches Primerpaar SEQ ID NOS: 49 und 50;
t) HHV4-spezifisches Primerpaar SEQ ID NOS: 45 und 46;
u) HHV6-spezifisches Primerpaar SEQ ID NOS: 47 und 48;
v) HHV7-spezifisches Primerpaar SEQ ID NOS: 43 und 44; und
w) JCV-spezifisches Primerpaar SEQ ID NOS: 53 und 54;

wobei die Krankheitserreger-spezifischen Primerpaare fähig sind, eine Vervielfältigung eines Polynukleotid-Amplikons einer anderen ausgewählten bekannten Länge wie eine Nukleinsäure von jedem der mindestens vier viralen Krankheitserreger herbeizuführen;

b) Zusammenbringen der Nukleinsäure aus der Probe, die unter Verdacht steht, irgendeinen der vier oder mehr viralen Krankheitserreger zu enthalten, mit den Krankheitserreger-spezifischen Primerpaaren aus Schritt (a) in einem Reaktionsgemisch in einem Vervielfältigungsschritt unter Bedingungen, die Vervielfältigung eines Polynucleotid-Amplikons fördern;
c) Entnehmen eines Aliquots aus dem Reaktionsgemisch zu einer Anzahl Abständen während des Vervielfältigungsschrittes;
d) Abtrennen der Amplikone in jedem Aliquot; und
e) Erfassen der Amplikone in jedem Aliquot, wobei die Erfassung eines Amplikons einer ausgewählten, bekannten Länge anzeigt, dass die Probe das dem Amplikon entsprechende Krankheitserreger-spezifische Target enthält.

2. Verfahren gemäß Anspruch 1, zudem umfassend Quantifizieren des erfassten Amplikons und Korrelieren der Menge erfassten Amplicons mit der Menge in der Probe vorhandenem Krankheitserreger.

3. Verfahren gemäß Anspruch 1 oder 2, wobei der Vervielfältigungsschritt umfasst Vervielfältigung durch Polymerase-Kettenreaktion (PCR).

4. Verfahren gemäß irgendeinem der Ansprüche 1 bis 3, wobei die Nukleinsäure aus der Probe vor dem Vervielfältigungsschritt eine reverse Transkription (RT) durchläuft.

5. Verfahren gemäß irgendeinem der Ansprüche 1 bis 4, wobei die Amplikone aus Schritt (c) durch Kapillarelektrophorese abgetrennt werden.

6. Verfahren gemäß irgendeinem der Ansprüche 1 bis 5, wobei die ein oder mehrer Krankheitserreger-spezifische Primere eine erfassbare Markierung umfassen.

7. Verfahren gemäß Anspruch 6, wobei die erfassbare Markierung eine fluoreszierende Markierung ist.

8. Verfahren gemäß Anspruch 6 oder 7, zudem umfassend Quantifizieren der Menge Krankheitserreger in der Probe durch Quantifizieren der Menge erfassbare Markierung im Aliquot des Reaktionsgemischs.

9. Verfahren gemäß irgendeinem der Ansprüche 1 bis 8, wobei das Verfahren fähig ist, mindestens vier Krankheitserreger in der Probe zu erfassen.

10. Verfahren gemäß irgendeinem der Ansprüche 1 bis 8, wobei das Verfahren fähig ist, mindestens fünf Krankheitserreger in der Probe zu erfassen.

11. Verfahren gemäß irgendeinem der Ansprüche 1 bis 10, wobei alle Krankheitserreger-spezifische Primerpaare fähig sind, die Vervielfältigung eines Polynukleotids unter mindestens einem gemeinsamen Satz Reaktionsbedingungen zu begünstigen.

12. Verfahren gemäß irgendeinem der Ansprüche 1 bis 11, wobei die Probe von einem immungeschwächten Menschen erhalten ist.

13. Verfahren gemäß Anspruch 12, wobei die immungeschwächte Person eine Transplantation erhalten hat.

14. Verfahren zur Erfassung und Quantifizierung einer Anzahl vorbestimmter Krankheitserreger in einer Probe, das Verfahren umfassend

a) Erhalten einer Probe, die unter Verdacht steht, mindestens einen aus einer Anzahl Krankheitserreger zu enthalten, ausgewählt aus der Gruppe EBV (HHV4), CMV, HHV6, HHV7, JCV und BKV, wobei jeder Krankheitserreger ein Krankheitserreger-spezifisches Nukleotid enthält;
b) Isolieren von Nukleinsäure aus der Probe;
c) Auswählen eines Satzes Krankheitserreger-spezifischer Primerpaare, die auf jeden aus der Anzahl Krankheitserreger-spezifischer Polynukleotide abzielen, wobei der Satz Primerpaare ausgewählt ist aus der Gruppe

1) vier oder mehr Vervielfältigungs-Primerpaare, ausgewählt aus der Gruppe

a) CMV-spezifisches Primerpaar SEQ ID NOS: 9 und 10;
b) BKV-spezifisches Primerpaar SEQ ID NOS: 7 und 8;
c) BKV-spezifisches Primerpaar SEQ ID NOS: 15 und 16;
d) HHV4-spezifisches Primerpaar SEQ ID NOS: 3 und 4;
e) HHV6-spezifisches Primerpaar SEQ ID NOS: 5 und 6;
f) HHV7-spezifisches Primerpaar SEQ ID NOS: 1 und 2; und
g) JCV-spezifisches Primerpaar SEQ ID NOS: 11 und 12; oder

2) vier oder mehr Vervielfältigungs-Primerpaare, ausgewählt aus der Gruppe

h) CMV-spezifisches Primerpaar SEQ ID NOS: 27 und 28;
i) CMV-spezifisches Primerpaar SEQ ID NOS: 37 und 38;
j) BKV-spezifisches Primerpaar SEQ ID NOS: 25 und 26;
k) HHV4-spezifisches Primerpaar SEQ ID NOS: 21 und 22;
l) HHV4-spezifisches Primerpaar SEQ ID NOS: 33 und 34;
m) HHV4-spezifisches Primerpaar SEQ ID NOS: 41 und 42;
n) HHV6-spezifisches Primerpaar SEQ ID NOS: 23 und 24;
o) HHV6-spezifisches Primerpaar SEQ ID NOS: 35 und 36;
p) HHV7-spezifisches Primerpaar SEQ ID NOS: 19 und 20; und
q) JCV-spezifisches Primerpaar SEQ ID NOS: 29 und 30; oder

3) vier oder mehr Vervielfältigungs-Primerpaare, ausgewählt aus der Gruppe

r) CMV-spezifisches Primerpaar SEQ ID NOS: 51 und 52;
s) BKV-spezifisches Primerpaar SEQ ID NOS: 49 und 50;
t) HHV4-spezifisches Primerpaar SEQ ID NOS: 45 und 46;
u) HHV6-spezifisches Primerpaar SEQ ID NOS: 47 und 48;
v) HHV7-spezifisches Primerpaar SEQ ID NOS: 43 und 44; und
w) JCV-spezifisches Primerpaar SEQ ID NOS: 53 und 54;
und wobei jedes Primerpaar fähig ist, Vervielfältigung eines Amplikons herbeizuführen mit einer Länge, die sich unterscheidet von Amplikonen, die durch jedes der anderen Krankheitserreger-spezifischen Primerpaare, die auf jedes andere Mitglied der Anzahl Krankheitserreger-spezifische Nukleinsäuren abzielen, hergestellt werden,

d) Auswählen mindestens eines Kompetitor-Polynukleotids, wobei das Kompetitor-Polynukleotid fähig ist, als Matrize zum Vermitteln der Vervielfältigung eines Amplikons durch ein Krankheitserreger-spezifisches Primerpaar zu funktionieren, wobei das hergestellte Amplikon eine Länge hat, die sich unterscheidet von durch jedes der weiteren Krankheitserreger-spezifischen Primerpaare hergestellten Amplikone, die auf jedes weitere Mitglied aus der Anzahl Krankheitserreger-spezifische Nukeinsäuren und weitere Kompetitor-Polynukleotide abzielen;

e) Zusetzen einer vorbestimmten Menge mindestens eines Kompetitor-Polynukleotids zu aus der Probe isolierter Nukleinsäure, um ein Testgemisch zu bilden;

f) Zusammenbringen des Testgemischs mit einer Anzahl Krankheitserreger-spezifischen Primerpaaren im Reaktionsgemisch unter Bedingungen, die Vervielfältigung eines Amplicons begünstigen;

g) Abtrennen der in Schritt f) gebildeten Amplikone;

h) Erfassen der Länge jedes der in Schritt f) gebildeten Amplikone;

i) Korrelieren der Länge jedes der erfassten Amplikone mit einem im Reaktionsgemisch vorhandenen Krankheitserreger oder Kompetitor-Polynukleotid;

j) Quantifizieren der Menge jedes in Schritt (f) gebildeten Amplikons; und

k) Bestimmen der Menge in der Probe vorhandenen Krankheitserreger auf der Basis der vorbestimmten Menge zur Probe zugesetztes Kompetitor-Polynukleotid.

15. Verfahren gemäß irgendeinem der vorhergehenden Ansprüche, wobei der Satz Primerpaare fünf oder mehr der Primerpaare (a)-(g) umfasst, fünf oder mehr der Primerpaare (h)-(q) oder fünf oder mehr der Primerpaare (r)-(w).

16. Zusammensetzung für die Multiplex-Erfassung von viralen Krankheitserregern gemäß dem Verfahren aus irgendeinem der vorhergehenden Ansprüche, wobei die Zusammensetzung umfasst einen Satz Vervielfältigungs-Primerpaare welche, wenn in einer PCR-Reaktion verwendet, eine Multiplex-Vervielfältigung einer Anzahl viraler Nukleinsäuren-Maskensequenzen ermöglicht, der Satz umfassend

1) vier oder mehr Vervielfältigungs-Primerpaare, ausgewählt aus der Gruppe

a) CMV-spezifisches Primerpaar SEQ ID NOS: 9 und 10;
b) BKV-spezifisches Primerpaar SEQ ID NOS: 7 und 8;
c) BKV-spezifisches Primerpaar SEQ ID NOS: 15 und 16;
d) HHV4-spezifisches Primerpaar SEQ ID NOS: 3 und 4;
e) HHV6-spezifisches Primerpaar SEQ ID NOS: 5 und 6;
f) HHV7-spezifisches Primerpaar SEQ ID NOS: 1 und 2; und
g) JCV-spezifisches Primerpaar SEQ ID NOS: 11 und 12; oder

2) vier oder mehr Vervielfältigungs-Primerpaare, ausgewählt aus der Gruppe

h) CMV-spezifisches Primerpaar SEQ ID NOS: 27 und 28;
i) CMV-spezifisches Primerpaar SEQ ID NOS: 37 und 38;
j) BKV-spezifisches Primerpaar SEQ ID NOS: 25 und 26;
k) HHV4-spezifisches Primerpaar SEQ ID NOS: 21 und 22;
l) HHV4-spezifisches Primerpaar SEQ ID NOS: 33 und 34;
m) HHV4-spezifisches Primerpaar SEQ ID NOS: 41 und 42;
n) HHV6-spezifisches Primerpaar SEQ ID NOS: 23 und 24;
o) HHV6-spezifisches Primerpaar SEQ ID NOS: 35 und 36;
p) HHV7-spezifisches Primerpaar SEQ ID NOS: 19 und 20; und
q) JCV-spezifisches Primerpaar SEQ ID NOS: 29 und 30; oder

3) vier oder mehr Vervielfältigungs-Primerpaare, ausgewählt aus der Gruppe

r) CMV-spezifisches Primerpaar SEQ ID NOS: 51 und 52;
s) BKV-spezifisches Primerpaar SEQ ID NOS: 49 und 50;
t) HHV4-spezifisches Primerpaar SEQ ID NOS: 45 und 46;
u) HHV6-spezifisches Primerpaar SEQ ID NOS: 47 und 48;
v) HHV7-spezifisches Primerpaar SEQ ID NOS: 43 und 44; und
w) JCV-spezifisches Primerpaar SEQ ID NOS: 53 und 54.

17. Zusammensetzung gemäß Anspruch 16, in welcher der Satz Vervielfältigungs-Primerpaare fünf oder mehr der Primerpaare (a)-(g) umfasst, fünf oder mehr der Primerpaare (h)-(q) oder fünf oder mehr der Primerpaare (r)-(w).

**Revendications**

1. Un procédé d'analyse d'un échantillon de façon à détecter la présence d'un agent pathogène quelconque d'un ensemble d'agents pathogènes viraux prédéterminés, le procédé comprenant :

a) la fourniture d'un ensemble de paires d'amorces spécifiques à un agent pathogène correspondant à chacun de quatre ou plus d'un ensemble d'agents pathogènes viraux sélectionnés dans le groupe se composant de EBV (HHV4), CMV, HHV6, HHV7, JCV et BKV, où l'ensemble de paires d'amorces est sélectionné dans le groupe se composant de

1) quatre ou plus paires d'amorces d'amplification sélectionnées dans le groupe se composant de

a) la paire d'amorces spécifique de CMV SEQ ID N°: 9 et 10 ;
b) la paire d'amorces spécifique de BKV SEQ ID N°: 7 et 8 ;
c) la paire d'amorces spécifique de BKV SEQ ID N°: 15 et 16 ;
d) la paire d'amorces spécifique de HHV4 SEQ ID N°: 3 et 4 ;
e) la paire d'amorces spécifique de HHV6 SEQ ID N°: 5 et 6 ;
f) la paire d'amorces spécifique de HHV7 SEQ ID N°: 1 et 2 ; et
g) la paire d'amorces spécifique de JCV SEQ ID N°: 11 et 12 ; ou

2) quatre ou plus paires d'amorces d'amplification sélectionnées dans le groupe se composant de :

h) la paire d'amorces spécifique de CMV SEQ ID N°: 27 et 28 ;
i) la paire d'amorces spécifique de CMV SEQ ID N°: 37 et 38 ;
j) la paire d'amorces spécifique de BKV SEQ ID N°: 25 et 26 ;
k) la paire d'amorces spécifique de HHV4 SEQ ID N°: 21 et 22 ;
l) la paire d'amorces spécifique de HHV4 SEQ ID N°: 33 et 34 ;
m) la paire d'amorces spécifique de HHV4 SEQ ID N°: 41 et 42 ;
n) la paire d'amorces spécifique de HHV6 SEQ ID N°: 23 et 24 ;
o) la paire d'amorces spécifique de HHV6 SEQ ID N°: 35 et 36 ;
p) la paire d'amorces spécifique de HHV7 SEQ ID N°: 19 et 20 ; et
q) la paire d'amorces spécifique de JCV SEQ ID N°: 29 et 30 ; ou

3) quatre ou plus paires d'amorces d'amplification sélectionnées dans le groupe se composant de :

r) la paire d'amorces spécifique de CMV SEQ ID N°: 51 et 52 ;
s) la paire d'amorces spécifique de BKV SEQ ID N°: 49 et 50 ;
t) la paire d'amorces spécifique de HHV4 SEQ ID N°: 45 et 46 ;
u) la paire d'amorces spécifique de HHV6 SEQ ID N°: 47 et 48 ;
v) la paire d'amorces spécifique de HHV7 SEQ ID N°: 43 et 44 ; et
w) la paire d'amorces spécifique de JCV SEQ ID N°: 53 et 54 ;

où les paires d'amorces spécifiques à un agent pathogène sont capables de médier une amplification d'un amplicon polynucléotide d'une longueur connue, sélectionnée différente à partir d'un acide nucléique de chacun desdits au moins quatre agents pathogènes viraux ;

b) la mise en contact d'un acide nucléique provenant de l'échantillon suspecté contenir l'un quelconque desdits quatre ou plus agents pathogènes viraux avec lesdites paires d'amorces spécifiques à un agent pathogène de l'opération (a) dans un mélange réactionnel dans une opération d'amplification dans des conditions qui favorisent une amplification d'un amplicon polynucléotide ;
c) le retrait d'une aliquote du mélange réactionnel à une pluralité d'intervalles au cours de l'opération d'amplification ;
d) la séparation des amplicons dans chaque aliquote ; et
e) la détection des amplicons dans chaque aliquote, où la détection d'un amplicon d'une longueur connue sélectionnée est indicative de l'échantillon contenant la cible spécifique à l'agent pathogène correspondant à l'amplicon.

2. Le procédé selon la revendication 1 comprenant en outre la quantification de l'amplicon détecté et la corrélation de la quantité d'amplicon détecté avec la quantité d'agent pathogène présente dans l'échantillon.

3. Le procédé selon la revendication 1 ou 2, où l'opération d'amplification comprend une amplification par la réaction en chaîne de la polymérase (PCR).

4. Le procédé selon l'une quelconque des revendications 1 à 3, où l'acide nucléique provenant de l'échantillon est soumis à une transcription inverse (RT) avant l'opération d'amplification.

5. Le procédé selon l'une quelconque des revendications 1 à 4, où les amplicons de l'opération (c) sont séparés par électrophorèse capillaire.

6. Le procédé selon l'une quelconque des revendications 1 à 5, où les une ou plusieurs amorces spécifiques à l'agent pathogène comprennent un marqueur détectable.

7. Le procédé selon la revendication 6, où le marqueur détectable est un marqueur fluorescent.

8. Le procédé selon la revendication 6 ou 7, comprenant en outre la quantification de la quantité d'agent pathogène dans l'échantillon par la quantification de la quantité de marqueur détectable dans l'aliquote du mélange réactionnel.

9. Le procédé selon l'une quelconque des revendications 1 à 8, où le procédé est capable de détecter au moins quatre agents pathogènes dans l'échantillon.

10. Le procédé selon l'une quelconque des revendications 1 à 8, où le procédé est capable de détecter au moins cinq agents pathogènes dans l'échantillon.

11. Le procédé selon l'une quelconque des revendications 1 à 10 où toutes les paires d'amorces spécifiques à un agent pathogène sont capables de favoriser une amplification d'un polynucléotide dans au moins un ensemble commun de conditions de réaction.

12. Le procédé selon l'une quelconque des revendications 1 à 11, où l'échantillon est obtenu auprès d'un humain immunovulnérable.

13. Le procédé selon la revendication 12, où ledit individu immunovulnérable a reçu une transplantation.

14. Un procédé de détection et de quantification d'une pluralité d'agents pathogènes prédéterminés dans un échantillon, le procédé comprenant :

a) l'obtention d'un échantillon suspecté contenir au moins un agent d'une pluralité d'agents pathogènes sélectionnés dans le groupe se composant de EBV (HHV4), CMV, HHV6, HHV7, JCV et BKV, où chaque agent pathogène comprend un polynucléotide spécifique à un agent pathogène,
b) l'isolation d'un acide nucléique provenant de l'échantillon,
c) la sélection d'un ensemble de paires d'amorces spécifiques à un agent pathogène ciblées sur chaque polynucléotide de la pluralité de polynucléotides spécifique à un agent pathogène, où l'ensemble de paires d'amorces est sélectionné dans le groupe se composant de

1) quatre ou plus paires d'amorces d'amplification sélectionnées dans le groupe se composant de

a) la paire d'amorces spécifique de CMV SEQ ID N°: 9 et 10 ;
b) la paire d'amorces spécifique de BKV SEQ ID N°: 7 et 8 ;
c) la paire d'amorces spécifique de BKV SEQ ID N°: 15 et 16 ;
d) la paire d'amorces spécifique de HHV4 SEQ ID N°: 3 et 4 ;
e) la paire d'amorces spécifique de HHV6 SEQ ID N°: 5 et 6 ;
f) la paire d'amorces spécifique de HHV7 SEQ ID N°: 1 et 2 ; et
g) la paire d'amorces spécifique de JCV SEQ ID N°: 11 et 12 ; ou

2) quatre ou plus paires d'amorces d'amplification sélectionnées dans le groupe se composant de :

h) la paire d'amorces spécifique de CMV SEQ ID N°: 27 et 28 ;
i) la paire d'amorces spécifique de CMV SEQ ID N°: 37 et 38 ;
j) la paire d'amorces spécifique de BKV SEQ ID N°: 25 et 26 ;
k) la paire d'amorces spécifique de HHV4 SEQ ID N°: 21 et 22 ;
l) la paire d'amorces spécifique de HHV4 SEQ ID N°: 33 et 34 ;
m) la paire d'amorces spécifique de HHV4 SEQ ID N°: 41 et 42 ;
n) la paire d'amorces spécifique de HHV6 SEQ ID N°: 23 et 24 ;
o) la paire d'amorces spécifique de HHV6 SEQ ID N°: 35 et 36 ;
p) la paire d'amorces spécifique de HHV7 SEQ ID N°: 19 et 20 ; et
q) la paire d'amorces spécifique de JCV SEQ ID N°: 29 et 30 ; ou

3) quatre ou plus paires d'amorces d'amplification sélectionnées dans le groupe se composant de :

r) la paire d'amorces spécifique de CMV SEQ ID N°: 51 et 52 ;
s) la paire d'amorces spécifique de BKV SEQ ID N°: 49 et 50 ;
t) la paire d'amorces spécifique de HHV4 SEQ ID N°: 45 et 46 ;
u) la paire d'amorces spécifique de HHV6 SEQ ID N°: 47 et 48 ;
v) la paire d'amorces spécifique de HHV7 SEQ ID N°: 43 et 44 ; et
w) la paire d'amorces spécifique de JCV SEQ ID N°: 53 et 54 ;

et où chaque paire d'amorces est capable de médier une amplification d'un amplicon polynucléotide d'une longueur qui est distincte des amplicons produits par chacune des autres paires d'amorces spécifiques à un agent pathogène ciblées sur chaque autre membre de la pluralité d'acides nucléiques spécifiques à un agent pathogène ;

d) la sélection d'au moins un polynucléotide compétiteur où le polynucléotide compétiteur est capable de jouer le rôle de modèle de façon à médier une amplification d'un amplicon par une paire d'amorces spécifique à un agent pathogène où l'amplicon produit est d'une longueur qui est distincte des amplicons produits par chacune des autres paires d'amorces spécifiques à un agent pathogène ciblées sur chaque autre membre de la pluralité d'acides nucléiques spécifiques à un agent pathogène et autres polynucléotides compétiteurs ;
e) l'ajout d'une quantité prédéterminée d'au moins un polynucléotide compétiteur à un acide nucléique isolé de l'échantillon de façon à créer un mélange de test ;
f) la mise en contact du mélange de test avec une pluralité de paires d'amorces spécifiques à un agent pathogène dans le mélange réactionnel dans des conditions qui favorisent une amplification d'un amplicon ;
g) la séparation des amplicons générés à l'opération (f) ;
h) la détection de la longueur de chaque amplicon généré à l'opération (f) ;
i) la corrélation de la longueur de chaque amplicon détecté avec un agent pathogène ou un polynucléotide compétiteur présent dans le mélange réactionnel ;
j) la quantification de la quantité de chaque amplicon généré à l'opération (f) ; et
k) la détermination d'une quantité d'agent pathogène présente dans l'échantillon en fonction de la quantité prédéterminée de polynucléotide compétiteur ajoutée au mélange de test.

**15.** Le procédé selon l'une quelconque des Revendications précédentes, dans lequel l'ensemble de paires d'amorces comprend cinq ou plus paires d'amorces (a)-(g), cinq ou plus paires d'amorces (h)-(q) ou cinq ou plus paires d'amorces (r)-(w).

**16.** Une composition pour une détection multiplex d'agents pathogènes viraux selon le procédé de l'une quelconque des Revendications précédentes, où la composition comprend un ensemble de paires d'amorces d'amplification qui, lorsqu'elles sont utilisées dans une réaction PCR, permettent une amplification multiplex d'une pluralité de séquences modèles d'acide nucléique viral, l'ensemble comprenant :

1) quatre ou plus paires d'amorces d'amplification sélectionnées dans le groupe se composant de :

a) la paire d'amorces spécifique de CMV SEQ ID N°: 9 et 10 ;
b) la paire d'amorces spécifique de BKV SEQ ID N°: 7 et 8 ;
c) la paire d'amorces spécifique de BKV SEQ ID N°: 15 et 16 ;

d) la paire d'amorces spécifique de HHV4 SEQ ID N°: 3 et 4 ;
e) la paire d'amorces spécifique de HHV6 SEQ ID N°: 5 et 6 ;
f) la paire d'amorces spécifique de HHV7 SEQ ID N°: 1 et 2 ; et
g) la paire d'amorces spécifique de JCV SEQ ID N°: 11 et 12 ; ou

2) quatre ou plus paires d'amorces d'amplification sélectionnées dans le groupe se composant de :

h) la paire d'amorces spécifique de CMV SEQ ID N°: 27 et 28 ;
i) la paire d'amorces spécifique de CMV SEQ ID N°: 37 et 38 ;
j) la paire d'amorces spécifique de BKV SEQ ID N°: 25 et 26 ;
k) la paire d'amorces spécifique de HHV4 SEQ ID N°: 21 et 22 ;
l) la paire d'amorces spécifique de HHV4 SEQ ID N°: 33 et 34 ;
m) la paire d'amorces spécifique de HHV4 SEQ ID N°: 41 et 42 ;
n) la paire d'amorces spécifique de HHV6 SEQ ID N°: 23 et 24 ;
o) la paire d'amorces spécifique de HHV6 SEQ ID N°: 35 et 36 ;
p) la paire d'amorces spécifique de HHV7 SEQ ID N°: 19 et 20 ; et
q) la paire d'amorces spécifique de JCV SEQ ID N°: 29 et 30 ; ou

3) quatre ou plus paires d'amorces d'amplification sélectionnées dans le groupe se composant de :

r) la paire d'amorces spécifique de CMV SEQ ID N°: 51 et 52 ;
s) la paire d'amorces spécifique de BKV SEQ ID N°: 49 et 50 ;
t) la paire d'amorces spécifique de HHV4 SEQ ID N°: 45 et 46 ;
u) la paire d'amorces spécifique de HHV6 SEQ ID N°: 47 et 48 ;
v) la paire d'amorces spécifique de HHV7 SEQ ID N°: 43 et 44 ; et
w) la paire d'amorces spécifique de JCV SEQ ID N°: 53 et 54.

17. La composition selon la revendication 16, dans laquelle l'ensemble de paires d'amorces d'amplification comprend cinq ou plus paires d'amorces (a)-(g), cinq ou plus paires d'amorces (h)-(q) ou cinq ou plus paires d'amorces (r)-(w).

| Pathogen | Genbank Accession Number | Version |
|---|---|---|
| HSV1 | NC_001806 | NC_001806.1<br>gi\|9629378 |
| HSV2 | NC_001798 | NC_001798.1<br>gi\|9629267 |
| EBV | NC_007346 | NC_007346.1<br>gi\|9625578 |
| CMV Human herpesvirus 5 (laboratory strain AD169), | NC_001347 | NC_001347.2<br>gi\|28373214 |
| CMV Human herpesvirus 5 (wild type strain Merlin) | NC_006273 | NC_006273.1<br>gi\|52139181 |
| HHV 6 | NC_001664 | NC_001664.1<br>GI:9628290 |
| HHV 6B | NC_001664 | NC_001664.1<br>gi\|9628290 |
| HHV7 | NC_001716 | NC_001716.2<br>gi\|51874225 |
| HHV8 | NC_003409.1 | NC_003409<br>gi\|18845965 |
| VZV | X04370 M14891 M16612 | X04370.1<br>GI:59989 |
| VZV | NC_001348 | NC_001348.1<br>GI:9625875 |
| hepatitis B | NC_001664 | NC_001664.1 GI:9628290 |

Fig. 1

| hepatitis C, | NC_004102. | NC_004102.1 gi|22129792 |
|---|---|---|
| adenovirus | | |
| Human adenovirus type 35 | AC_000019 | AC_000019.1 gi|56160914 |
| Human adenovirus type 7 | AC_000018 | AC_000018.1 gi|56160876 |
| Human adenovirus type 11 | AC_000015 | AC_000015.1 gi|56160777 |
| Human adenovirus type 17 | AC_000006 | AC_000006.1 gi|56160472| |
| EEEV | NC_003899 . | NC_003899.1 gi|21218484 |
| WNE | NC_001563 | NC_001563.2 gi|11528013 |
| JCV | NC_001699 | NC_001699.1 gi|9628642 |
| BKV | NC_001538 | NC_001538.1 gi|9627180 |

Fig. 1 (continued)

Fig. 2

Fig. 3

Fig. 4

**Set 1**

| | Sequence | SEQ ID NO Alternatives | | Sequence | SEQ ID NO |
|---|---|---|---|---|---|
| HHV7 | TCGGTTTGTATCGCTGAAGACACAT | 1 | | | |
| | CTGAAATCCGCTTGAGAGCCATAGT | 2 | | | |
| HHV4 | TCAACACATGCTCATCTCCCTTCTC | 3 | | | |
| | ATCTAAGGTCCATCGCGGAGTCATT | 4 | | | |
| HHV6 | TGCACTTGGTCAGGAGTCGATAAAA | 5 | | | |
| | GGTCACGTATACCATTCCAACCAT | 6 | | | |
| BK | TGCTTATCGAGTTGAGTGCTGGGTA | 7 | | GCTCCTCAATGGATGTTGCCTTTAC | 15 |
| | CCACACCCTGTTCATCTAGCAACAC | 8 | | CCCCTGGACACTCTCCTTTCTTTT | 16 |
| CMV | GTGTTCGGAAGTGATCGTGTTTGAC | 9 | | | |
| | CGTGTTTCGTCCTCGAAAGGTATC | 10 | | | |
| JC | GTGCAAATCAAAGATCTGTCCTCA | 11 | | | |
| | GGGGTCCTTCCTTTCTCCTTTTCTT | 12 | | | |
| endog | TCCTCCCTGTACGAAAGGACAAGAG | 13 | | ATCAAGATCATTGCTCCTCCTGAGC | 17 |
| | TAAGAAGAGGAATTGAACCTCTGACTGTAA | 14 | | CATACTCCTGCTTGCTGATCCACAT | 18 |

**Set 2**

| | Sequence | SEQ ID NO Alternatives | | Sequence | SEQ ID NO |
|---|---|---|---|---|---|
| HHV7 | GTATGTGCTGTAAAGGCCACGTCAG | 19 | | GACTAATGTGGTGGGGGCTATGGTA | 33 |
| | ATGGTAGTGATGTGCTTTGGGGTCT | 20 | | AATGACTCCAACACCTCCGTCTCTC | 34 |
| HHV4 | ATCAACAACTTTGTAGCCCGCAAGT | 21 | | TGTTTACGGTGCATGTGCAATTTT | 35 |
| | CCTTAAACCAGGACACGTTGAGACC | 22 | | TCCCAATTGTCTAGCATGTTTCTCCA | 36 |
| HHV6 | GTGTTTACGGTGCATGTGCAATTTT | 23 | | | |
| | TCCCAATTGTCTAGCATGTTTCTCCA | 24 | | | |
| BK | GCTTGATCCATGTCCAGAGTCTTCA | 25 | | | |
| | GGAAGGAAAGGCTGGATTCTGAGAT | 26 | | | |
| CMV | CCATTTCTTGGATGGCTAACGTCTC | 27 | | TCCGGCGATGTTTACTTTATCAACC | 37 |
| | CTCTAACAGGTTTTGCTCGGTTTGG | 28 | | CCGTGATAAAACACAAACTGGCAAA | 38 |
| JC | TTATGCCATGCCCCTGAAGGTAAATC | 29 | | | |
| | TATTCAAGGGGCCAATAGACAGTGG | 30 | | | |
| endog | TCCTCCCTGTACGAAAGGACAAGAG | 31 | | ATCAAGATCATTGCTCCTCCTGAGC | 39 |
| | TAAGAAGAGGAATTGAACCTCTGACTGTAA | 32 | | CATACTCCTGCTTGCTGATCCACAT | 40 |
| | | | | CGAGGTGCATGATGATACCATAGAGAG | 41 |
| | | | | AACATCCTGGTGGATTCACAGACA | 42 |

Fig. 5A

Set 3

Fig. 5B

EP 1 951 888 B1

| | | SEQ ID NO | Alternatives | SEQ ID NO |
|---|---|---|---|---|
| HHV7 | AAATCGCGCAGGTAGTTTTCCACTA | 43 | | |
| | GTCCTTGTCTCAGCATGTGTTTGCT | 44 | | |
| HHV4 | GTGGACTTGATGAAGCTGTTCTGGA | 45 | | |
| | CCTGCCCATCTGTATGTGCTATGAG | 46 | | |
| HHV6 | TGCACTTGGTCAGGAGTCGATAAAA | 47 | | |
| | TCCAGGGAACTTGATGTTGATCTGA | 48 | | |
| BK | ATTATTTGGACCCACCATTGCAGAG | 49 | | |
| | GGCCTATTTGTTCCAAAAAGCCTTC | 50 | | |
| CMV | CTCCCGCTTATCCTCAGGTACAATG | 51 | | |
| | GGAGGATGTTTGCAGAATGCCTTAG | 52 | | |
| JC | TTATGCCATGCCCTGAAGGTAAATC | 53 | | |
| | TTGATCTCTGTGGGGGAAAGTCATT | 54 | | |
| endog | TCCTCCCTGTACGAAAGGACAAGAG | 55 | ATCAAGATCATTGCTCCTCCTGAGC | 57 |
| | TAAGAAGAGGAATTGAACCTCTGACTGTAA | 56 | CATACTCCTGCTTGCTGATCCACAT | 58 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683202 A
- US 4683195 A
- US 6268132 B
- US 5763167 B
- WO 9302215 A
- WO 9211273 A
- US 5213961 A
- US 5219727 A
- US 6524793 B
- US 5558863 A
- US 6936251 B
- US 5545520 A
- US 5756302 A
- US 20040091852 A
- US 20030013077 A
- US 20040121975 A
- US 6593123 B
- US 20040197769 A
- US 6238859 B
- US 6605602 B
- US 38728603 A
- US 4458066 A
- US 4800159 A
- WO 0132887 A
- US 5525711 A, Hawkins
- US 5750409 A
- US 5366860 A
- US 5231191 A
- US 5840999 A
- US 5847162 A
- US 4439356 A
- US 4481136 A
- US 5188934 A
- US 5654442 A
- US 5066580 A
- US 5486616 A
- US 5569587 A
- US 5569766 A
- US 5627027 A
- US 5321130 A
- US 5410030 A
- US 5436134 A
- US 5534416 A
- US 5582977 A
- US 5658751 A
- US 5656449 A
- US 5863753 A
- WO 9736960 A
- WO 9927020 A
- WO 9916832 A
- EP 0050684 A
- JP 10000928 A
- US 6217731 B
- US 6001230 B
- US 5963456 B
- US 60735085 B

### Non-patent literature cited in the description

- PCR Technology: Principles and Applications for DNA Amplification. Freeman Press, 1992
- PCR Protocols : A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **WU ; WALLACE.** *Genomics,* 1989, vol. 4, 560
- **LANDEGREN et al.** *Science,* 1988, vol. 241, 1077
- **KWOH et al.** *Proc. Natl. Acad. Sci. USA,* 1989, vol. 86, 1173
- **GUATELLI et al.** *Proc. Natl. Acad. Sci. USA,* 1990, vol. 87, 1874
- **SOOKNANAN, R. ; MALEK, L.** *Bio Technology,* 1995, vol. 13, 563-65
- **DELUCA et al.** Immunofluorescence Analysis, in Antibody As a Tool. John Wiley & Sons, Ltd, 1982
- **HAWKINS et al.** *Nucleic Acids Research,* 1995, vol. 23, 2872-2880
- **SEELA et al.** *Helvetica Chimica Acta,* 2000, vol. 83, 910-927
- **WIERZCHOWSKI et al.** *Biochimica et Biophysica Acta,* 1996, vol. 1290, 9-17
- **VIRTA et al.** *Nucleosides, Nucleotides & Nucleic Acids,* 2003, vol. 22, 85-98
- **STRYER et al.** *Ann. Rev. Biochem.,* 1978, vol. 47, 819
- **SELVIN.** *Methods Enzymol.,* 1995, vol. 246, 300

- **OHTSUKI et al.** *Proc. Natl. Acad. Sci.,* 2001, vol. 98, 4922-4925
- **LUTZ et al.** *Bioorg. Med. Chem. Lett.,* 1998, vol. 8, 1149-1152
- **VOEGEL ; BENNER.** *Helv. Chim. Acta,* 1996, vol. 76, 1863-1880
- **HORLACHER et al.** *Proc. Natl. Acad. Sci.,* 1995, vol. 92, 6329-6333
- **SWITZER et al.** *Biochemistry,* 1993, vol. 32, 10489-10496
- **TOR ; DERVAN.** *J. Am. Chem. Soc.,* vol. 115, 4461-4467
- **PICCIRILLI et al.** *Biochemistry,* 1991, vol. 30, 10350-10356
- **SWITZER et al.** *J. Am. Chem. Soc.,* 1989, vol. 111, 8322-8323
- **DAVIS et al.** Basic Methods in Molecular Biology. Elsevier, 1986
- Current Protocols in Molecular Biology. John Weley & Sons, Inc, 1997
- **KIMURA H et al.** Quantitative analysis of Epstein-Barr virus load by using a real-time PCR assay. *J. Clin Microbiol.,* 1999, vol. 37, 132
- **MARTELL M et al.** High-throughput real-time reverse transcription-PCR quantitation of hepatitis C virus RNA. *J Clin Microbiol.,* February 1999, vol. 37 (2), 327-32
- **MERCIER B et al.** Simultaneous screening for HBV DNA and HCV RNA genomes in blood donations using a novel TaqMan PCR assay. *J Virol Methods,* January 1999, vol. 77 (1), 1-9
- **CHELLY et al.** *Nature,* vol. 333, 858-860
- **KIEVITS et al.** *J. Virol. Methods,* 1991, vol. 35, 273-86
- **WALKER.** *Nucleic Acids Res.,* 1994, vol. 22, 2670-7
- **RAJEVIC.** *Pflugers Arch.,* 2001, vol. 442 (6), R190-2
- **BORSON et al.** *Biotechniques,* 1998, vol. 25, 130-7
- **GEORGE et al.** *J Chromatogr B Biomed Sci Appl,* 1997, vol. 695, 93-102
- **ODIN et al.** *J Chromatogr B Biomed Sci Appl,* 1999, vol. 734, 47-53
- **HARLOW ; LANE.** Antibodies, A Laboratory Manual. Cold Spring Harbor Publications, 1988
- **GRANDIEN M.** *Clin Diagn Virol.,* 05 May 1996, 81-90
- *J Virol Methods,* May 1996, vol. 59 (1-2), 177-87
- **COYLE PV ; DESAI A ; WYATT D ; MCCAUGHEY C ; O'NEILL HJ.** *J Virol Methods,* December 1999, vol. 83 (1-2), 75-82
- **THIRUMALAPURA NR ; MORTON RJ ; RAMACHANDRAN A ; MALAYER JR.** *J Immunol Methods,* March 2005, vol. 298 (1-2), 73-81
- **YU KY ; NOH Y ; CHUNG M ; PARK HJ ; LEE N ; YOUN M ; JUNG BY ; YOUN BS.** *Clin Diagn Lab Immunol.,* May 2004, vol. 11 (3), 446-51
- **HAMASUR B ; BRUCHFELD J ; HAILE M ; PAWLOWSKI A ; BJORVATN B ; KALLENIUS G ; SVENSON SB.** *J Microbiol Methods,* May 2001, vol. 45 (1), 41-52
- Methods in Molecular Biology. Immunocytochemical Methods and Protocols. Humana Press, 1999
- Enzyme-lynked immunoassay (ELISA. The ELISA Guidebook. Humana Press, 2000
- **NIESTERS H et al.** Development of a real-time quantitative assay for detection of Epstein-Barr virus. *J Clin Microbiol.,* February 2000, vol. 38 (2), 712-5
- **A. J. NAHMIAS ; B. ROIZMAN.** *New Engl. J. Med.,* 1973, vol. 289, 667-674
- **B. LIPSCHUTZ.** *Arch. Derm. Syph. (Berl,* 1921, vol. 136, 428-482
- **A. J. NAHMIAS ; B. ROIZMAN.** *New Engl. J. Med.,* 1973, vol. 13, 667-674
- Laboratory Diagnosis of Viral Infections. Marcel Dekker, Inc, 1985, 313-328
- The Epstein-Barr Virus. Springer-Verlag, 1979, 297
- Laboratory Diagnosis of Viral Infection. Marcel Dekker, Inc, 1985, 257-271
- Laboratory Diagnosis of Viral Infections. Marcel Dekker, Inc, 1985, 329-340
- **R. BOYD et al.** Basic Medical Microbiology. Little, Brown and Company, 1981, 527
- **DYMOCK, B. W.** *Emerging Drugs,* 2001, vol. 6, 13-42
- **LOHMAN et al.** *Science,* 1999, vol. 285, 110-113
- **HOOFNAGLE.** *N. Eng. J. Med.,* 1990, vol. 323, 337-339
- **DIENSTAG et al.** Harrison's Principles of Internal Medicine. McGraw-Hill, 1993, 1458-1483
- **RANEY et al.** Molecular Biology of the Hepatitis B Virus. CRC Press, 1991, 1-38
- **BULLER et al.** *J. Virol.,* 1981, vol. 40, 241-47
- **BERNS ; BOHENZKY.** Advances in Virus Research. Academic Press, Inc, 1987, vol. 32, 243-307
- **MUZYCZKA, N.** *Current Topics in Microbiol. and Immunol.,* 1992, vol. 158, 97-129
- **TEN BROECK, C. et al.** *J. Exp. Med.,* 1935, vol. 62, 677
- **SELF et al.** *Cell,* 1979, vol. 18, 963-77
- Bacterial "genes-to screens. **PUCCI MJ , B. T. ; DOUGHERTY TJ.** Pathogen Genomics. Humana Press Inc, 2002, 83-96
- Self-Sustained Sequence Replication (3SR. **GINGERAS et al.** Annales de Biologie Clinique. 1990, vol. 48, 498-501
- **GUATELLI et al.** *Proc. Natl. Acad. Sci. U.S.A.,* 1990, vol. 87, 1874

- **KWOH et al.** *Proc. Natl. Acad. Sci. U.S.A.,* 1989, vol. 83, 1173-1177
- **COMPTON.** *Nature,* 1991, vol. 350, 91-92
- **KIEVITS et al.** *J. Virol Meth.,* 1991, vol. 35, 273-286
- **BARANY.** *Proc. Natl. Acad. Sci. USA,* 1991, vol. 88, 189
- **WALKER et al.** *Nucleic Acids Res.,* 1992, vol. 20, 1691-1696
- **SPARGO et al.** *Mol. Cellular Probes,* 1993, vol. 7, 395-404
- **WESTIN et al.** *Nature Biotechnology,* 2000, vol. 18, 199-204
- **KANEHISA, M.** *Polynucleotides Res.,* 1984, vol. 12, 203
- **NARANG et al.** *Methods in Enzymology,* 1979, vol. 68, 90
- **BROWN et al.** *Methods in Enzymology,* 1979, vol. 68, 109
- **BEAUCAGE et al.** *Tetrahedron Letters,* 1981, vol. 22, 1859
- **AUSUBEL.** Short Protocols in Molecular Biology. John Wiley & Sons, Inc, 1995
- **MULLIS ; FALOONA.** *Methods Enzymol.,* 1987, vol. 155, 335
- **LUNDBERG et al.** *Gene,* 1991, vol. 108, 1
- **HINNISDAELS et al.** *Biotechniques,* 1996, vol. 20, 186-8
- **MYERS ; GELFAND.** *Biochemistry,* 1991, vol. 30, 7661
- **STENESH ; MCGOWAN.** *Biochim Biophys Acta,* 1977, vol. 475, 32
- **CARIELLO et al.** *Polynucleotides Res,* 1991, vol. 19, 4193
- **DIAZ ; SABINO ; BRAZ.** *J. Med. Res,* 1998, vol. 31, 1239
- **CHIEN et al.** *J. Bacteoriol,* 1976, vol. 127, 1550
- **TAKAGI et al.** *Appl. Environ. Microbiol.,* 1997, vol. 63, 4504
- **JUNCOSA-GINESTA.** Deep-Vent DNA polymerase. *Biotechniques,* 1994, vol. 16, 820
- **DIAZ ; SABINO ; BRAZ.** *J. Med. Res.,* 1998, vol. 31, 1239
- **LECOMTE ; DOUBLEDAY.** *Polynucleotides Res.,* 1983, vol. 11, 7505
- **NORDSTROM et al.** *J. Biol. Chem.,* 1981, vol. 256, 3112
- **CANN et al.** *Proc. Natl. Acad. Sci. USA,* 1998, vol. 95, 14250-5
- Current Protocols in Molecular Biology. Current Protocols, 1996
- **ARNHEIM ; LEVINSON.** *C&EN,* 1990, 6-47
- *The Journal Of NIH Research,* 1991, vol. 3, 81-94
- **LOMELL et al.** *J. Clin. Chem,* 1989, vol. 35, 1826
- **LANDEGREN et al.** *Science,* 1988, vol. 241, 1077-1080
- **BRUNT.** *Biotechnology,* 1990, vol. 8, 291-294
- **WU ; WALLACE.** *Gene,* 1989, vol. 4, 560
- **BARRINGER et al.** *Gene,* 1990, vol. 89, 117
- **SOOKNANAN ; MALEK.** *Biotechnology,* 1995, vol. 13, 563-564
- **INNIS ; SAMBROOK ; AUSUBEL et al.** PCR Protocols, A Guide to Methods and Applications
- **CHIRGWIN et al.** *Biochemistry,* 1979, vol. 18, 5294
- **CHOMCZYNSKI ; SACCHI.** *Anal. Biochem.,* 1987, vol. 162, 156
- Handbook of Fluoresdent Probes and Research Chemicals. Molecular Probes, Inc, 1996
- **SAUER et al.** *J. Fluorescence,* 1995, vol. 5, 247-261
- **LEE et al.** *Nucl. Acids Res.,* 1992, vol. 20, 2471-2483
- **TU et al.** *Nucl. Acids Res.,* 1998, vol. 26, 2797-2802
- **LEE et al.** *Polynucleotides Research,* 1997, vol. 25, 2816
- Handbook of Fluorescent Probes and Research Chemicals. Molecular Probes, Inc,
- **HORI et al.** *J. Antibiotics, Ser. A,* 1966, vol. 17, 96-99
- **AIZAWA et al.** *Agr. Biol. Chem.,* 1965, vol. 29, 375-376
- **KOYAMA et al.** *Tetrahedron Lett.,* 1966, 597-602
- **UMEZAWA et al.** *Antibiotics Ser. A,* 1965, vol. 18, 178-181
- **ISHIZUKA et al.** *J. Antibiotics,* 1968, vol. 21, 1-4
- **SAWA et al.** *Antibiotics,* 1968, vol. 21, 334-339
- **UEMATSU ; SUAHDOLNIK.** *Biochemistry,* 1972, vol. 11, 4669-4674
- **DARNALL et al.** *PNAS,* 1967, vol. 57, 548-553
- **SWEENY et al.** *Cancer Res.,* 1973, vol. 33, 2619-2623
- **KUSAKABE et al.** *J. Antibiotics,* 1972, vol. 25, 44-47
- **OCHI et al.** *J. Antibiotics,* 1974, vol. xxiv, 909-916
- **QUESADA et al.** *Biotechniques,* 1991, vol. 10, 616-25
- **NELSON et al.** Volatilization of High Molecular Weight DNA by Pulsed Laser Ablation of Frozen Aqueous Solutions. *Science,* 1989, vol. 246, 1585-87
- **HUTH-FEHRE et al.** *Rapid Communications in Mass Spectrometry,* 1992, vol. 6, 209-13
- **K. TANG et al.** *Rapid Communications in Mass Spectrometry,* 1994, vol. 8, 727-730

- **WILLIAMS et al.** Time-of Flight Mass Spectrometry of Nucleic Acids by Laser Ablation and Ionization from a Frozen Aqueous Matrix. *Rapid Communications in Mass Spectrometry,* 1990, vol. 4, 348-351

- **ROSS.** High level multiplex genotyping by MALDI-TOF mass spectrometry. *Nature Biotechnology,* 1998, vol. 16, 1347-1351

SEQUENCE LISTING

<110> Slepnev, vladimir I shiosaki, Kazumi Garcia, Elizabeth Hart, Kyle

<120> Multiplexed Quantitative Detection of pathogens

<130> 66699WO(219781)

<150> 60/735,085
<151> 2005-11-09

<160> 58

<170> PatentIn version 3.3

<210> 1
<211> 25
<212> DNA
<213> Human herpesvirus 7

<400> 1
tcggtttgta tcgctgaaga cacat 25

<210> 2
<211> 25
<212> DNA
<213> Human herpesvirus 7

<400> 2
ctgaaatccg cttgagagcc atagt        25

<210> 3
<211> 25
<212> DNA
<213> Human herpesvirus 4

<400> 3
tcaacacatg ctcatctccc ttctc        25

<210> 4
<211> 25
<212> DNA
<213> Human herpesvirus 4

<400> 4
atctaaggtc catcccggag tcatt        25


<210> 5
<211> 25
<212> DNA
<213> Human herpesvirus 6


<400> 5
tgcacttggt caggagtcga taaaa        25


<210> 6
<211> 25
<212> DNA
<213> Human herpesvirus 6


<400> 6
ggtcacgtat accattccca accat        25


<210> 7
<211> 25
<212> DNA
<213> BK virus


<400> 7
tgcttatcca gttgagtgct gggta        25


<210> 8
<211> 25
<212> DNA
<213> BK virus


<400> 8
ccacaccctg ttcatctagc aacac        25


<210> 9
<211> 25
<212> DNA
<213> Human cytomegalovirus


<400> 9
gtgttcggaa gtgatcgtgt ttgac        25


<210> 10
<211> 25
<212> DNA
<213> Human cytomegalovirus


<400> 10
cgtgttttcg tcctcgaaag gtatc        25


<210> 11
<211> 25
<212> DNA
<213> JC virus


<400> 11
gtgcaaatca aagatctgct cctca        25

<210> 12
<211> 25
<212> DNA
<213> JC virus

<400> 12
ggggtccttc ctttctcctt ttctt          25

<210> 13
<211> 25
<212> DNA
<213> Homo sapiens

<400> 13
tcctccctgt acgaaaggac aagag          25

<210> 14
<211> 30
<212> DNA
<213> Homo sapiens

<400> 14
taagaagagg aattgaacct ctgactgtaa          30

<210> 15
<211> 25
<212> DNA
<213> BK vi rus

<400> 15
gctcctcaat ggatgttgcc tttac          25

<210> 16
<211> 25
<212> DNA
<213> BK virus

<400> 16
cccctggaca ctctcctttt ctttt          25

<210> 17
<211> 25
<212> DNA
<213> Homo sapiens

<400> 17
atcaagatca ttgctcctcc tgagc          25

<210> 18
<211> 25
<212> DNA
<213> Homo sapiens

<400> 18
catactcctg cttgctgatc cacat          25

<210> 19
<211> 25

<212> DNA
<213> Human herpesvirus 7

<400> 19
gtatgtgctg taaaggccac gtcag          25

<210> 20
<211> 25
<212> DNA
<213> Human herpesvirus 7

<400> 20
atggtagtga tgtgctttgg ggtct          25

<210> 21
<211> 25
<212> DNA
<213> Human herpesvirus 4

<400> 21
atcaacaact ttgtagcccg caagt          25

<210> 22
<211> 25
<212> DNA
<213> Human herpesvirus 4

<400> 22
ccttaaacca ggacacgttg agacc          25

<210> 23
<211> 25
<212> DNA
<213> Human herpesvirus 6

<400> 23
gtgtttacgg tgcatgtgca atttt          25

<210> 24
<211> 25
<212> DNA
<213> Human herpesvirus 6

<400> 24
tcccaattgt ctagcatgtt ctcca          25

<210> 25
<211> 25
<212> DNA
<213> BK virus

<400> 25
gcttgatcca tgtccagagt cttca          25

<210> 26
<211> 25
<212> DNA
<213> BK virus

<400> 26
ggaaggaaag gctggattct gagat        25


<210> 27
<211> 25
<212> DNA
<213> Human cytomegalovirus


<400> 27
ccatttcttg gatggctaac gtctc        25


<210> 28
<211> 25
<212> DNA
<213> Human cytomegalovirus


<400> 28
ctctaacagg ttttgctcgg tttgg        25


<210> 29
<211> 25
<212> DNA
<213> JC virus


<400> 29
ttatgccatg ccctgaaggt aaatc        25


<210> 30
<211> 25
<212> DNA
<213> JC virus


<400> 30
tattcaaggg gccaatagac agtgg        25


<210> 31
<211> 25
<212> DNA
<213> Homo sapiens


<400> 31
tcctccctgt acgaaaggac aagag        25


<210> 32
<211> 30
<212> DNA
<213> Homo sapiens


<400> 32
taagaagagg aattgaacct ctgactgtaa        30


<210> 33
<211> 25
<212> DNA
<213> Human herpesvirus 4


<400> 33
gactaatgtg gtgggggcta tggta        25

<210> 34
<211> 25
<212> DNA
<213> Human herpesvirus 4

<400> 34
aatgactcca acacctccgt ctctc          25

<210> 35
<211> 25
<212> DNA
<213> Human herpesvirus 6

<400> 35
tgtttacggt gcatgtgcaa ttttt          25

<210> 36
<211> 25
<212> DNA
<213> Human herpesvirus 6

<400> 36
tcccaattgt ctagcatgtt ctcca          25

<210> 37
<211> 25
<212> DNA
<213> Human cytomegalovirus

<400> 37
tccggcgatg tttactttat caacc          25

<210> 38
<211> 25
<212> DNA
<213> Human cytomegalovirus

<400> 38
ccgtgataaa acacaaactg gcaaa          25

<210> 39
<211> 25
<212> DNA
<213> Homo sapiens

<400> 39
atcaagatca ttgctcctcc tgagc          25

<210> 40
<211> 25
<212> DNA
<213> Homo sapiens

<400> 40
catactcctg cttgctgatc cacat          25

<210> 41
<211> 25

<212> DNA
<213> Human herpesvirus 4

<400> 41
cgaggtgcat gataccatag agcag          25

<210> 42
<211> 25
<212> DNA
<213> Human herpesvirus 4

<400> 42
aacatcctgg tggatttcac agaca          25

<210> 43
<211> 25
<212> DNA
<213> Human herpesvirus 7

<400> 43
aaatcgcgca ggtagttttc cacta          25

<210> 44
<211> 25
<212> DNA
<213> Human herpesvirus 7

<400> 44
gtccttgtct cagcatgtgt ttgct          25

<210> 45
<211> 25
<212> DNA
<213> Human herpesvirus 4

<400> 45
gtggacttga tgaagctgtt ctgga          25

<210> 46
<211> 25
<212> DNA
<213> Human herpesvirus 4

<400> 46
cctgcccatc tgtatgtgct atgag          25

<210> 47
<211> 25
<212> DNA
<213> Human herpesvirus 6

<400> 47
tgcacttggt caggagtcga taaaa          25

<210> 48
<211> 25
<212> DNA
<213> Human herpesvirus 6

<400> 48
tccagggaac ttgatgttga tctga          25

<210> 49
<211> 25
<212> DNA
<213> BK virus

<400> 49
attatttgga cccaccattg cagag          25

<210> 50
<211> 25
<212> DNA
<213> BK virus

<400> 50
ggcctatttg ttccaaaaag ccttc          25

<210> 51
<211> 25
<212> DNA
<213> Human cytomegalovirus

<400> 51
ctcccgctta tcctcaggta caatg          25

<210> 52
<211> 25
<212> DNA
<213> Human cytomegalovirus

<400> 52
ggaggatgtt tgcagaatgc cttag          25

<210> 53
<211> 25
<212> DNA
<213> JC virus

<400> 53
ttatgccatg ccctgaaggt aaatc          25

<210> 54
<211> 25
<212> DNA
<213> JC virus

<400> 54
ttgatctctg tgggggaaag tcatt          25

<210> 55
<211> 25
<212> DNA
<213> Homo sapiens

<400> 55
tcctccctgt acgaaaggac aagag          25

<210> 56
<211> 30
<212> DNA
<213> Homo sapiens

<400> 56
taagaagagg aattgaacct ctgactgtaa          30

<210> 57
<211> 25
<212> DNA
<213> Homo sapiens

<400> 57
atcaagatca ttgctcctcc tgagc          25

<210> 58
<211> 25
<212> DNA
<213> Homo sapiens

<400> 58
catactcctg cttgctgatc cacat          25